# EUROPÄISCHE PATENTSCHRIFT

(11) **EP 3 707 141 B1**
(45) Veröffentlichungstag und Bekanntmachung des Hinweises auf die Patenterteilung: **22.12.2021**
(21) Anmeldenummer: 18800549.0
(22) Anmeldetag: 31.10.2018
(51) Int. Cl.: C07D 487/04, A61K 31/519, A61P 11/00

(54) **SUBSTITUIERTE 2,4-DIHYDRO-3H-1,2,4-TRIAZOL-3-ONE UND IHRE VERWENDUNG**
SUBSTITUTED 2,4-DIHYDRO-3H-1,2,4-TRIAZOL-3-ONES AND USE OF SAME
2,4-DIHYDRO-3H-1,2,4-TRIAZOL-3-ONES SUBSTITUÉES ET LEUR UTILISATION

(30) Priorität: 07.11.2017 EP 17200382
(43) Veröffentlichungstag der Anmeldung: 16.09.2020
(73) Patentinhaber: Bayer Aktiengesellschaft, 51373 Leverkusen (DE); Bayer Pharma Aktiengesellschaft, 13353 Berlin (DE)
(72) Erfinder: BIBER, Nicole, 42115 Wuppertal (DE); BROCKSCHNIEDER, Damian, 42781 Haan (DE); KÖLLING, Florian, 42115 Wuppertal (DE); MEDING, Jörg, 42115 Wuppertal (DE); MIYATAKE ONDOZABAL, Hideki, 10115 Berlin (DE); NEUBAUER, Thomas, 42111 Wuppertal (DE); SCHÄFER, Martina, 13465 Berlin (DE); ZUBOV, Dmitry, 42857 Remscheid (DE); TERJUNG, Carsten, 44799 Bochum (DE)
(74) Vertreter: BIP Patents
(86) Internationale Anmeldenummer: PCT/EP2018/079851
(87) Internationale Veröffentlichungsnummer: WO 2019/091847

(56) Entgegenhaltungen:
- GAËLLE MARIAULE ET AL: "3-Oxo-hexahydro-1 H -isoindole-4-carboxylic Acid as a Drug Chiral Bicyclic Scaffold: Structure-Based Design and Preparation of Conformationally Constrained Covalent and Noncovalent Prolyl Oligopeptidase Inhibitors", JOURNAL OF MEDICINAL CHEMISTRY, Bd. 59, Nr. 9, 12. Mai 2016 (2016-05-12), Seiten 4221-4234, XP055529980, ISSN: 0022-2623, DOI: 10.1021/acs.jmedchem.5b01296
- CURT D. HAFFNER ET AL: "Pyrrolidinyl pyridone and pyrazinone analogues as potent inhibitors of prolyl oligopeptidase (POP)", BIOORGANIC & MEDICINAL CHEMISTRY LETTERS, Bd. 18, Nr. 15, 1. August 2008 (2008-08-01), Seiten 4360-4363, XP055529997, AMSTERDAM, NL ISSN: 0960-894X, DOI: 10.1016/j.bmcl.2008.06.067

## Beschreibung

Die vorliegende Anmeldung betrifft neue substituierte 2,4-dihydro-3H-1,2,4-triazol-3-one, Verfahren zu ihrer Herstellung, die Verbindungen zur Verwendung allein oder in Kombinationen in einem Verfahren zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von inflammatorischen Lungen-Erkrankungen.

Das Enzym Prolyl-Endopeptidase (PREP, Prolyl-Oligopetidase, PE, POP) ist eine Serinprotease, welche Peptide bis zu einer Länge von 30 Aminosäuren hinter der Aminosäure Prolin spaltet. [Moriyama et al., J. Biochem. 1988, 104:112-117]. PREP wird in allen Organen und Geweben konstitutiv, unter anderem von Leukozyten und Epithelzellen exprimiert und sezerniert. Die Freisetzung wird bei Kontakt mit reizenden, entzündlichen Stoffen erhöht. [Szul et al., Am. J. Respir. Cell Mol. Biol. 2016, 54:359-369] Während PREP im zentralen Nervensystem am Abbau von peptidischen Neurotransmittern beteiligt ist, ist PREP in der gesamten Peripherie, so auch in der Lunge, ein Enzym, das in degenerativen bzw. entzündlichen Prozessen Abbauprodukte des Kollagens u.a. spaltet. Aufgrund des hohen Gehaltes von Prolin und Glyzin in der Aminosäuresequenz des Kollagens, werden dort Tripeptide mit der Sequenz Prolin-Glyzin-Prolin (PGP) durch den Abbau der Kollagenfragmente gebildet [Weathington et al., Nat. Med. 2006, 12:317-323].In Sputen von Patienten mit chronischen, inflammatorischen Lungenerkrankungen, wie der Chronisch Obstruktiven Lungenerkrankung (Chronic Obstructive Pulmonary Disease, COPD) oder der Zystischen Fibrose (Mukoviszidose, CF) wurden signifikant erhöhte PGP-Konzentrationen beschrieben [O'Reilly et al., Respir. Res. 2009, 10:38 doi:10.1186/1465-9921-10-38; Gaggar et al., J. Immunol. 2008, 180:5662-5669]. PGP, das von PREP produziert wird, ist ein Chemokin für neutrophile Granulozyten (kurz: Neutrophile), d. h. PGP führt zu einer Einwanderung von Neutrophilen in Gewebe mit erhöhten PGP-Konzentrationen. Es gibt Hinweise, dass der Mechanismus dieses Anlockens von Neutrophilen auf einer direkten Stimulierung der Neutrophilen über PGP-sensitive Rezeptoren auf der neutrophilen Zellmembran (z. B. CXCR1, CXCR2) beruht oder indirekt durch die Freisetzung von weiteren Chemokinen, wie beispielsweise Interleukinen (z. B. Interleukin-8, CXCL8) durch andere Zelltypen (z. B. Makrophagen, Epithelzellen) bewirkt wird. Die chemokine Wirkung von PGP auf Neutrophile wurde mehrfach in vitro und in vivo demonstriert. [Weathington et al., Nat. Med. 2006, 12:317-323; De Kruijf et al., Eur. J. Pharmacol. 2010, 643:29-33; Overbeek et al., Eur. J. Pharmacol. 2011, 668:428-434; Braber et al., Eur. J. Pharmacol. 2011, 668:443-449] Im Tierversuch konnte die Wirkung von PGP durch die Gabe eines komplementären Tripeptides (Threonin-Arginin-Threonin, RTR) sowie durch die Applikation eines CXCR2-Rezeptor-Blockers aufgehoben werden. Es wurde gezeigt, dass eine wiederholte Applikation von PGP in die Lunge von Mäusen ein Lungenemphysem auslösen kann. Zudem wird durch Zigarettenrauch die PGP-Konzentration in den Lungen von Mäusen, die Zigarettenrauch ausgesetzt werden, erhöht. Die gleichzeitige Gabe von PGPneutralisierendem RTR kann die oben genannten, Zigarettenrauch-induzierten Effekte aufheben [Braber et al., Eur J. Pharmacol. 2011, 668:443-449; Braber et al., Am. J. Physiol. Lung Cell Mol. Pysiol. 2011, 300:L255-L265].

Die Chronisch Obstruktive Lungenerkrankung (Chronic Obstructive Pulmonary Disease, COPD) ist eine Erkrankung der Lunge, die mit chronischer Bronchitis, Atemnot, Husten und Auswurf sowie mit dem Untergang von Lungengewebe (Emphysem) einhergeht. Die Lungenfunktion ist aufgrund der obstruktiven Veränderung der Bronchien und durch den Verlust von funktionalem Lungengewebe mit fortschreitender Erkrankung zunehmend eingeschränkt. Eine Überblähung der Lunge ist aufgrund der behinderten Ausatmung häufig.

Die häufigste Ursache für die Entstehung einer COPD ist eine chronische Inhalation von Zigarettenrauch. Darüber hinaus entwickeln weltweit, mit regionalen Unterschieden 10 bis 40 % der Patienten eine COPD, die nicht auf Zigarettenrauch, sondern vermutlich auf die Exposition mit Umweltgiften, wie beispielsweise Rauch von Kohle- bzw. Holzfeuer oder Dieselabgasen zurückzuführen sind [Salvi, Clin. Chest Med. 2014, 35:17-27].

Derzeit kann die COPD nur symptomatisch behandelt werden. In erster Linie erhalten COPD-Patienten bronchienerweiternde Medikamente, die das Atmen erleichtern. Der Einsatz entzündungshemmender Medikamente ist bislang limitiert.

Neben der beschriebenen chronischen Symptomatik leiden COPD-Patienten häufig unter akut einsetzenden, zeitlich begrenzten Verschlechterungen des ohnehin beeinträchtigten Gesundheitszustandes, die eine zusätzliche Behandlung erforderlich macht [Ewig, Klinikarzt 2013, 42:182-187]. Die Therapie dieser, als akute Exazerbationen bezeichneten Krankheitsschübe beschränkt sich bislang auf die Gabe von Sauerstoff und systemisch verabreichten Kortikosteroiden.

In einer klinischen COPD-Studie wurde gezeigt, dass das antiinflammatorisch wirksame Roflumilast (PDE4-Inhibitor) zu einer Verringerung der Konzentration von PGP in Sputum und Serum der behandelten COPD-Patienten führt [Wells et al., Am. J. Respir. Crit. Care Med. 2015, 192:934-942].

Erhöhte, proinflammatorische PGP-Konzentrationen wurden darüber hinaus unter anderem in der Schocklunge (Akutes, respiratorisches Syndrom, ARDS) und der Hornhautverletzung des Auges beschrieben. [Hahn et al., Sci. Adv. 2015, 1: e1500175; Pfister et al., Invest. Ophthalmol. Vis. Sci. 1998, 39:1744-1750] Auch hier wird ein proentzündlicher Einfluss von PGP formuliert, der zum einen zu erhöhter Permeabilität von Gefäßen, zum anderen, wie bereits oben beschreiben, zu einer vermehrten Rekrutierung von Neutrophilen (Neutrophilie) und damit zu einer gesteigerten Entzündung führt. Da die Bildung von PGP bei entzündlichen Prozessen unmittelbar mit der Zerstörung von Geweben verbunden ist, und PGP wiederum die Entzündung fördert, ist eine Beteiligung von PGP an sich selbsterhaltenden, chronisch-entzündlichen Prozessen wahrscheinlich. Hier ist insbesondere die COPD bzw. die akut exazerbierende COPD (AE-COPD) zu nennen, der eine chronische Inflammation zugrunde liegt [Russell et al., Curr Opin Pilm Med. 2016, 22:91-99; Anzueto, Eur. Respir. Rev. 2010, 19:113-118]. Aber auch andere, chronisch-entzündliche Erkrankungen und Wundheilungsstörungen der Lunge und anderer Gewebe und Organe, wie z. B. der Haut, des Auges, der Blutgefäße, von Bindegeweben, des Skelettes oder der Muskulatur könnten von einer Reduzierung der PGP-Konzentrationen durch die PREP-Inhibierung profitieren.

Potentielle Anwendungsgebiete für PREP-Inhibitoren sind akute und chronische, pathologische Prozesse bei denen PREP bzw. Substrate und Produkte von PREP beteiligt sind. Da diese anti-entzündliche Wirkung der PREP-Inhibition vermutlich nicht direkt in die Funktion des Immunsystems eingreift, und daher möglicherweise keine immunsupressive Wirkung zu erwarten ist, könnte ein Vorteil der PREP-Inhibiton darin bestehen, dass diesbezüglich weniger Nebenwirkungen bei behandelten Patienten auftreten könnten als mit klassischen immunmodulierenden Wirkprinzipien. Da PGP über das konstitutiv exprimierte, d. h. ständig vorhandene Enzym PREP aus Kollagenfragmenten gebildet wird, und die PGP-Produktion daher nicht vom Immunsystem kontrolliert bzw. reguliert wird, ist zu erwarten, dass die antiinflammatorische Wirkung auch in Patienten wirkt, bei denen etwa aufgrund von Resistenzen, die Wirksamkeit gegen Kortikosteroide verringert ist. Kortikosteroidresistenzen sind insbesondere bei COPD-Patienten in stabilen Phasen außerhalb von akuten Exazerbationen beschrieben. Des Weiteren ist eine additive bzw. synergistische Wirksamkeit von der Kombination mit Kortikosteroiden auch bei vollständig vorhandener, sowie bei eingeschränkter oder stark verminderter Wirkung von Kortikosteroiden zu erwarten. Ebenso ist eine Kombination mit sämtlichen anderen inflammatorischen Wirkmechanismen möglich.

Da PGP, dessen Bildung durch PREP-Inhibition verhindert wird, vermutlich in sämtlichen Erkrankungen mit inflammatorischen Komponenten und Beteiligung des Kollagens bzw. dessen Fragmente eine treibende Rolle beim Entzündungsgeschehen hat, kann eine PREP-Inhibition positive Einflüsse auf viele Erkrankungen wie Autoimmunerkrankungen, chronisch-entzündliche Erkrankungen wie rheumatoide Erkrankungen, infektiösen Geschehen, degenerativen Prozessen (Haut, Organe, Knochen, Muskulatur) haben.

Die Aufgabe der vorliegenden Erfindung war die Identifizierung und Bereitstellung neuer, niedermolekularer Verbindungen, die als potente Inhibitoren des Enzyms Prolylendopeptidase (PREP, Prolyloligopeptidase, PE, POP) wirken, und bei akuten oder chronischen, pathologischen bzw. entzündlich-degenerativen Prozessen über die Reduktion der PREP-abhängigen PGP-Produktion vor allem die Rekrutierung von neutrophilen Granulozyten in Organe, insbesondere der Lunge reduzieren.

In der Anmeldung WO2006052962A2 werden bicyclische Triazole zur Bekämpfung von über Integrin-Inhibition verlaufende Krankheiten beschrieben. 1,2-Dihydro-3H-indazol-3-one als NOX-Inhibitoren zur Bekämpfung von Krankheiten wie COPD, Alzheimer, inflammatorischen oder fibrotischen Erkrankungen sind aus WO2011062864A2 bekannt. In J.Med.Chem 2016; 9 , 4221-4234 werden Hexadyro-1*H*-isoindole als PREP Inhibioren offenbart.

Gegenstand der vorliegenden Erfindung sind Verbindungen der allgemeinen Formel (I) 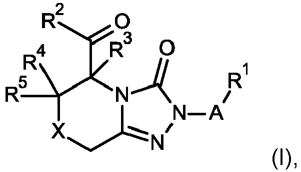 in welcher
- A: für (C₁-C₄)-Alkylen oder CD₂ steht,
wobei (C₁-C₄)-Alkylen mit Hydroxy, (C₁-C₄)-Alkoxy sowie bis zu fünfmal mit Fluor substituiert sein kann,
oder
für eine Gruppe der Formel 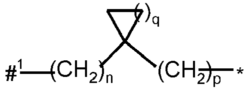 steht, worin
n für 0 oder 1 steht,
p für 0 oder 1 steht,
q für 1 oder 2 steht,
wobei
#¹ die Verknüpfung zum Stickstoffatom des 5-Oxo-4,5-dihydro-1H-1,2,4-triazol-1-yl-Ringes markiert,
* die Verknüpfung zu R¹ markiert,
- X: für eine Gruppe der Formel 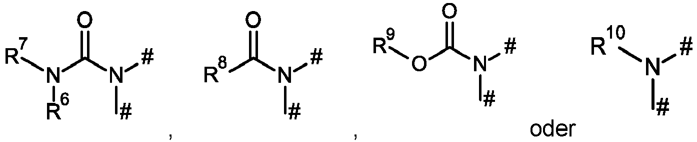 steht,
wobei
# die Verknüpfung zu dem jeweiligen Kohlenstoffatom des Ringes markiert,
R⁶ für (C₁-C₆)-Alkyl, (C₃-C₆)-Cycloalkyl, Phenyl oder 5- bis 6-gliedriges Heteroaryl steht,
worin (C₁-C₆)-Alkyl mit Hydroxy, Cyano, (C₁-C₄)-Alkoxy oder bis zu fünfmal mit Fluor substituiert sein kann,
worin (C₃-C₆)-Cycloalkyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin Phenyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin 5- bis 6-gliedriges Heteroaryl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
R⁷ für Wasserstoff oder (C₁-C₄)-Alkyl steht,
worin (C₁-C₄)-Alkyl bis zu fünfmal mit Fluor substituiert sein kann,
R⁸ für (C₁-C₄)-Alkyl, (C₃-C₆)-Cycloalkyl, Phenyl oder 5- bis 6-gliedriges Heteroaryl steht,
worin (C₁-C₄)-Alkyl mit Hydroxy, Cyano, (C₁-C₄)-Alkoxyoder bis zu fünfmal mit Fluor substituiert sein kann,
worin (C₃-C₆)-Cycloalkyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin Phenyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann, w
orin 5- bis 6-gliedriges Heteroaryl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
R⁹ für (C₁-C₄)-Alkyl, (C₃-C₆)-Cycloalkyl oder Phenyl steht, worin (C₁-C₄)-Alkyl bis zu fünfmal mit Fluor substituiert sein kann,
worin (C₃-C₆)-Cycloalkyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin Phenyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
R¹⁰ für Wasserstoff, (C₁-C₄)-Alkyl, (C₃-C₆)-Cycloalkyl oder Phenyl steht,
worin (C₁-C₄)-Alkyl mit Hydroxy, Cyano, (C₁-C₄)-Alkoxy, (C₃-C₆)-Cycloalkyl oder bis zu fünfmal mit Fluor substituiert sein kann,
worin (C₃-C₆)-Cycloalkyl bis zu viermal mit Fluor substituiert sein kann,
worin (C₃-C₆)-Cycloalkyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin Phenyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin 5- bis 6-gliedriges Heteroaryl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann, oder
für Sauerstoff steht,
- R¹: für (C₃-C₇)-Cycloalkyl, Phenyl oder 5- bis 10-gliedriges Heteroaryl steht,
wobei (C₃-C₇)-Cycloalkyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin (C₁-C₄)-Alkyl bis zu fünfmal mit Fluor substituiert sein kann,
wobei Phenyl mit 1 bis 4 Substituenten unabhängig voneinander ausgewählt aus der Gruppe Halogen, Cyano, (C₁-C₄)-Alkyl, (C₃-C₆)-Cycloalkyl, Trifluormethyl, (C₁-C₄)-Alkoxy, (C₃-C₅)-Cycloalkoxy, Monofluormethoxy, Difluormethoxy, Trifluormethoxy, Hydroxycarbonyl, (C₁-C₄)-Alkoxycarbonyl, Aminocarbonyl, Mono-(C₁-C₄)-alkylaminocarbonyl, Di-(C₁-C₄)-alkylaminocarbonyl, (C₃-C₅)-Cycloalkyl-aminocarbonyl, (C₁-C₄)-Alkylsulfanyl, (C₁-C₄)-Alkylsulfonyl, (C₁-C₄)-Alkylsulfonimidoyl, (C₁-C₄)-Cycloalkylsulfonyl, Aminosulfonyl, Mono-(C₁-C₄)-alkylaminosulfonyl, Di-(C₁-C₄)-alkylaminosulfonyl, (C₁-C₄)-Alkylsulfinyl, Amino, Mono-(C₁-C₄)-alkylamino oder Di-(C₁-C₄)-alkylamino substituiert ist,
oder
wobei 5- bis 10-gliedriges Heteroaryl mit 1 bis 4 Substituenten unabhängig voneinander ausgewählt aus der Gruppe Halogen, Cyano, (C₁-C₄)-Alkyl, (C₃-C₆)-Cycloalkyl, Trifluormethyl, (C₁-C₄)-Alkoxy, (C₃-C₅)-Cycloalkoxy, Difluormethoxy, Trifluormethoxy, 2,2,2-Trifluorethoxy, Phenyl, Hydroxycarbonyl, (C₁-C₄)-Alkoxycarbonyl, Aminocarbonyl, Mono-(C₁-C₄)-alkylaminocarbonyl, Di-(C₁-C₄)-alkylaminocarbonyl, (C₃-C₅)-Cycloalkyl-aminocarbonyl, (C₁-C₄)-Alkylsulfanyl, (C₁-C₄)-Alkylsulfonyl, (C₁-C₄)-Alkylsulfonimidoyl, (C₁-C₄)-Cycloalkylsulfonyl, Aminosulfonyl, Mono-(C₁-C₄)-alkylaminosulfonyl, Di-(C₁-C₄)-alkylaminosulfonyl, (C₁-C₄)-Alkylsulfinyl, Amino, Mono-(C₁-C₄)-alkylamino oder Di-(C₁-C₄)-alkylamino substituiert sein kann,
worin Phenyl mit Methyl, Ethyl, Chlor, Fluor oder Methoxy substituiert sein kann,
- R²: für eine Gruppe der Formel 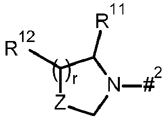 steht, wobei
#² die Verknüpfung zum Carbonyl-Kohlenstoffatom markiert,
r für 0 oder 1 steht,
Z für O, NR¹⁴, S, SO, SO₂ oder CR^{13A}R^{13B} steht,
worin
R^{13A} für Wasserstoff, Halogen, Cyano, (C₁-C₄)-Alkyl, (C₃-C₆)-Cycloalkyl, Trifluormethyl, Difluormethyl, Fluormethyl, Hydroxy, (C₁-C₄)-Alkoxy, (C₃-C₅)-Cycloalkoxy, Difluormethoxy, Trifluormethoxy oder 2,2,2-Trifluorethoxy steht,
worin (C₁-C₄)-Alkyl mit Hydroxy, Amino, Mono-(C₁-C₄)-alkylamino oder Di-(C₁-C₄)-alkylamino substituiert sein kann,
R^{13B} für Wasserstoff, Fluor oder (C₁-C₄)-Alkyl, steht,
worin (C₁-C₄)-Alkyl bis zu fünfmal mit Fluor substituiert sein kann,
R¹⁴ für Wasserstoff oder Methyl steht,
R¹¹ für Wasserstoff, Cyano, (C₁-C₄)-Alkyl, Acetyl oder Formyl steht,
worin (C₁-C₄)-Alkyl mit Hydroxy oder bis zu fünfmal mit Fluor substituiert sein kann,
worin Acetyl mit Hydroxy oder bis zu dreimal mit Fluor substituiert sein kann,
R¹² für Wasserstoff, Fluor oder (C₁-C₄)-Alkyl steht,
oder
R¹¹ und R¹² zusammen mit den Kohlenstoffatomen, an die sie gebunden sind, einen Cyclopropyl- oder Cyclobutyl-Ring bilden,
worin der Cyclopropyl- oder Cyclobutyl-Ring bis zu zweimal mit Fluor substituiert sein kann, oder
R¹² und R^{13A} zusammen mit den Kohlenstoffatomen, an die sie gebunden sind, einen Cyclopropyl- oder Cyclobutyl-Ring bilden,
worin der Cyclopropyl- oder Cyclobutyl -Ring bis zu zweimal mit Fluor substituiert sein kann,
oder
R^{13A} und R^{13B} zusammen mit dem Kohlenstoffatom, an die sie gebunden sind, einen Cyclopropyl- oder Cyclobutyl-Ring bilden,
worin der Cyclopropyl- oder Cyclobutyl-Ring bis zu zweimal mit Fluor substituiert sein kann,
wobei R¹², R^{13A} und R^{13B} für Wasserstoff stehen, wenn R¹¹ nicht für Wasserstoff steht,
wobei R¹¹ für Wasserstoff steht, wenn einer der Substituenten R¹², R^{13A} oder R^{13B} nicht für Wasserstoff steht,
oder
für eine Gruppe der Formel 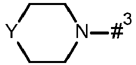 steht, wobei
#³ die Verknüpfung zum Carbonyl-Kohlenstoffatom markiert,
Y für NR¹⁵, CR^{16A}R^{16B}, Sauerstoff oder Schwefel steht,
worin
R¹⁵ für Wasserstoff oder Methyl steht,
R^{16A} für Wasserstoff oder Methyl steht,
R^{16B} für Wasserstoff oder Methyl steht,
- R³: für Wasserstoff steht,
- R⁴: für Wasserstoff oder (C₁-C₄)-Alkyl steht, worin (C₁-C₄)-Alkyl mit (C₁-C₄)-Alkoxy, Hydroxy, Mono-(C₁-C₄)-alkylamino oder Di-(C₁-C₄)-alkylamino sowie bis zu fünfmal mit Fluor substituiert sein kann,
- R⁵: für Wasserstoff steht,
sowie die Salze, Solvate und Solvate der Salze der Verbindungen der Formel (I).

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.Im Falle von Carbonsäuren als Zwischen- oder Endprodukten kann alternativ auch eine Trennung über diastereomere Salze mit Hilfe chiraler Amin-Basen erfolgen.

Sofern die erfindungsgemäßen Verbindungen in tautomeren Formen vorkommen können, umfasst die vorliegende Erfindung sämtliche tautomere Formen.

Die Verbindungen der allgemeinen Formel (I) können als isotopische Varianten vorliegen. Die Erfindung umfasst daher eine oder mehrere isotopische Varianten der Verbindungen der allgemeinen Formel (I), insbesondere deuteriumhaltige Verbindungen der allgemeinen Formel (I).

Der Begriff "isotopische Variante" einer Verbindung oder eines Reagenzes ist definiert als eine Verbindung mit einem unnatürlichen Anteil eines oder mehrerer der Isotope, aus denen eine derartige Verbindung aufgebaut ist.

Der Begriff "isotopische Variante der Verbindung der allgemeinen Formel (I)" ist definiert als eine Verbindung der allgemeinen Formel (I) mit einem unnatürlichen Anteil eines oder mehrerer der Isotope, aus denen eine derartige Verbindung aufgebaut ist.

Unter dem Ausdruck "unnatürlicher Anteil" ist ein Anteil eines derartigen Isotops zu verstehen, der höher als dessen natürliche Häufigkeit ist. Die in diesem Zusammenhang anzuwendenden natürlichen Häufigkeiten von Isotopen finden sich in "Isotopic Compositions of the Elements 1997", Pure Appl. Chem., 70(1), 217-235, 1998.

Beispiele für derartige Isotope sind stabile und radioaktive Isotope von Wasserstoff, Kohlenstoff, Stickstoff, Sauerstoff, Phosphor, Schwefel, Fluor, Chlor, Brom und lod, wie ²H (Deuterium), ³H (Tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁵I, ¹²⁹I bzw. ¹³¹I.

Im Hinblick auf die Behandlung und/oder Prophylaxe der hier angegebenen Störungen enthalten die isotopischen Variante(n) der Verbindungen der allgemeinen Formel (I) vorzugsweise Deuterium ("deuteriumhaltige Verbindungen der allgemeinen Formel (I)"). Isotopische Varianten der Verbindungen der allgemeinen Formel (I), in die ein oder mehrere radioaktive Isotope, wie ³H oder ¹⁴C, eingebaut sind, sind beispielsweise bei Arzneistoff- und/oder Substratsgewebeverteilungsstudien von Nutzen. Diese Isotope sind wegen ihrer leichten Einbaubarkeit und Nachweisbarkeit besonders bevorzugt. In eine Verbindung der allgemeinen Formel (I) können Positronen emittierende Isotope wie ¹⁸F oder ¹¹C eingebaut werden. Diese isotopischen Varianten der Verbindungen der allgemeinen Formel (I) eignen sich zur Verwendung bei in-vivo-Bildgebungsanwendungen. Deuteriumhaltige und ¹³C-haltige Verbindungen der allgemeinen Formel (I) können im Rahmen präklinischer oder klinischer Studien bei Massenspektrometrie-Analysen verwendet werden.

Isotopische Varianten der Verbindungen der allgemeinen Formel (I) können im Allgemeinen nach dem Fachmann bekannten Verfahren wie den in den hier beschriebenen Schemata und/oder Beispielen beschrieben hergestellt werden, indem man ein Reagenz durch eine isotopische Variante des Reagenzes, vorzugsweise ein deuteriumhaltiges Reagenz, ersetzt. Je nach den gewünschten Deuterierungsstellen kann in einigen Fällen Deuterium aus D₂O entweder direkt in die Verbindungen oder in Reagenzien, die für die Synthese derartiger Verbindungen verwendet werden können, eingebaut werden. Ein nützliches Reagenz zum Einbau von Deuterium in Moleküle ist auch Deuteriumgas. Eine schnelle Route zum Einbau von Deuterium ist die katalytische Deuterierung von olefinischen Bindungen und acetylenischen Bindungen. Zum direkten Austausch von Wasserstoff gegen Deuterium in funktionelle Gruppen enthaltenden Kohlenwasserstoffen können auch Metallkatalysatoren (d.h. Pd, Pt und Rh) in Gegenwart von Deuteriumgas verwendet werden. Verschiedene deuterierte Reagenzien und Synthesebausteine sind im Handel von Firmen wie beispielsweise C/D/N Isotopes, Quebec, Kanada; Cambridge Isotope Laboratories Inc., Andover, MA, USA; und CombiPhos Catalysts, Inc., Princeton, NJ, USA, erhältlich.

Der Begriff "deuteriumhaltige Verbindung der allgemeinen Formel (I)" ist definiert als eine Verbindung der allgemeinen Formel (I), in der ein oder mehrere Wasserstoffatome durch ein oder mehrere Deuteriumatome ersetzt sind und in der die Häufigkeit von Deuterium in jeder deuterierten Position der Verbindung der allgemeinen Formel (I) höher ist als die natürliche Häufigkeit von Deuterium, die etwa 0,015% beträgt. Insbesondere ist in einer deuteriumhaltigen Verbindung der allgemeinen Formel (I) die Häufigkeit von Deuterium in jeder deuterierten Position der Verbindung der allgemeinen Formel (I) höher als 10%, 20%, 30%, 40%, 50%, 60%, 70% oder 80%, vorzugsweise höher als 90%, 95%, 96% oder 97%, noch weiter bevorzugt höher als 98% oder 99% in dieser Position bzw. diesen Positionen. Es versteht sich, dass die Häufigkeit von Deuterium in jeder deuterierten Position von der Häufigkeit von Deuterium in anderen deuterierten Positionen unabhängig ist.

Durch den selektiven Einbau eines oder mehrerer Deuteriumatome in eine Verbindung der allgemeinen Formel (I) können die physikalisch-chemischen Eigenschaften (wie beispielsweise Azidität [C. L. Perrin, et al., J. Am. Chem. Soc., 2007, 129, 4490], Basizität [C. L. Perrin et al., J. Am. Chem. Soc., 2005, 127, 9641], Lipophilie [B. Testa et al., Int. J. Pharm., 1984, 19(3), 271]) und/oder das Stoffwechselprofil des Moleküls verändert und Veränderungen des Verhältnisses von Stammverbindung zu Metaboliten oder der gebildeten Mengen von Metaboliten verursacht werden. Derartige Veränderungen können zu bestimmten therapeutischen Vorteilen führen und daher unter bestimmten Umständen bevorzugt sein. Es ist über verringerte Stoffwechselraten und Stoffwechselumschaltung, bei der sich das Verhältnis von Metaboliten ändert, berichtet worden (A. E. Mutlib et al., Toxicol. Appl. Pharmacol., 2000, 169, 102). Diese Veränderungen der Exposition gegenüber Stammverbindung und Metaboliten können wichtige Konsequenzen hinsichtlich Pharmakodynamik, Verträglichkeit und Wirksamkeit einer deuteriumhaltigen Verbindung der allgemeinen Formel (I) haben. In einigen Fällen wird durch den Deuteriumersatz die Bildung eines unerwünschten oder toxischen Metaboliten verringert oder eliminiert und die Bildung eines gewünschten Metaboliten verstärkt (z.B. Nevirapin: A. M. Sharma et al., Chem. Res. Toxicol., 2013, 26, 410; Efavirenz: A. E. Mutlib et al., Toxicol. Appl. Pharmacol., 2000, 169, 102). In anderen Fällen besteht der Haupteffekt der Deuterierung darin, die Geschwindigkeit der systemischen Clearance zu verringern. Dadurch wird die biologische Halbwertszeit der Verbindung erhöht. Zu den potentiellen klinischen Vorteilen gehören die Fähigkeit zur Aufrechterhaltung einer ähnlichen systemischen Exposition mit verringerten Spitzenspiegeln und erhöhten Talspiegeln. Dies könnte je nach der Pharmakokinetik/Pharmakodynamik-Beziehung der jeweiligen Verbindung zu geringeren Nebenwirkungen und erhöhter Wirksamkeit führen. Beispiele für diesen Deuterium-Effekt sind ML-337 (C. J. Wenthur et al., J. Med. Chem., 2013, 56, 5208) und Odanacatib (K. Kassahun et al., WO2012/112363). Es ist auch noch über weitere Fälle berichtet worden, bei denen verringerte Verstoffwechslungsraten zu einer Erhöhung der Arzneistoffexposition ohne Änderung der Rate der systemischen Clearance führen (z.B. Rofecoxib: F. Schneider et al., Arzneim. Forsch. / Drug. Res., 2006, 56, 295; Telaprevir: F. Maltais et al., J. Med. Chem., 2009, 52, 7993). Deuterierte Arzneistoffe, die diesen Effekt zeigen, können verringerte Dosierungsanforderungen haben (z.B. geringere Zahl von Dosen oder niedrigere Dosierung zur Erzielung der gewünschten Wirkung) und/oder zu geringeren Metabolitenbelastungen führen.

Eine Verbindung der allgemeinen Formel (I) kann mehrere potenzielle Angriffsstellen für die Verstoffwechslung aufweisen. Zur Optimierung der oben beschriebenen Effekte auf die physikalisch-chemischen Eigenschaften und das Stoffwechselprofil können deuteriumhaltige Verbindungen der allgemeinen Formel (I) mit einem bestimmten Muster eines oder mehrerer Deuterium-Wasserstoff-Austausche gewählt werden. Insbesondere ist/sind das Deuteriumatom/die Deuteriumatome deuteriumhaltiger Verbindung(en) der allgemeinen Formel (I) an ein Kohlenstoffatom gebunden und/oder steht/stehen in den Positionen der Verbindung der allgemeinen Formel (I), bei denen es sich um Angriffsstellen für Verstoffwechslungsenzyme wie z.B. Cytochrom P₄₅₀ handelt.

Außerdem werden auch Prodrugs der erfindungsgemäßen Verbindungen beschrieben. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl, 2-Ethylbutyl, 1,4-Dimethylpentyl, 4,4-Dimethylpentyl und 1,4,4-Trimethylpentyl.
Alkylcarbonyl stehen im Rahmen der Erfindung für einen linearen oder verzweigten Alkylrest mit der jeweils angegebenen Anzahl an Kohlenstoffatomen und einer am Kohlenstoffatom angebundenen Carbonylgruppe. Beispielhaft und vorzugsweise seien genannt: Methylcarbonyl, Ethylcarbonyl, n-Propylcarbonyl, Isopropylcarbonyl und tert.-Butylcarbonyl.
Alkoxy steht im Rahmen der Erfindung für einen linearen oder verzweigten Alkoxyrest mit der jeweils angegebenen Anzahl an Kohlenstoffatomen. Beispielhaft und vorzugsweise seien genannt: Methoxy, Ethoxy, n-Propoxy, Isopropoxy, n-Butoxy und tert.-Butoxy.
Cycloalkoxy steht im Rahmen der Erfindung für einen einen monocyclischen, gesättigten Alkylrest mit der jeweils angegebenen Anzahl an Ring-Kohlenstoffatomen, der über ein Sauerstoffatom verknüpft ist. Beispielhaft und vorzugsweise seien genannt: Cyclopropyloxy, Cyclobutyloxy, Cyclopentyloxy und Cyclohexyloxy.
Cycloalkyl bzw. Carbocyclus steht in Rahmen der Erfindung für einen monocyclischen, gesättigten Alkylrest mit der jeweils angegebenen Anzahl an Ring-Kohlenstoffatomen. Beispielhaft und vorzugsweise seien genannt: Cyclopropyl, Cyclobutyl, Cyclopentyl, Cyclohexyl und Cycloheptyl.
Cycloalkylcarbonyl stehen im Rahmen der Erfindung für einen einen monocyclischen, gesättigten Alkylrest mit der jeweils angegebenen Anzahl an Ring-Kohlenstoffatomen und einer am Kohlenstoffatom angebundenen Carbonylgruppe. Beispielhaft und vorzugsweise seien genannt: Cyclopropylcarbonyl, Cyclobutylcarbonyl, Cyclopentylcarbonyl, Cyclohexylcarbonyl und Cycloheptylcarbonyl.
Alkoxycarbonyl stehen im Rahmen der Erfindung für einen linearen oder verzweigten Alkoxyrest mit der jeweils angegebenen Anzahl an Kohlenstoffatomen und einer am Sauerstoffatom angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl. Heterocyclus bzw Heterocyclyl steht im Rahmen der Erfindung für einen monocyclischen oder bicyclischen, gesättigten Heterocyclus mit der jeweils angegebenen Anzahl an Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Tetrahydrothiopyranyl, Morpholinyl, Thiomorpholinyl, Hexahydroazepinyl und Hexahydro-1,4-diazepinyl. Bevorzugt sind Azetidinyl, Oxetanyl, Pyrrolidinyl, Tetrahydrofuranyl, Piperidinyl, Piperazinyl, Tetrahydropyranyl und Morpholinyl.
Aza-Heterocvclyl steht im Rahmen der Erfindung für einen monocyclischen oder bicyclischen, gesättigten oder teilweise ungesättigten Heterocyclus mit mit der jeweils angegebenen Anzahl an Ringatomen, der ein Stickstoffatom enthält und darüberhinaus ein oder zwei weitere Ring-Heteroatome aus der Reihe N, O, S, SO und/oder SO₂ enthalten kann und über ein Ring-Stickstoffatom verknüpft ist. Beispielhaft seien genannt: Pyrrolidinyl, Pyrazolidinyl, Piperidinyl, Piperazinyl, Morpholinyl, Thiomorpholinyl, 1,1-Dioxothiomorpholinyl, Hexahydroazepinyl, Hexahydro-1,4-diazepinyl, 1,2,3,4-Tetrahydroisochinolinyl, 1,2,3,4-Tetrahydrochinolinyl, Indolinyl, 8-Azabicyclo[3.2.1]octanyl, 9-Azabicyclo[3.3.1]nonanyl, 3-Azabicyclo[4.1.0]heptanyl und Quinuclidinyl.
Heteroaryl steht im Rahmen der Erfindung für einen monocyclischen oder bicyclischen aromatischen Heterocyclus (Heteroaromaten) mit der jeweils angegebenen Anzahl an Ringatomen, der bis zu vier gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Chinolinyl, Thiazolyl, Oxazolyl, Isoxazolyl, Isothiazolyl, Triazolyl, Oxadiazolyl, Thiadiazolyl, Tetrazolyl, Pyridyl, Pyrimidinyl, Pyridazinyl, Pyrazinyl und Triazinyl.
Mono-alkylaminocarbonyl steht im Rahmen der Erfindung für eine Amino-Gruppe, die über eine Carbonylgruppe verknüpft ist und die einen linearen oder verzweigten Alkylsubstituenten mit der jeweils angegebenen Anzahl an Kohlenstoffatomen aufweist. Beispielhaft und vorzugsweise seien genannt: Methylaminocarbonyl, Ethylaminocarbonyl, *n*-Propylaminocarbonyl, Isopropylaminocarbonyl, *n*-Butylaminocarbonyl, *tert*.-Butylaminocarbonyl, *n*-Pentylaminocarbonyl und *n*-Hexylaminocarbonyl.
Di-alkylaminocarbonyl steht im Rahmen der Erfindung für eine Amino-Gruppe, die über eine Carbonylgruppe verknüpft ist und die zwei gleiche oder verschiedene lineare oder verzweigte Alkylsubstituenten mit der jeweils angegebenen Anzahl an Kohlenstoffatomen aufweist. Beispielhaft und vorzugsweise seien genannt: *N*,*N*-Dimethylaminocarbonyl, *N*,*N*-Diethylaminocarbonyl, *N*-Ethyl-*N*-methylaminocarbonyl, *N*-Methyl-*N*-*n*-propylaminocarbonyl, *N*-*n*-Butyl-*N-*methylaminocarbonyl,*N*-tert.-Butyl-*N*-methylaminocarbonyl,*N*-*n*-Pentyl-*N*-methylaminocarbonyl und *N*-*n*-Hexyl-*N*-methylaminocarbonyl.
Mono-alkylaminosulfonyl steht im Rahmen der Erfindung für eine Amino-Gruppe, die über eine Sulfonylgruppe verknüpft ist und die einen linearen oder verzweigten Alkylsubstituenten mit der jeweils angegebenen Anzahl an Kohlenstoffatomen aufweist. Beispielhaft und vorzugsweise seien genannt: Methylaminosulfonyl, Ethylaminosulfonnyl, *n*-Propylaminosulfonyl, Isopropylaminosulfonyl, *n*-Butylaminosulfonyl, *tert*.-Butylaminosulfonyl, *n*-Pentylaminosulfonyl und *n*-Hexylaminosulfonyl.
Di-alkylaminosulfonyl steht im Rahmen der Erfindung für eine Amino-Gruppe, die über eine Sulfonylgruppe verknüpft ist und die zwei gleiche oder verschiedene lineare oder verzweigte Alkylsubstituenten mit der jeweils angegebenen Anzahl an Kohlenstoffatomen aufweist. Beispielhaft und vorzugsweise seien genannt: *N*,*N*-Dimethylaminosulfonyl, *N,N*-Diethylaminosulfonyl, *N*-Ethyl-*N*-methylaminosulfonyl, *N*-Methyl-*N*-*n*-propylaminosulfonyl, *N*-*n*-Butyl-*N-*methylaminosulfonyl, *N*-tert.-Butyl-*N*-methylaminosulfonyl, *N*-*n*-Pentyl-*N*-methylaminosulfonyl und *N*-*n*-Hexyl-*N*-methylaminosulfonyl.
Mono-alkylamino steht im Rahmen der Erfindung für eine Amino-Gruppe, die einen linearen oder verzweigten Alkylsubstituenten mit der jeweils angegebenen Anzahl an Kohlenstoffatomen aufweist. Beispielhaft und vorzugsweise seien genannt: Methylamino, Ethylamino, *n-*Propylamino, Isopropylamino, *n*-Butylamino, *tert*.-Butylamino, *n*-Pentylamino und *n-*Hexylamino.
Di-alkylamino steht im Rahmen der Erfindung für eine Amino-Gruppe, die zwei gleiche oder verschiedene lineare oder verzweigte Alkylsubstituenten mit der jeweils angegebenen Anzahl an Kohlenstoffatomen aufweist. Beispielhaft und vorzugsweise seien genannt: *N,N-*Dimethylamino, *N*,*N*-Diethylamino, *N*-Ethyl-*N*-methylamino, *N*-Methyl-*N*-*n*-propylamino, *N-n-*Butyl-*N*-methylamino, *N*-tert.-Butyl-*N*-methylamino, *N*-*n*-Pentyl-*N*-methylaminol und *N*-*n*-Hexyl-*N*-methylamino.
Halogen schließt im Rahmen der Erfindung Fluor, Chlor, Brom und lod ein. Bevorzugt sind Chlor oder Fluor.
Eine Oxo-Gruppe steht im Rahmen der Erfindung für ein Sauerstoffatom, das über eine Doppelbindung an ein Kohlenstoffatom gebunden ist.

In den Formeln der Gruppe, für die A, X und R² stehen können, steht der Endpunkt der Linie, an dem ein Zeichen #¹ bzw. #² bzw. #³ bzw. #⁴ bzw. #⁵ bzw. * bzw. ** bzw. *** steht, nicht für ein Kohlenstoffatom beziehungsweise eine CH₂-Gruppe, sondern ist Bestandteil der Bindung zu dem jeweils bezeichneten Atom, an das A bzw. X bzw. R² gebunden sind.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit einem oder zwei gleichen oder verschiedenen Substituenten ist bevorzugt. Ganz besonders bevorzugt ist die Substitution mit einem Substituenten.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Bevorzugt im Rahmen der vorliegenden Erfindung sind Verbindungen der allgemeinen Formel (I), in welcher
- A: für (C₁-C₄)-Alkylen steht,
- X: für eine Gruppe der Formel 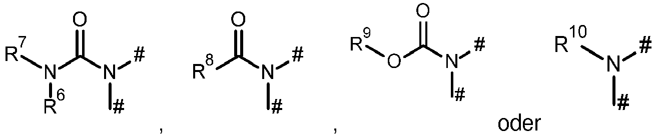 steht,
wobei
# die Verknüpfung zu dem jeweiligen Kohlenstoffatom des Ringes markiert,
R⁶ für (C₁-C₆)-Alkyl, (C₃-C₅)-Cycloalkyl, Phenyl oder 6-gliedriges Heteroaryl steht,
worin (C₁-C₆)-Alkyl bis zu dreimal mit Fluor substituiert sein kann,
worin (C₃-C₅)-Cycloalkyl bis zu dreimal mit Fluor substituiert sein kann,
worin Phenyl mit 1 bis 2 Substituenten unabhängig voneinander ausgewählt aus der Gruppe Fluor, Chlor Brom, Cyano, Trifluormethyl, Methyl, Ethyl, Methoxy und Ethoxy substituiert sein kann, worin 6-gliedriges Heteroaryl mit 1 bis 2 Substituenten unabhängig voneinander ausgewählt aus der Gruppe Fluor, Chlor, Cyano, Trifluormethyl, Methyl, Ethyl, Methoxy und Ethoxy substituiert sein kann,
R⁷ für Wasserstoff oder Methyl steht,
worin Methyl bis zu dreimal mit Fluor substituiert sein kann,
R⁸ für (C₁-C₄)-Alkyl, (C₃-C₆)-Cycloalkyl, Phenyl oder 6-gliedriges Heteroaryl steht,
worin (C₁-C₆)-Alkyl bis zu dreimal mit Fluor substituiert sein kann,
worin (C₃-C₅)-Cycloalkyl bis zu dreimal mit Fluor substituiert sein kann,
R⁹ für (C₁-C₄)-Alkyl oder Phenyl steht,
worin (C₁-C₄)-Alkyl bis zu dreimal mit Fluor substituiert sein kann,
R¹⁰ für Wasserstoff, (C₁-C₄)-Alkyl, (C₃-C₆)-Cycloalkyl oder Phenyl steht,
worin (C₁-C₄)-Alkyl mit Cyclopropyl oder Cyclobuytl oder bis zu fünfmal mit Fluor substituiert sein kann,
worin Cyclopropyl oder Cyclobuytl bis zu zweimal mit Fluor substituiert sein kann,
worin (C₃-C₆)-Cycloalky bis zu dreimal mit Fluor substituiert sein kann,
worin Phenyl mit 1 bis 3 Substituenten unabhängig voneinander ausgewählt aus der Gruppe Fluor, Chlor Brom, Cyano, Trifluormethyl, Methyl, Ethyl, Methoxy und Ethoxy substituiert sein kann, oder
für Sauerstoff steht,
- R¹: für Phenyl oder 6-gliedriges Heteroaryl steht,
wobei Phenyl mit 1 bis 3 Substituenten unabhängig voneinander ausgewählt aus der Gruppe Fluor, Chlor, Brom, Cyano, Methyl, Ethyl, Cyclopropyl, Trifluormethyl, Methoxy, Ethoxy, Monofluormethoxy, Difluormethoxy, Trifluormethoxy, Hydroxycarbonyl, Hydroxycarbonyl, Methoxycarbonyl, Ethoxycarbonyl, tert-Butoxycarbonyl, Methylaminocar-bBbonyl, bonyl,bonyl Dimethylaminocarbonyl substituiert ist,
wobei 6-gliedriges Heteroaryl mit 1 bis 3 Substituenten unabhängig voneinander ausaus der Gruppe Fluor, Chlor, Brom, Cyano, Methyl, Ethyl, Cyclopropyl, Trifluormethyl, Methoxy, Ethoxy, Monofluormethoxy, Difluormethoxy, Trifluormethoxy, Hydroxycarbonyl, Hydroxycarbonyl, Methoxycarbonyl, Ethoxycarbonyl, tert-Butoxycarbonyl, Methylaminocarbonyl, Dimethylaminocarbonyl substituiert ist,
- R²: für eine Gruppe der Formel 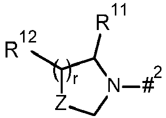 steht, wobei
#² die Verknüpfung zum Carbonyl-Kohlenstoffatom markiert,
r für 1 steht,
Z für CR^{13A}R^{13B} steht,
worin
R^{13A} für Wasserstoff, Fluor, Methyl, Trifluormethyl, Difluormethyl, Fluormethyl, Hydroxy, Methoxy, Difluormethoxy oder Trifluormethoxy steht,
R^{13B} für Wasserstoff oder Fluor steht,
R¹¹ für Wasserstoff steht,
R¹² für Wasserstoff oder Fluor steht,
- R³: für Wasserstoff steht,
- R⁴: für Wasserstoff, Fluor oder Methyl, steht,
worin Methyl bis zu dreimal mit Fluor substituiert sein kann,
- R⁵: für Wasserstoff steht,
sowie die Salze, Solvate und Solvate der Salze der Verbindungen der Formel (I).

Besonders bevorzugt im Rahmen der vorliegenden Erfindung sind Verbindungen der allgemeinen Formel (I), in welcher
- A: für -CH₂- steht,
- X: für eine Gruppe der Formel 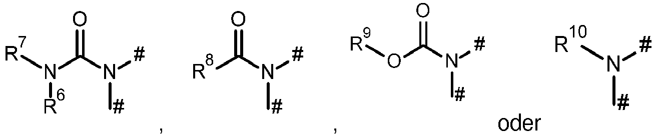 steht,
wobei
# die Verknüpfung zu dem jeweiligen Kohlenstoffatom des Ringes markiert,
R⁶ für (C₁-C₄)-Alkyl, Cyclopropyl oder Phenyl steht,
worin (C₁-C₄)-Alkyl bis zu dreimal mit Fluor substituiert sein kann,
worin Phenyl mit 1 oder 2 Substituenten unabhängig voneinander ausgewählt aus der Gruppe Chlor oder Trifluormethyl substituiert ist,
R⁷ für Wasserstoff steht,
R⁸ für Methyl, Ethyl, Propyl, 3,3,3-Trifluorpropyl oder Cyclopropyl steht,
R⁹ für tert.-Butyl steht,
R¹⁰ für Wasserstoff, Methyl, Ethyl, 2,2,2-Trifluorethyl, Cyclopropyl oder Cyclobutyl steht,
worin Methyl mit Cyclopropyl substituiert sein kann,
worin Cyclopropyl bis zu zweimal mit Fluor substituiert sein kann,
- R¹: für Phenyl oder Pyridyl steht, wobei Phenyl mit 1 oder 2 Substituenten unabhängig voneinander ausgewählt aus der Gruppe Fluor, Methyl oder Trifluormethyl substituiert ist,
wobei Pyridyl mit 1 oder 2 Substituenten unabhängig voneinander ausgewählt aus der Gruppe Fluor, Chlor oder Trifluormethyl substituiert ist,
- R²: für eine Gruppe der Formel 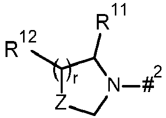 steht, wobei
#² die Verknüpfung zum Carbonyl-Kohlenstoffatom markiert,
r für 1 steht,
Z für CR^{13A}R^{13B} steht,
worin
R^{13A} für Wasserstoff oder Fluor steht,
R^{13B} für Wasserstoff steht,
R¹¹ für Wasserstoff steht,
R¹² für Wasserstoff oder Fluor steht,
- R³: für Wasserstoff steht,
- R⁴: für Wasserstoff oder Methyl steht,
- R⁵: für Wasserstoff steht,
sowie die Salze, Solvate und Solvate der Salze der Verbindungen der Formel (I).

Bevorzugt im Rahmen der vorliegenden Erfindung sind auch Verbindungen der allgemeinen Formel (I), in welcher
- A: für -CH₂-, steht,
sowie ihre Solvate, Salze und Solvate Salze.

Bevorzugt im Rahmen der vorliegenden Erfindung sind auch Verbindungen der allgemeinen Formel (I), in welcher
- X: für eine Gruppe der Formel 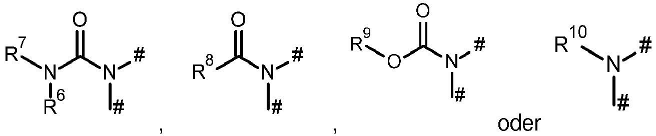 steht,
wobei
# die Verknüpfung zu dem jeweiligen Kohlenstoffatom des Ringes markiert,
R⁶ für (C₁-C₄)-Alkyl, Cyclopropyl oder Phenyl steht,
worin (C₁-C₄)-Alkyl bis zu dreimal mit Fluor substituiert sein kann,
worin Phenyl mit 1 oder 2 Substituenten unabhängig voneinander ausgewählt aus der Gruppe Chlor oder Trifluormethyl substituiert ist,
R⁷ für Wasserstoff steht,
R⁸ für Methyl, Ethyl, Propyl, 3,3,3-Trifluorpropyl oder Cyclopropyl steht,
R⁹ für tert.-Butyl steht,
R¹⁰ für Wasserstoff, Methyl, Ethyl, 2,2,2-Trifluorethyl, Cyclopropyl oder Cyclobutyl steht,
worin Methyl mit Cyclopropyl substituiert sein kann,
worin Cyclopropyl bis zu zweimal mit Fluor substituiert sein kann, sowie ihre Solvate, Salze und Solvate Salze.

Bevorzugt sind im Rahmen der vorliegenden Erfindung auch Verbindungen der Formel (I), in welcher
- X: für eine Gruppe der Formel 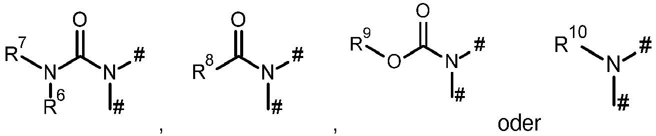 steht,
wobei
# die Verknüpfung zu dem jeweiligen Kohlenstoffatom des Ringes markiert,
R⁶ für (C₁-C₄)-Alkyl, Cyclopropyl oder Phenyl steht,
worin (C₁-C₄)-Alkyl bis zu dreimal mit Fluor substituiert sein kann,
worin Phenyl mit 1 oder 2 Substituenten unabhängig voneinander ausgewählt aus der Gruppe Chlor oder Trifluormethyl substituiert ist,
R⁷ für Wasserstoff steht,
R⁸ für 3,3,3-Trifluorpropyl oder Cyclopropyl steht,
R⁹ für tert.-Butyl steht,
R¹⁰ für Wasserstoff, Methyl, 2,2,2-Trifluorethyl, oder Cyclobutyl steht,
worin Methyl mit Cyclopropyl substituiert sein kann,
worin Cyclopropyl bis zu zweimal mit Fluor substituiert sein kann,
sowie ihre Solvate, Salze und Solvate Salze.

Bevorzugt sind im Rahmen der vorliegenden Erfindung auch Verbindungen der Formel (I), in welcher
- X: für eine Gruppe der Formel 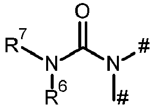 steht,
wobei
# die Verknüpfung zu dem jeweiligen Kohlenstoffatom des Ringes markiert,
R⁶ für (C₁-C₄)-Alkyl, Cyclopropyl oder Phenyl steht,
worin (C₁-C₄)-Alkyl bis zu dreimal mit Fluor substituiert sein kann,
worin Phenyl mit 1 oder 2 Substituenten unabhängig voneinander ausgewählt aus der Gruppe Chlor oder Trifluormethyl substituiert ist,
R⁷ für Wasserstoff steht,
sowie ihre Solvate, Salze und Solvate Salze.

Bevorzugt sind im Rahmen der vorliegenden Erfindung auch Verbindungen der Formel (I), in welcher
- X: für eine Gruppe der Formel 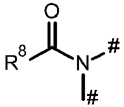 steht, wobei
# die Verknüpfung zu dem jeweiligen Kohlenstoffatom des Ringes markiert,
R⁸ für 3,3,3-Trifluorpropyl oder Cyclopropyl steht,
sowie ihre Solvate, Salze und Solvate Salze.

Bevorzugt sind im Rahmen der vorliegenden Erfindung auch Verbindungen der Formel (I), in welcher
- X: für eine Gruppe der Formel 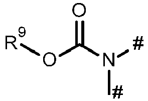 steht,
wobei
# die Verknüpfung zu dem jeweiligen Kohlenstoffatom des Ringes markiert,
R⁹ für tert.-Butyl steht,
sowie ihre Solvate, Salze und Solvate Salze.

Bevorzugt sind im Rahmen der vorliegenden Erfindung auch Verbindungen der Formel (I), in welcher
- X: für eine Gruppe der Formel 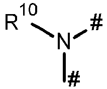 steht, wobei
# die Verknüpfung zu dem jeweiligen Kohlenstoffatom des Ringes markiert,
R¹⁰ für Wasserstoff, Methyl, 2,2,2-Trifluorethyl, oder Cyclobutyl steht,
worin Methyl mit Cyclopropyl substituiert sein kann,
worin Cyclopropyl bis zu zweimal mit Fluor substituiert sein kann,
sowie ihre Solvate, Salze und Solvate Salze.

Bevorzugt sind im Rahmen der vorliegenden Erfindung auch Verbindungen der Formel (I), in welcher
- R¹: für Phenyl oder Pyridyl steht,
wobei Phenyl mit 1 oder 2 Substituenten unabhängig voneinander ausgewählt aus der Gruppe Fluor, Methyl oder Trifluormethyl substituiert ist,
wobei Pyridyl mit 1 oder 2 Substituenten unabhängig voneinander ausgewählt aus der Gruppe Fluor, Chlor oder Trifluormethyl substituiert ist,
sowie ihre Solvate, Salze und Solvate Salze.

Bevorzugt sind im Rahmen der vorliegenden Erfindung auch Verbindungen der Formel (I), in welcher
- R¹: für Phenyl steht,
wobei Phenyl mit 1 oder 2 Substituenten unabhängig voneinander ausgewählt aus der Gruppe Fluor, Methyl oder Trifluormethyl substituiert ist,
sowie ihre Solvate, Salze und Solvate Salze.

Bevorzugt sind im Rahmen der vorliegenden Erfindung auch Verbindungen der Formel (I), in welcher
- R¹: für Pyridyl steht,
wobei Pyridyl mit 1 oder 2 Substituenten unabhängig voneinander ausgewählt aus der Gruppe Fluor, Chlor oder Trifluormethyl substituiert ist,
sowie ihre Solvate, Salze und Solvate Salze.

Bevorzugt sind im Rahmen der vorliegenden Erfindung auch Verbindungen der Formel (I), in welcher
- R²: für eine Gruppe der Formel 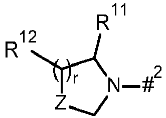 steht, wobei
#² die Verknüpfung zum Carbonyl-Kohlenstoffatom markiert,
r für 1 steht,
Z für CR^{13A}R^{13B} steht,
worin
R^{13A} für Wasserstoff oder Fluor steht,
R^{13B} für Wasserstoff steht,
R¹¹ für Wasserstoff steht,
R¹² für Wasserstoff oder Fluor steht,

Bevorzugt sind im Rahmen der vorliegenden Erfindung auch Verbindungen der Formel (I), in welcher
- R³: für Wasserstoff steht,
sowie ihre Solvate, Salze und Solvate Salze.

Bevorzugt sind im Rahmen der vorliegenden Erfindung auch Verbindungen der Formel (I), in welcher
- R⁴: für Wasserstoff oder Methyl steht,
sowie ihre Solvate, Salze und Solvate Salze.

Bevorzugt sind im Rahmen der vorliegenden Erfindung auch Verbindungen der Formel (I), in welcher
- R⁵: für Wasserstoff steht,
sowie ihre Solvate, Salze und Solvate Salze.

Weiterer Gegenstand der Erfindung ist ein Verfahren zur Herstellung der erfindungsgemäßen Verbindungen der Formel (I) dadurch gekennzeichnet, dass man
[A] eine Verbindung der Formel (II) 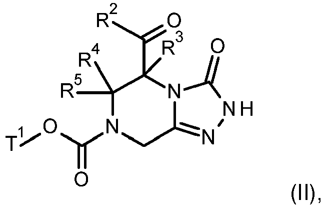 in welcher R¹, R², R³, R⁴ und R⁵ jeweils die oben genannten Bedeutungen haben, und
   - T¹: für (C₁-C₄)-Alkyl oder Benzyl steht,
   in einem inerten Lösungsmittel in Gegenwart einer geeigneten Base mit einer Verbindung der Formel (III) 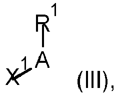 in welcher
   A und R¹ die oben genannten Bedeutungen haben,
   und
   - X¹: für eine geeignete Abgangsgruppe, insbesondere Chlor, Brom, lod, Mesylat {(methylsulfonyl)oxy}, Triflat {[(trifluormethyl)sulfonyl]oxy}, Nonaflat {[(nonafluorbutyl)sulfonyl]oxy}, Nosylat {[(4-nitrophenyl)sulfonyl]oxy} oder Tosylat {[(4-methyl-phenyl)sulfonyl]oxy} steht,
   zu einer Verbindung der Formel (IV) 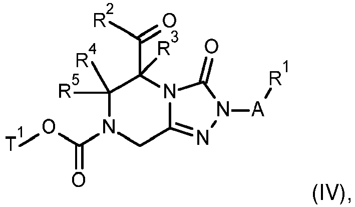 in welcher A, R¹, R², R³, R⁴, R⁵ und T¹ jeweils die oben genannten Bedeutungen haben, umsetzt,
   diese dann gegebenenfalls durch Entfernen der Gruppe "T¹" in einem inerten Lösungsmittel in Gegenwart einer geeigneten Base oder Säure in eine Verbindung der Formel (V) 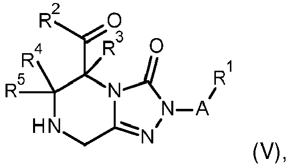 überführt,
   in welcher A, R¹, R², R³, R⁴ und R⁵ jeweils die oben genannten Bedeutungen haben,
   und diese dann gegebenenfalls in einem inerten Lösungsmittel
[A1] in Gegenwart einer geeigneten Base mit einer Verbindung der Formel (VI) 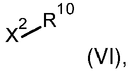 in welcher
   - R¹⁰: die oben genannten Bedeutungen hat,
   und
   - X²: für eine geeignete Abgangsgruppe, insbesondere Chlor, Brom, lod, Mesylat {(methylsulfonyl)oxy}, Triflat {[(trifluormethyl)sulfonyl]oxy}, Nonaflat {[(nonafluorbutyl)sulfonyl]oxy}, Nosylat {[(4-nitrophenyl)sulfonyl]oxy} oder Tosylat {[(4-methyl-phenyl)sulfonyl]oxy} steht,
   umsetzt und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt,
   oder
[A2] in Gegenwart einer geeigneten Base mit einer Verbindung der Formel (VII) 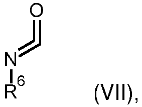 in welcher
   R⁶ die oben genannten Bedeutungen hat,
   umsetzt und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt,
   oder
[A3] in Gegenwart einer geeigneten Base mit einer Verbindung der Formel (VIII) 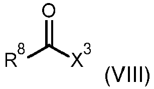 in welcher
   R⁸ die oben genannten Bedeutungen hat,
   und
   - X³: für eine geeignete Abgangsgruppe, insbesondere Chlor, Brom oder Hydroxy steht,
   umsetzt, und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt,
   oder
[B] eine Verbindung der Formel (IX) 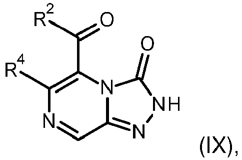 in welcher R², R⁴ und R⁵ jeweils die oben genannten Bedeutungen haben,
   in einem inerten Lösungsmittel in Gegenwart einer geeigneten Base mit einer Verbindung der Formel (III) 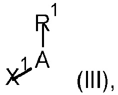 in welcher
   A und R¹ die oben genannten Bedeutungen haben,
   und
   - X¹: für eine geeignete Abgangsgruppe, insbesondere Chlor, Brom, lod, Mesylat {(methylsulfonyl)oxy}, Triflat {[(trifluormethyl)sulfonyl]oxy}, Nonaflat {[(nonafluorbutyl)sulfonyl]oxy}, Nosylat {[(4-nitrophenyl)sulfonyl]oxy} oder Tosylat {[(4-methyl-phenyl)sulfonyl]oxy} steht,
   zu einer Verbindung der Formel (X) 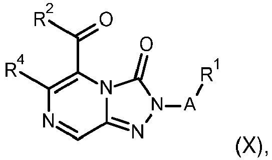 in welcher A, R¹, R² und R⁴ jeweils die oben genannten Bedeutungen haben, umsetzt, und anschließend in einem geeigneten Lösungsmittel in Gegenwart eines Palladiumkatalysators in einer Wasserstoffatmosphäre hydriert und die resultierende Verbindung der Formel (V) 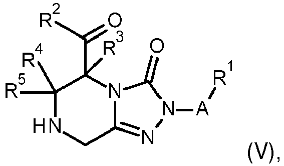 wie unter [A] beschrieben umsetzt und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Die Verbindungen der Formeln (III), (VI), (VII) und (VIII) sind kommerziell erhältlich, literaturbekannt oder können in Analogie zu literaturbekannten Verfahren hergestellt werden.

Inerte Lösungsmittel für den Verfahrensschritt (II) + (III) → (IV) bzw. (IX) + (III) → (X) sind beispielsweise Halogenkohlenwasserstoffe wie Dichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Alkohole wie Methanol, Ethanol, n-Propanol, Isopropanol, n-Butanol, 2,2,2-Trifluorethanol oder andere Lösungsmittel wie Aceton, Methylethylketon, Essigsäureethylester, Acetonitril, *N*,*N*-Dimethylformamid, *N*,*N*-Dimethylacetamid, Dimethylsulfoxid, *N*,*N'*-Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Dimethylformamid oder Acetonitril verwendet.

Als Basen für den Verfahrensschritt (II) + (III) → (IV) bzw. (IX) + (III) → (X) eignen sich die üblichen anorganischen oder organischen Basen. Hierzu gehören bevorzugt Lithium, Natrium, Kalium, Alkalihydroxide wie beispielsweise Lithium-, Natrium- oder Kaliumhydroxid, Alkoholate wie Kalium-tert-butylat, Methanolat, Ethanolat, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid oder organische Amine wie Triethylamin, *N*-Methylmorpholin, *N*-Methylpiperidin, *N*,*N*-Diisopropylethylamin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Cäsiumcarbonat verwendet.

Die Reaktion erfolgt im Allgemeinen in einem Temperaturbereich von 0°C bis +120°C, bevorzugt bei +20°C bis +80°C, ganz besonders bevorzugt bei Raumtemperatur, gegebenenfalls in einer Mikrowelle. Die Umsetzung kann bei normalem, erhöhtem oder bei erniedrigtem Druck durchgeführt werden (z.B. von 0.5 bis 5 bar).

Die Hydrolyse der Ester der Verbindungen (IV) zu Verbindungen der Formel (V) erfolgt nach üblichen Methoden, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der tert.-Butylester erfolgt die Esterspaltung bevorzugt mit Säuren.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, n-Butanol oder tert.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol und/oder n-Propanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium- oder Lithiumhydroxid.

Als Säuren eignen sich für die Esterspaltung im Allgemeinen Schwefelsäure, Chlorwasserstoff/Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der tert.-Butylester und Salzsäure im Falle der Methylester.

Die Esterspaltung erfolgt im Allgemeinen in einem Temperaturbereich von 0°C bis +100°C, bevorzugt bei +0°C bis +50°C, besonders bevorzugt b ei Raumtemperatur.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Inerte Lösungsmittel für die Umsetzung (V) + (VI) → (I) sind beispielsweise Halogenkohlenwasserstoffe wie Dichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Alkohole wie Methanol, Ethanol, n-Propanol, Isopropanol, n-Butanol, 2,2,2-Trifluorethanol oder andere Lösungsmittel wie Aceton, Methylethylketon, Essigsäureethylester, Acetonitril, *N*,*N*-Dimethylformamid, *N*,*N*-Dimethylacetamid, Dimethylsulfoxid, *N*,*N'*-Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Dimethylformamid oder Acetonitril verwendet.

Als Basen für den Verfahrensschritt (V) + (VI) → (I) eignen sich die üblichen anorganischen oder organischen Basen. Hierzu gehören bevorzugt Lithium, Natrium, Kalium, Alkalihydroxide wie beispielsweise Lithium-, Natrium- oder Kaliumhydroxid, Alkoholate wie Kalium-tert-butylat, Methanolat, Ethanolat, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid oder organische Amine wie Triethylamin, *N*-Methylmorpholin, N-Methylpiperidin, *N,N*-Diisopropylethylamin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Cäsiumcarbonat verwendet.

Die Reaktion erfolgt im Allgemeinen in einem Temperaturbereich von 0°C bis +120°C, bevorzugt bei +20°C bis +80°C, ganz besonders bevorzugt bei Raumtemperatur, gegebenenfalls in einer Mikrowelle. Die Umsetzung kann bei normalem, erhöhtem oder bei erniedrigtem Druck durchgeführt werden (z.B. von 0.5 bis 5 bar).

Inerte Lösungsmittel für die Umsetzung (V) + (VII) → (I) sind beispielsweise Halogenkohlenwasserstoffe wie Dichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Alkohole wie Methanol, Ethanol, n-Propanol, Isopropanol, n-Butanol, 2,2,2-Trifluorethanol oder andere Lösungsmittel wie Aceton, Methylethylketon, Essigsäureethylester, Acetonitril, *N*,*N*-Dimethylformamid, *N*,*N*-Dimethylacetamid, Dimethylsulfoxid, *N*,*N'*-Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Dimethylformamid oder Acetonitril verwendet.

Als Basen für den Verfahrensschritt (V) + (VII) → (I) eignen sich die üblichen anorganischen oder organischen Basen. Hierzu gehören bevorzugt Lithium, Natrium, Kalium, Alkoholate wie Kalium-tert-butylat, Methanolat, Ethanolat, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid oder organische Amine wie Triethylamin, *N*-Methylmorpholin, *N*-Methylpiperidin, *N,N*-Diisopropylethylamin, 1,5-Diazabicyclo[4.3.0]non-5-en (DBN), 1,8-Diazabicyclo[5.4.0]undec-7-en (DBU) oder 1,4-Diazabicyclo[2.2.2]octan (DA-BCO^{®}). Bevorzugt wird Cäsiumcarbonat verwendet.

Die Reaktion erfolgt im Allgemeinen in einem Temperaturbereich von 0°C bis +120°C, bevorzugt bei +20°C bis +80°C, ganz besonders bevorzugt bei Raumtemperatur, gegebenenfalls in einer Mikrowelle. Die Umsetzung kann bei normalem, erhöhtem oder bei erniedrigtem Druck durchgeführt werden (z.B. von 0.5 bis 5 bar).

Inerte Lösungsmittel für die Amidkupplung (V) + (VIII) → (I) wenn X³ für Hydroxy steht sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N,N*-Dimethylformamid, *N,N*-Dimethylacetamid, *N,N'*-Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Kondensationsmittel für die Amidbildung (V) + (VIII) → (I) wenn X³ für Hydroxy steht eignen sich beispielsweise Carbodiimide wie *N,N*-Diethyl-, *N,N'*-Dipropyl-, *N,N'*-Diisopropyl-, *N,N'-*Dicyclohexylcarbodiimid (DCC) oder *N*-(3-Dimethylaminopropyl)-*N'*-ethylcarbodiimid-Hydrochlorid (EDC), Phosgen-Derivate wie *N,N'*-Carbonyldiimidazol (CDI), 1,2-Oxazoliumverbindungen wie 2-Ethyl-5-phenyl-1,2-oxazolium-3-sulfat oder 2-*tert*.-Butyl-5-methyl-isoxazoliumperchlorat, Acylaminoverbindungen wie 2-Ethoxy-1-ethoxycarbonyl-1,2-dihydrochinolin, oder Isobutylchlorformiat, Propanphosphonsäureanhydrid (T3P), 1-Chlor-*N,N,*2-trimethylprop1-en-1-amin, Cyanophosphonsäurediethylester, Bis-(2-oxo-3-oxazolidinyl)-phosphorylchlorid, Benzotriazol-1-yloxy-tris(dimethylamino)phosphonium-hexafluorphosphat, Benzotriazol-1-yloxy-tris(pyrrolidino)phosphonium-hexafluorphosphat (PyBOP), *O*-(Benzotriazol-1-yl)-*N,N,N',N'*-tetramethyluronium-tetrafluorborat (TBTU), *O*-(Benzotriazol-1-yl)-*N,N,N',N'*-tetramethyluronium-hexafluorphosphat (HBTU), 2-(2-Oxo-1-(2*H*)-pyridyl)-1,1,3,3-tetramethyluronium-tetrafluorborat (TPTU), *O*-(7-Azabenzotriazol-1-yl)-*N,N,N',N'*-tetramethyluronium-hexafluorphosphat (HATU) oder *O-*(1*H*-6-Chlorbenzotriazol-1-yl)-1,1,3,3-tetramethyluronium-tetrafluorborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, *N*-Methylpiperidin oder *N,N*-Diisopropylthylamin. Bevorzugt wird HATU verwendet.

Die Kondensationen (V) + (VIII) → (I) wenn X³ für Hydroxy steht wird im Allgemeinen in einem Temperaturbereich von -20°C bis +100°C, bevorzugt b ei 0°C bis +60°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Alternativ kann die Carbonsäure der Formel (VIII, wenn X³ für Hydroxy steht) auch zunächst in das entsprechende Carbonsäurechlorid überführt werden und dieses dann direkt oder in einer separaten Umsetzung mit einem Amin der Formel (VI) zu den erfindungsgemäßen Verbindungen umgesetzt werden. Die Bildung von Carbonsäurechloriden aus Carbonsäuren erfolgt nach den dem Fachmann bekannten Methoden, beispielsweise durch Behandlung mit Thionylchlorid, oder Oxalylchlorid in Gegenwart einer geeigneten Base, beispielsweise in Gegenwart von Pyridin, sowie optional unter Zusatz von Dimethylformamid, optional in einem geeigneten inerten Lösemittel.

Inerte Lösungsmittel für die Umsetzung (V) + (VIII) → (I) wenn X³ für Chlor oder Brom steht sind beispielsweise Halogenkohlenwasserstoffe wie Dichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Alkohole wie Methanol, Ethanol, n-Propanol, Isopropanol, n-Butanol, 2,2,2-Trifluorethanol oder andere Lösungsmittel wie Aceton, Methylethylketon, Essigsäureethylester, Acetonitril, *N,N*-Dimethylformamid, *N*,*N*-Dimethylacetamid, Dimethylsulfoxid, *N,N'*-Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Dichlormethan verwendet.

Als Basen für den Verfahrensschritt (V) + (VIII) → (I) wenn X³ für Chlor oder Brom steht eignen sich die üblichen anorganischen oder organischen Basen. Hierzu gehören bevorzugt Lithium, Natrium, Kalium, Alkalihydroxide wie beispielsweise Alkoholate wie Kalium-tert-butylat, Methanolat, Ethanolat, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid oder organische Amine wie Triethylamin, *N*-Methylmorpholin, *N*-Methylpiperidin, *N,N*-Diisopropylethylamin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Cäsiumcarbonat verwendet.

Die Reaktion erfolgt im Allgemeinen in einem Temperaturbereich von 0°C bis +120°C, bevorzugt bei +20°C bis +80°C, ganz besonders bevorzugt bei Raumtemperatur, gegebenenfalls in einer Mikrowelle. Die Umsetzung kann bei normalem, erhöhtem oder bei erniedrigtem Druck durchgeführt werden (z.B. von 0.5 bis 5 bar).

Inerte Lösungsmittel für den Verfahrensschritt (X) → (V) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Aceton, Essigsäureethylester, Acetonitril, *N,N*-Dimethylformamid, *N,N-*Dimethylacetamid, *N,N'*-Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Der Verfahrensschritt (X) → (V) wird im Allgemeinen in einem Temperaturbereich von -20°C bis +100°C, bevorzugt bei 0°C bis +60°C durchgeführ t. Die Umsetzung kann bei normalem oder erhöhtem Wasserstoff-Druck erfolgen (z.B. von 1.0 bis 100 bar). Im Allgemeinen arbeitet man bei erhöhtem Wasserstoff-Druck.

Die Verbindungen der Formel (II) können hergestellt werden, indem eine Verbindung der Formel (XI) 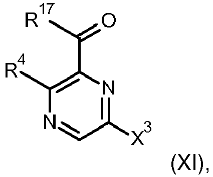 in welcher
- R¹⁷: für Hydroxy, Chlor, Brom oder Iod steht,
- X³: für Fluor Chlor, Brom oder lod steht,
und R⁴ die oben genannten Bedeutungen hat,
in einem inerten Lösungsmittel unter Amidkupplungsbedingungen mit einem Amin der Formel (VI-A) bzw. (VI-B), 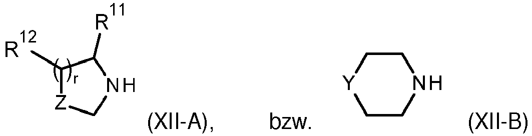 in welcher Y, Z, R¹¹ und R¹² jeweils die oben genannten Bedeutungen haben,
zu einer einer Verbindung der Formel (XIII) 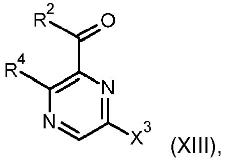 in welcher
- X³: für Fluor Chlor, Brom oder lod steht,
und R⁴ und R² jeweils die oben genannten Bedeutungen hat,
umsetzt,
und
[A4] diese in einem inerten Lösungsmittel gegebenfalls in Gegenwart einer geeigneten Base und gegebenenfalls eines Palladiumkatalysators mit Hydrazin Hydrat oder eines geeigneten Hydrazin Derivates in eine Verbindung der Formel (XIV), 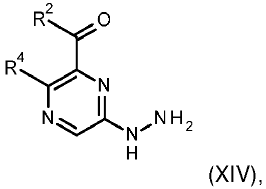 überführt,
in welcher
R² und R⁴ jeweils die oben genannten Bedeutungen haben,
und diese in einem geeigneten Lösungsmittel mit einem Phosgen-Derivat zu einer Verbindung der Formel (IX), 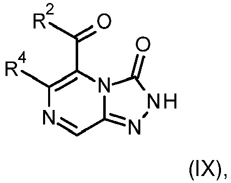 in welcher R² und R⁴ jeweils die oben genannten Bedeutungen haben,
cyclisiert werden und anschließend in einem geeigneten Lösungsmittel in Gegenwart eines Palladiumkatalysators und Di-tert-butyldicarbonat in einer Wasserstoffatmosphäre hydriert wird,
und die resultierenden Verbindungen der Formel (II) gegebenenfalls mit den entsprechenden (i) Lösungsmitteln und/oder (ii) Säuren oder Basen in ihre Solvate, Salze und/oder Solvate der Salze überführ,
oder
[A5] diese in einem inerten Lösungsmittel in Gegenwart einer geeigneten Base und gegebenenfalls eines Palladiumkatalysators mit einer Verbindung der Formel XII, 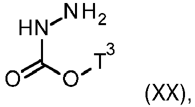 in welcher
T³ für Benzyl oder tert-Butyl steht,
zu einer Verbindung der Formel (XXI), 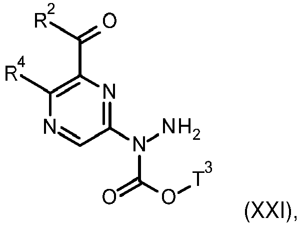 überführt,
in welcher
R² ,R⁴ und T³ die jeweils die oben genannten Bedeutungen haben,
umsetzt, die Schutzgruppe T³ in einem geeigneten inerten Lösungsmittel durch Hydrogenolyse in Gegenwart eines Palladiumkatalysators abgespalten wird und die daraus resultierende Verbindung der Formel (XIV) 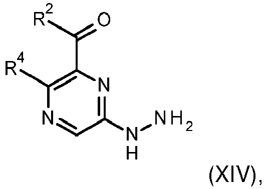 in einem geeigneten Lösungsmittel mit einem Phosgen-Derivat zu einer Verbindung der Formel (IX), 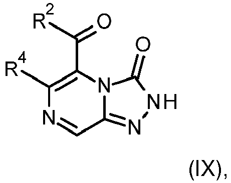 in welcher R² und R⁴ jeweils die oben genannten Bedeutungen haben,
cyclisiert werden und anschließend in einem geeigneten Lösungsmittel in Gegenwart eines Palladiumkatalysators und Di-tert-butyldicarbonat in einer Wasserstoffatmosphäre hydriert wird,
und die resultierenden Verbindungen der Formel (II) gegebenenfalls mit den entsprechenden (i) Lösungsmitteln und/oder (ii) Säuren oder Basen in ihre Solvate, Salze und/oder Solvate der Salze überführt.

Die Verbindungen der Formeln (XI), (XII-A) und (XII-B) sind kommerziell erhältlich, literaturbekannt oder können in Analogie zu literaturbekannten Verfahren hergestellt werden.

Die beschriebenen Verfahren werden durch die nachfolgenden Schemata (Schema 1-2) beispielhaft verdeutlicht:

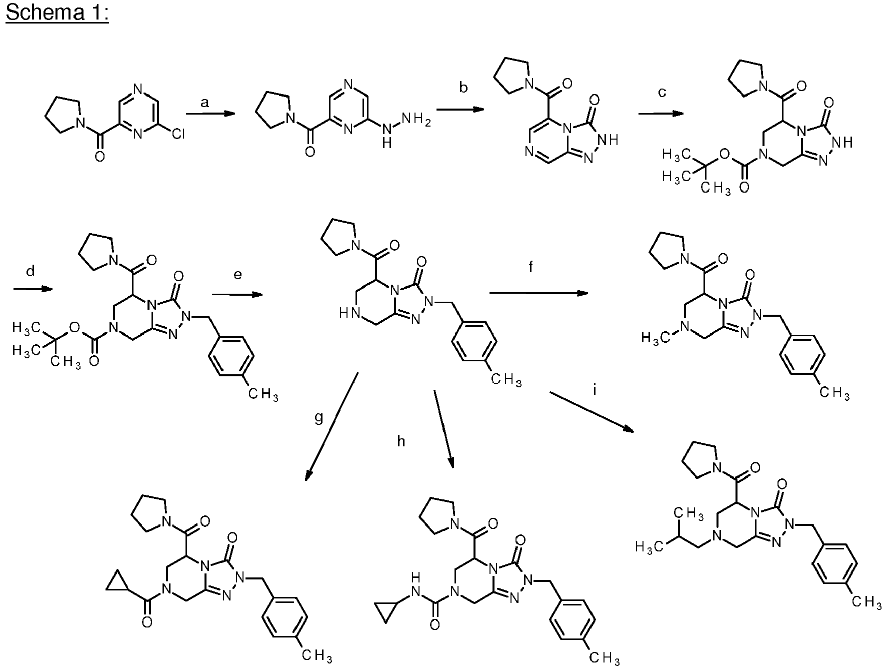

a) Hydrazinhydrat, Ethanol, 80°C; b) Carbonyldiimid azol, THF, RT; c) Pd/C 5%, 1 bar H₂ in 1,4-Dioxan, Di-tert-butyldicarbonat, RT; d) Cäsiumcarbonat, 1-(Brommethyl)-3,5-dichlorbenzol in Acetonitril, RT; e) Trifluoressigsäure, Dichlormethan, RT; f) lodmethan, N,N-Diisopropylethylamin, THF, RT; g) Cyclopropancarbonylchlorid, N,N-Diisopropylethylamin, THF, RT; h) Isocyanatocyclopropan, Dichlormethan, RT; i) 2-Methylpropanal, Natriumtriacetoxyborhydrid, Essigsäure, THF, RT.

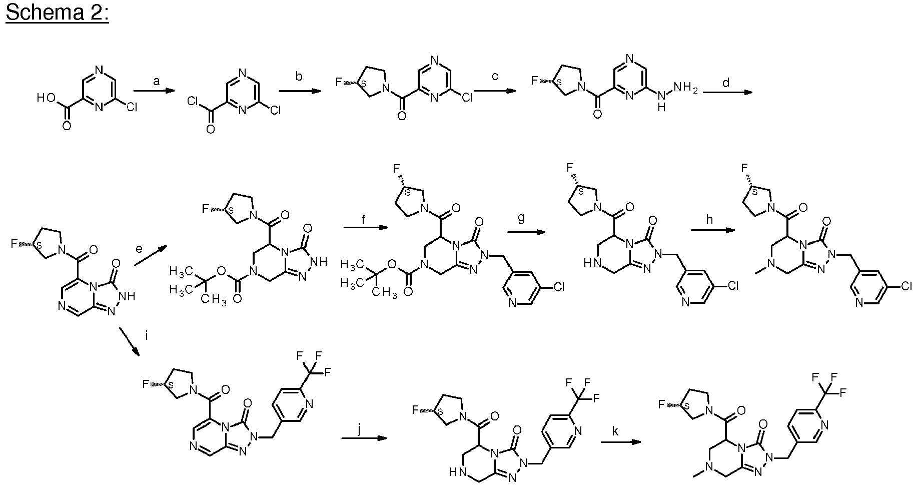

a) Thionylchlorid, 90°C; b) (3S)-3-Fluorpyrrolidin hydrochlorid, N,N-Diisopropylethylamin, Dichlormethan, RT; c) Hydrazinhydrat, Ethanol, 80°C; d) Carbonyldiimidazol, THF, RT; e) Pd/C 5%, 1 bar H₂ in 1,4-Dioxan, Di-tert-butyldicarbonat, RT; f) Cäsiumcarbonat, 3-Chlor-5-(chlormethyl)pyridine, Acetonitril, RT; g) Trifluoressigsäure, Dichlormethan, RT; h) lodmethan, N,N-Diisopropylethylamin, THF, RT; i) Cäsiumcarbonat, 5-(Chlormethyl)-2-(trifluormethyl)-pyridinhydrochlorid, Acetonitril, RT; i) Pd/C 10%, 1 bar H₂ in Methanol/Ethanol, 95°C: k) Formaldehyd, Natriumtriacetoxyborhydrid, Essigsäure, THF, RT.

Inerte Lösungsmittel für die Amidkupplung (XI) + (XII-A) → (XIII) bzw. (XI) + (XII-B) → (XIII) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N,N-*Dimethylformamid, *N,N*-Dimethylacetamid, *N,N'*-Dimethylpropylenharnstoff (DMPU) oder N-Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Kondensationsmittel für die Amidbildung (XI) + (XII-A) → (XIII) bzw. (XI) + (XII-B) → (XIII) eignen sich beispielsweise Carbodiimide wie *N,N*'-Diethyl-, *N,N*'-Dipropyl-, *N,N'*-Diisopropyl-, *N,N'*-Dicyclohexylcarbodiimid (DCC) oder *N*-(3-Dimethylaminopropyl)-*N'*-ethylcarbodiimid-Hydrochlorid (EDC), Phosgen-Derivate wie *N,N'*-Carbonyldiimidazol (CDI), 1,2-Oxazoliumverbindungen wie 2-Ethyl-5-phenyl-1,2-oxazolium-3-sulfat oder 2-*tert*.-Butyl-5-methyl-isoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid (T3P), 1-Chlor-*N,N*,2-trimethylprop1-en-1-amin, Cyanophosphonsäurediethylester, Bis-(2-oxo-3-oxazolidinyl)-phosphorylchlorid, Benzotriazol-1-yloxy-tris(dimethylamino)phosphonium-hexafluorphosphat, Benzotriazol-1-yloxy-tris(pyrrolidino)phosphonium-hexafluorphosphat (PyBOP), *O*-(Benzotriazol-1-yl)-*N,N,N',N'-*tetramethyluronium-tetrafluorborat (TBTU), *O*-(Benzotriazol-1-yl)-*N,N,N',N'*-tetramethyluronium-hexafluorphosphat (HBTU), 2-(2-Oxo-1-(2*H*)-pyridyl)-1,1,3,3-tetramethyluronium-tetrafluorborat (TPTU), *O*-(7-Azabenzotriazol-1-yl)-*N,N,N',N'*-tetramethyluronium-hexafluorphosphat (HATU) oder *O*-(1*H*-6-Chlorbenzotriazol-1-yl)-1,1,3,3-tetramethyluronium-tetrafluorborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, *N*-Methylpiperidin oder *N*,*N*-Diisopropylethylamin. Bevorzugt wird HATU verwendet.

Die Kondensationen (XI) + (XII-A) → (XIII) bzw. (XI) + (XII-B) → (XIII) wird im Allgemeinen in einem Temperaturbereich von -20°C bis +100°C, bevor zugt bei 0°C bis +60°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Alternativ kann die Carbonsäure der Formel (XI, wenn R¹⁷ für Hydroxy steht) auch zunächst in das entsprechende Carbonsäurechlorid überführt werden und dieses dann direkt oder in einer separaten Umsetzung mit einem Amin der Formel (VI) zu den erfindungsgemäßen Verbindungen umgesetzt werden. Die Bildung von Carbonsäurechloriden aus Carbonsäuren erfolgt nach den dem Fachmann bekannten Methoden, beispielsweise durch Behandlung mit Thionylchlorid, oder Oxalylchlorid in Gegenwart einer geeigneten Base, beispielsweise in Gegenwart von Pyridin, sowie optional unter Zusatz von Dimethylformamid, optional in einem geeigneten inerten Lösemittel.

Der Verfahrensschritt (XIII) → (XIV) erfolgt in einem unter den Reaktionsbedingungen inerten Lösungmittel. Geeignete Lösungsmittel sind beispielsweise Ether wie 1,4-Dioxan, Tetrahydrofuran, 2-Methyltetrahydrofuran, Diethylether, Di-*n*-butylether, Glykoldimethylether oder Diethylenglykoldimethylether, Alkohole wie Methanol, Ethanol, tert.-Butanol oder Amylalkohole oder anderen Lösungsmitteln wie Dimethylformamid (DMF), Dimethylsulfoxid (DMSO), Dimethylacetamid (DMA), Toluol oder Acetonitril. Ebenso ist es möglich, Gemische der genannten Lösungsmittel einzusetzen. Bevorzugt ist *Ethanol.*

Der Verfahrensschritt (XIII) → (XIV) wird im Allgemeinen in einem Temperaturbereich von 0°C bis +200°C, bevorzugt bei +10°C bis +150°C durchgef ührt. Die Umsetzungen können auch in geschlossenen Gefäßen (Mikrowellenröhren) in der Mikrowelle durchgeführt werden. Die Umsetzung kann bei normalem, erhöhtem oder bei erniedrigtem Druck erfolgen (z.B. von 0.5 bis 5 bar). Im Allgemeinen arbeitet man bei Normaldruck oder in geschlossenen Gefäßen (Mikrowellenröhren) unter- oder oberhalb des Siedepunktes des verwendeten Lösungsmittels. Bevorzugt sind Reaktionen in geschlossenen Gefäßen (Mikrowellenröhrchen), bei Temperaturen oberhalb des Siedepunktes des Lösungsmittels und unter erhöhtem Druck mit oder ohne Verwendung einer Mikrowelle.

Inerte Lösungsmittel für den Verfahrensschritt (XIV) → (IX) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N,N*-Dimethylformamid, *N,N*-Dimethylacetamid, *N,N'*-Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Phosgenderivat für den Verfahrensschritt (XIV) → (IX) eignen sich beispielsweise *N,N'-*Carbonyldiimidazol (CDI), Trichlormethylchlorkohlensäureester (Diphosgen), Bis(trichlormethyl)carbonat (Triphosgen) oder Chlorameisensäurearylester.Bevorzugt wird *N,N'*-Carbonyldiimidazol (CDI) verwendet.

Der Verfahrensschritt (XIV) → (IX) wird im Allgemeinen in einem Temperaturbereich von -20°C bis +100°C, bevorzugt bei 0°C bis +60°C durchgeführ t. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (IX) → (II) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Aceton, Essigsäureethylester, Acetonitril, *N,N*-Dimethylformamid, *N,N-*Dimethylacetamid, *N,N'*-Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Der Verfahrensschritt (IX) → (II) wird im Allgemeinen in einem Temperaturbereich von -20°C bis +100°C, bevorzugt bei 0°C bis +60°C durchgeführ t. Die Umsetzung kann bei normalem oder erhöhtem Wasserstoff-Druck erfolgen (z.B. von 1.0 bis 100 bar). Im Allgemeinen arbeitet man bei erhöhtem Wasserstoff-Druck.

In den Ausgangsverbindungen gemäß Formel (II) gegebenenfalls vorhandene Hydroxy-, Amino und/oder Amido-Gruppen können, falls zweckmäßig oder erforderlich, auch in temporärer geschützter Form eingesetzt und dann am Ende der jeweiligen Reaktionssequenz wieder freigesetzt werden [zur Eignung, Enführung und Entfernung solcher Schutzgruppen siehe z.B. T.W.Greene und P.G.M. Wuts, Protective Groups in Organic Synthesis, Wiley, New York, 1999).

Weitere erfindungsgemäße Verbindungen können gegebenenfalls auch hergestellt werden durch Umwandlungen von funktionellen Gruppen einzelner Substituenten, insbesondere den unter R¹, R², R⁴, aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetallkatalysierte Kupplungsreaktionen, Eliminierungen, Alkylierung, Aminierung, Veresterung, Esterspaltung, Veretherung, Etherspaltung, Bildung von Carbonamiden, Dehydratisierungen sowie Einführung und Entfernung temporärer Schutzgruppen.

Detaillierte Vorschriften befinden sich auch im Experimentellen Teil im Abschnitt zur Herstellung der Ausgangsverbindungen und Intermediate.

Erfindungsgemäßen Verbindungen der Formel (I) bei denen X für Sauerstoff steht, lassen sich wie im nachfolgenden Schema (Schema 3) gezeigt herstellen.

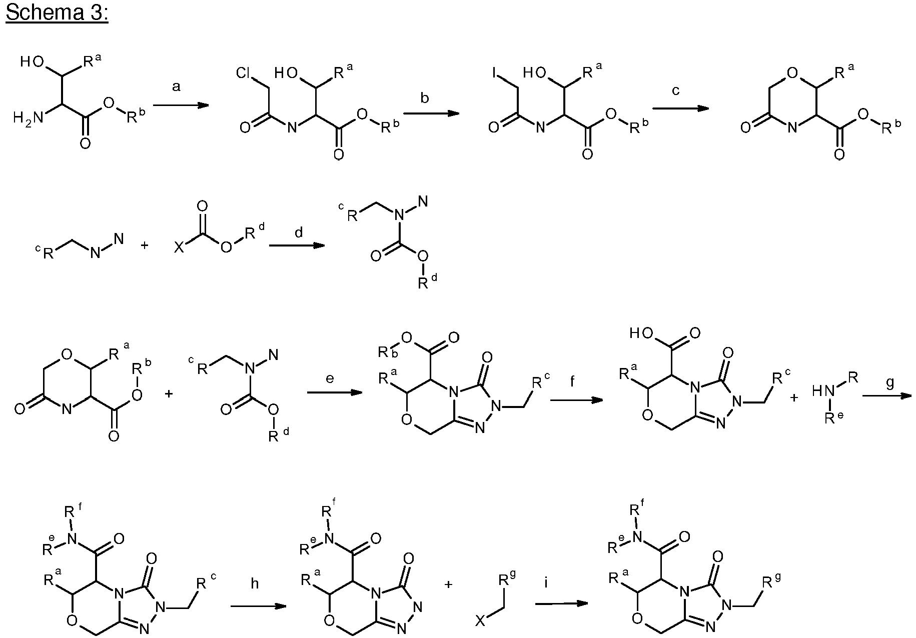

a) Chloracetylchlorid, Triethylamin, THF, 0°C; b) N atriumiodid, Acetonitril, RT; c) Silber(I)oxid, Dichlormethan, RT, Lichtausschluss d) Triethylamin, Dichlormethan, -78°C, e) Trimethyloxoniumtetrafluoroborat, Dichlormethan, DMF, RT-150°C; f) Lithiumhydroxid, THF, RT; g) Diisopropylethylamin, THF, RT; h) TFA, 150°C Mikrowelle; i) Caesiumcarbonat, Acetonitril, RT

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen potente, chemisch stabile Inhibitoren der humanen Prolyl-Endopeptidase (PREP, PE, Prolyl-Oligopeptidase, POP) dar und eignen sich daher zur Behandlung und/oder Prävention von Erkrankungen und pathologischen Prozessen, insbesondere solcher, bei denen im Zuge eines infektiösen oder nicht-infektiösen Entzündungsgeschehens und/oder eines Gewebe- oder Gefäßumbaus die PREP bzw. das PREP-Produkt PGP (Prolin-Glyzin-Prolin) involviert ist.

Dazu zählen im Sinne der vorliegenden Erfindung insbesondere Erkrankungen der Atemwege und der Lunge, wie die chronisch-obstruktive Lungenerkrankung (COPD), die zystische Fibrose (Mukoviszidose, CF), Asthma und die Gruppe der interstitiellen Lungenerkrankungen (ILD), sowie Erkrankungen des Herz-Kreislauf-Systems, wie die Arteriosklerose und Myokarditis.

Zu den Ausprägungen der COPD gehören insbesondere das z.B. durch Zigarettenrauch induzierte Lungenemphysem, die chronische Bronchitis (CB), die pulmonale Hypertonie in der COPD (PH-COPD), Bronchiektasie (BE) und Kombinationen hiervon, insbesondere in akut exazerbierenden Stadien der Erkrankung (AE-COPD).

Zu den Ausprägungen von Asthma gehören asthmatische Erkrankungen unterschiedlicher Schweregrade mit intermittierendem oder persistierendem Verlauf, wie refraktäres Asthma, bronchiales Asthma, allergisches Asthma, intrinsisches Asthma, extrinsisches Asthma und durch Medikamente oder Staub induziertes Asthma.

Zu der Gruppe der interstitiellen Lungenerkrankungen (ILD) gehören die idiopathische pulmonale Fibrose (IPF), die Lungensarkoidose und die akute interstitielle Pneumonie, nichtspezifische interstitielle Pneumonien, lymphoide interstitielle Pneumonien, respiratorische Bronchiolitis mit interstitieller Lungenerkrankung, kryptogene organisierende Pneumonien, desquamative interstitielle Pneumonien und nicht-klassifizierbare idiopathische interstitielle Pneumonien, ferner granulomatöse interstitielle Lungenerkrankungen, interstitielle Lungenerkrankungen bekannter Ursache und andere interstitielle Lungenerkrankungen unbekannter Ursache.

Die erfindungsgemäßen Verbindungen können auch zur Behandlung und/oder Prävention von weiteren Erkrankungen der Atemwege und der Lunge verwendet werden, wie z.B. der pulmonalen arteriellen Hypertonic (PAH) und anderer Formen der pulmonalen Hypertonie (PH), des Bronchiolitis obliterans-Syndroms (BOS), des akuten Atemwegssyndroms (ARDS), der akuten Lungenschädigung (ALI), der alpha-1-Antitrypsin-Defizienz (AATD) und der zystischen Fibrose (CF), von verschiedenen Formen der Bronchitis (chronische Bronchitis, infektiöse Bronchitis, eosinophile Bronchitis), von Bronchiektasie, Pneumonie, Farmerlunge und verwandten Krankheiten, infektiös und nicht-infektiös bedingten Husten- und Erkaltungskrankheiten (chronischer entzündlicher Husten, iatrogener Husten), Nasenschleimhautentzündungen (einschließlich medikamentöse Rhinitis, vasomotorische Rhinitis und Jahreszeit-abhängige, allergische Rhinitis, z.B. Heuschnupfen) und von Polypen.

Die erfindungsgemäßen Verbindungen können darüber hinaus zur Behandlung und/oder Prävention von kardiovaskulären Erkrankungen eingesetzt werden, wie beispielsweise Bluthochdruck (Hypertonie), Herzinsuffizienz, koronare Herzerkrankung, stabile und instabile Angina pectoris, renale Hypertonie, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden des Grades I-III, supraventrikuläre Tachyarrhythmie, Vorhofflimmern, Vorhofflattern, Kammerflimmern, Kammerflattern, ventrikuläre Tachyarrhythmie, Torsade de pointes-Tachykardie, Extrasystolen des Vorhofs und des Ventrikels, AV-junktionale Extrasystolen, Sick-Sinus-Syndrom, Synkopen, AV-Knoten-Reentry-Tachykardie, Wolff-Parkinson-White-Syndrom, akutes Koronarsyndrom (ACS), autoimmune Herzerkrankungen (Perikarditis, Endokarditis, Valvolitis, Aortitis, Kardiomyopathien), Boxerkardiomyopathie, Aneurysmen, Schock wie kardiogener Schock, septischer Schock und anaphylaktischer Schock, ferner zur Behandlung und/oder Prävention von thromboembolischen Erkrankungen und Ischämien, wie myokardiale Ischämie, Myokardinfarkt, Hirnschlag, Herzhypertrophie, transistorische und ischämische Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, periphere Durchblutungsstörungen, Reperfusionsschäden, arterielle und venöse Thrombosen, Mikroalbuminurie, Herzmuskelschwäche, endotheliale Dysfunktion, mikro- und makrovaskuläre Schädigungen (Vaskulitis), sowie zur Verhinderung von Restenosen beispielsweise nach Thrombolyse-Therapien, percutan-transluminalen Angioplastien (PTA), percutan-transluminalen Koronarangioplastien (PTCA), Herztransplantationen und Bypass-Operationen.

Im Sinne der vorliegenden Erfindung umfasst der Begriff Herzinsuffizienz sowohl akute als auch chronische Erscheinungsformen der Herzinsuffizienz wie auch spezifische oder verwandte Krankheitsformen hiervon, wie akute dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Die erfindungsgemäßen Verbindungen eignen sich außerdem zur Behandlung und/oder Prävention von Nierenerkrankungen, insbesondere von Niereninsuffizienz und Nierenversagen. Im Sinne der vorliegenden Erfindung umfassen die Begriffe Niereninsuffizienz und Nierenversagen sowohl akute als auch chronische Erscheinungsformen hiervon wie auch diesen zugrundeliegende oder verwandte Nierenerkrankungen, wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantat-Abstoßung und Immunkomplex-induzierte Nierenerkrankungen, durch toxische Substanzen induzierte Nephropathie, Kontrastmittelinduzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Hypertonie, Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Darüber hinaus sind die erfindungsgemäßen Verbindungen zur Behandlung und/oder Prävention von Erkrankungen des Urogenitalsystems geeignet, wie beispielsweise benignes Prostata-Syndrom (BPS), benigne Prostatahyperplasie (BPH), benigne Prostatavergrößerung (BPE), Blasenentleerungsstörungen (BOO), untere Harnwegssyndrome (LUTS), neurogene überaktive Blase (OAB), Inkontinenz wie beispielsweise Misch-, Drang-, Stress- oder Überlauf-Inkontinenz (MUI, UUI, SUI, OUI), Beckenschmerzen sowie erektile Dysfunktion und weibliche sexuelle Dysfunktion.

Die erfindungsgemäßen Verbindungen können auch zur Behandlung von Erkrankungen des weiblichen Reproduktionssystems, wie Uterusmyome, Endometriose, Dysmenorrhöe und vorzeitige Geburtswehen, verwendet werden. Weiterhin eignen sie sich zur Prophylaxe oder Behandlung von Hirsutismus und Hypertrichose.

Zudem besitzen die erfindungsgemäßen Verbindungen anti-inflammatorische Wirkung und können daher als entzündungshemmende Mittel zur Behandlung und/oder Prävention von Sepsis (SIRS), multiplem Organversagen (MODS, MOF), entzündlichen Erkrankungen der Niere, chronischen Darmentzündungen (IBD, Morbus Crohn, Colitis ulcerosa), Pankreatitis, Peritonitis, Cystitis, Urethritis, Prostatitis, Epidimytitis, Oophoritis, Salpingitis, Vulvovaginitis, rheumatoiden Erkrankungen, Arthrose, entzündlichen Erkrankungen des Zentralnervensystems, multipler Sklerose, entzündlichen Hauterkrankungen und entzündlichen Augenerkrankungen eingesetzt werden.

Die erfindungsgemäßen Verbindungen sind ferner zur Behandlung und/oder Prävention von fibrotischen Erkrankungen der inneren Organe, wie beispielsweise der Lunge, des Herzens, der Niere, des Knochenmarks und insbesondere der Leber, sowie von dermatologischen Fibrosen und fibrotischen Erkrankungen des Auges geeignet. Im Sinne der vorliegenden Erfindung umfasst der Begriff fibrotische Erkrankungen insbesondere solche Erkrankungen wie Leberfibrose, Leberzirrhose, Lungenfibrose, Endomyokardfibrose, Nephropathie, Glomerulonephritis, interstitielle Nierenfibrose, fibrotische Schäden in Folge von Diabetes, Knochenmarksfibrose, Peritonealfibrose und ähnliche fibrotische Erkrankungen, Sklerodermie, Morphaea, Keloide, hypertrophe Narbenbildung, Naevi, diabetische Retinopathie, proliferative Vitroretinopathie und Erkrankungen des Bindegewebes (z.B. Sarkoidose). Die erfindungsgemäßen Verbindungen können ebenso verwendet werden zur Förderung der Wundheilung, zur Bekämpfung postoperativer Narbenbildung, z.B. nach Glaukom-Operationen, und zu kosmetischen Zwecken bei alternder oder verhornender Haut.

Auch können die erfindungsgemäßen Verbindungen zur Behandlung und/oder Prävention von Anämien verwendet werden, wie hämolytischen Anämien, insbesondere Hämoglobinopathien wie Sichelzellanämie und Thalassämien, megaloblastären Anämien, Eisenmangel-Anämien, Anämien durch akuten Blutverlust, Verdrängungsanämien und aplastischen Anämien.

Die erfindungsgemäßen Verbindungen sind zudem zur Behandlung von Krebserkrankungen geeignet, wie beispielsweise von Hautkrebs, Hirntumoren, Brustkrebs, Knochenmarktumoren, Leukämien, Liposarcomen, Karzinomen des Magen-Darm-Traktes, der Leber, Bauchspeicheldrüse, Lunge, Niere, Harnleiter, Prostata und des Genitaltraktes sowie von bösartigen Tumoren des lymphoproliferativen Systems, wie z.B. Hodgkin's und Non-Hodgkin's Lymphom.

Darüber hinaus können die erfindungsgemäßen Verbindungen eingesetzt werden zur Behandlung und/oder Prävention von Arteriosklerose, Lipidstoffwechselstörungen und Dyslipidämien (Hypolipoproteinämie, Hypertriglyceridämie, Hyperlipidämie, kombinierte Hyperlipidämien, Hypercholesterolämie, Abetalipoproteinämie, Sitosterolämie), Xanthomatose, Tangier-Krankheit, Fettsucht (Adipositas), Fettleibigkeit (Obesitas), metabolischen Erkrankungen (Metabolisches Syndrom, Hyperglykämie, Insulin-abhängiger Diabetes, nicht-Insulin-abhängiger Diabetes, Gestationsdiabetes, Hyperinsulinämie, Insulinresistenz, Glukose-Intoleranz und diabetische Spätfolgen wie Retinopathie, Nephropathie und Neuropathie), von Erkrankungen des Gastrointestinaltrakts und des Abdomen (Glossitis, Gingivitis, Periodontitis, Oesophagitis, eosinophile Gastroenteritis, Mastocytose, Morbus Crohn, Colitis, Proctitis, Pruritis ani, Diarrhöe, Zöliakie, Hepatitis, Leberfibrose, Leberzirrhose, Pankreatitis und Cholecystitis), von Erkrankungen des Zentralen Nervensystems und von neurodegenerativen Störungen (Schlaganfall, Alzheimer'sche Krankheit, Parkinson'sche Krankheit, Demenz, Epilepsie, Depressionen, Multiple Sklerose), Immunerkrankungen, Schilddrüsenerkrankungen (Hyperthyreose), Hauterkrankungen (Psoriasis, Akne, Ekzeme, Neurodermitis, vielfältige Formen der Dermatitis wie z.B. Dermatitis abacribus, Dermatitis actinica, Dermatitis allergica, Dermatitis ammoniacalis, Dermatitis artefacta, Dermatitis autogenica, Dermatitis atrophicans, Dermatitis calorica, Dermatitis combustionis, Dermatitis congelationis, Dermatitis cosmetica, Dermatitis escharotica, Dermatitis exfoliativa, Dermatitis gangraenose, Dermatitis haemostatica, Dermatitis herpetiformis, Dermatitis lichenoides, Dermatitis linearis, Dermatitis maligna, Dermatitis medimencatosa, Dermatitis palmaris et plantaris, Dermatitis parasitaria, Dermatitis photoallergica, Dermatitis phototoxica, Dermatitis pustularis, Dermatitis seborrhoica, Dermatitis solaris, Dermatitis toxica, Dermatitis ulcerosa, Dermatitis veneata, infektiöse Dermatitis, pyogene Dermatitis und Rosazea-artige Dermatitis, sowie Keratitis, Bullosis, Vasculitis, Cellulitis, Panniculitis, Lupus erythematodes, Erythema, Lymphome, Hautkrebs, Sweet-Syndrom, Weber-Christian-Syndrom, Narbenbildung, Warzenbildung, Frostbeulen), von entzündlichen Augenerkrankungen (Saccoidosis, Blepharitis, Conjunctivitis, Iritis, Uveitis, Chorioiditis, Ophthalmitis), viralen Erkrankungen (durch Influenza-, Adeno- und Coronaviren, wie z.B. HPV, HCMV, HIV, SARS), von Erkrankungen des Skelettknochens und der Gelenke sowie der Skelettmuskel (vielfältige Formen der Arthritis wie z.B. Arthritis alcaptonurica, Arthritis ankylosans, Arthritis dysenterica, Arthritis exsudativa, Arthritis fungosa, Arthritis gonorrhoica, Arthritis mutilans, Arthritis psoriatica, Arthritis purulenta, Arthritis rheumatica, Arthritis serosa, Arthritis syphilitica, Arthritis tuberculosa, Arthritis urica, Arthritis villonodularis pigmentosa, atypische Arthritis, hämophile Arthritis, juvenile chronische Arthritis, rheumatoide Arthritis und metastatische Arthritis, des weiteren das Still-Syndrom, Felty-Syndrom, Sjörgen-Syndrom, Clutton-Syndrom, Poncet-Syndrom, Pott-Syndrom und Reiter-Syndrom, vielfältige Formen der Arthropathien wie z.B. Arthropathie deformans, Arthropathie neuropathica, Arthropathie ovaripriva, Arthropathie psoriatica und Arthropathie tabica, systemische Sklerosen, vielfältige Formen der entzündlichen Myopathien wie z.B. Myopathie epidemica, Myopathie fibrosa, Myopathie myoglobinurica, Myopathie ossificans, Myopathie ossificans neurotica, Myopathie ossificans progressiva multiplex, Myopathie purulenta, Myopathie rheumatica, Myopathie trichinosa, Myopathie tropica und Myopathie typhosa, sowie das Günther-Syndrom und das Münchmeyer-Syndrom), von entzündlichen Arterienveränderungen (vielfältige Formen der Arteritis wie z.B. Endarteritis, Mesarteritis, Periarteritis, Panarteritis, Arteritis rheumatica, Arteritis deformans, Arteritis temporalis, Arteritis cranialis, Arteritis gigantocellularis und Arteritis granulomatosa, sowie das Horton-Syndrom, Churg-Strauss-Syndrom und die Takayasu-Arteritis), des Muckle-Well-Syndroms, der Kikuchi-Krankheit, von Polychondritis, Sklerodermia sowie von weiteren Erkrankungen mit einer entzündlichen oder immunologischen Komponente, wie beispielsweise Katarakt, Kachexie, Osteoporose, Gicht, Inkontinenz, Lepra, Sezary-Syndrom und paraneoplastisches Syndrom, bei Abstossungsreaktionen nach Organtransplantationen und zur Wundheilung und Angiogenese insbesondere bei chronischen Wunden.

Aufgrund ihres biochemischen und pharmakologischen Eigenschaftsprofils eignen sich die erfindungsgemäßen Verbindungen insbesondere zur Behandlung und/oder Prävention von inflammatorischen Lungenerkrankungen, vor allem der chronisch-obstruktiven Lungenerkrankung (COPD), des Lungenemphysems, der chronischen Bronchitis, der Bronchiektasie, der pulmonalen Hypertonie in der COPD (PH-COPD), der akuten Exazerbation in der COPD, der zystischen Fibrose (Mukoviszidose, CF), des Asthmas, sowie der idiopathischen Lungenfibrose (IPF), des Bronchiolitis obliterans-Syndrom (BOS), der Arteriosklerose, der Myokarditis, sowie entzündlichen Haut- und Augenerkrankungen bzw. entzündlichen Erkrankungen der inneren Organe.

Aufgrund ihres biochemischen und pharmakologischen Eigenschaftsprofils eignen sich die erfindungsgemäßen Verbindungen ganz im Besonderen zur Behandlung und/oder Prävention von inflammatorischen Lungenerkrankungen, vor allem der chronisch-obstruktiven Lungenerkrankung (COPD), des Lungenemphysems, der chronischen Bronchitis, der Bronchiektasie, der pulmonalen Hypertonie in der COPD (PH-COPD), der akuten Exazerbation in der COPD, der zystischen Fibrose (Mukoviszidose, CF), des Asthmas, sowie der idiopathischen Lungenfibrose (IPF) und des Bronchiolitis obliterans-Syndroms (BOS).

Die zuvor genannten, gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Weiterer Gegenstand der vorliegenden Erfindung sind somit die erfindungsgemäßen Verbindungen zur Verwendung in einem Verfahren zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Arzneimittel, enthaltend mindestens eine der erfindungsgemäßen Verbindungen, zur Behandlung und/oder Prävention von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als hierfür geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil, Tadalafil, Udenafil, Dasantafil, Avanafil, Mirodenafil oder Lodenafil;
- NO- und Häm-unabhängige Aktivatoren der löslichen Guanylatcyclase (sGC), wie insbesondere die in WO 01/19355, WO 01/19776, WO 01/19778, WO 01/19780, WO 02/070462 und WO 02/070510 beschriebenen Verbindungen;
- NO-unabhängige, jedoch Häm-abhängige Stimulatoren der löslichen Guanylatcyclase (sGC), wie insbesondere Riociguat sowie die in WO 00/06568, WO 00/06569, WO 02/42301, WO 03/095451, WO 2011/147809, WO 2012/004258, WO 2012/028647 und WO 2012/059549 beschriebenen Verbindungen;
- Prostacyclin-Analoga und IP-Rezeptor-Agonisten, wie beispielhaft und vorzugsweise I-loprost, Beraprost, Treprostinil, Epoprostenol oder Selexipag;
- Endothelin-Rezeptor-Antagonisten, wie beispielhaft und vorzugsweise Bosentan, Darusentan, Ambrisentan oder Sitaxsentan;
- Verbindungen, die die humane neutrophile Elastase (HNE) inhibieren, wie beispielhaft und vorzugsweise Sivelestat oder DX-890 (Reltran);
- die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren, wie beispielhaft und vorzugsweise Nintedanib, Dasatinib, Nilotinib, Bosutinib, Regorafenib, Sorafenib, Sunitinib, Cediranib, Axitinib, Telatinib, Imatinib, Brivanib, Pazopanib, Vatalanib, Gefitinib, Erlotinib, Lapatinib, Canertinib, Lestaurtinib, Pelitinib, Semaxanib oder Tandutinib;
- Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12);
- Verbindungen, die die Bindung von Serotonin an dessen Rezeptor blockieren, beispielhaft und vorzugsweise Antagonisten des 5-HT_{2B}-Rezeptors wie PRX-08066;
- Antagonisten von Wachstumsfaktoren, Zytokinen und Chemokinen, beispielhaft und vorzugsweise Antagonisten von TGF-β, CTGF, IL-1, IL-4, IL-5, IL-6, IL-8, IL-13 und Integrinen;
- die Rho-Kinase inhibierende Verbindungen, wie beispielhaft und vorzugsweise Fasudil, Y-27632, SLx-2119, BF-66851, BF-66852, BF-66853, KI-23095 oder BA-1049;
- Verbindungen, die die lösliche Epoxidhydrolase (sEH) inhibieren, wie beispielsweise *N,N'-*Dicyclohexylharnstoff, 12-(3-Adamantan-1-yl-ureido)-dodecansäure oder 1-Adamantan-1-yl-3-{5-[2-(2-ethoxyethoxy)ethoxy]pentyl}-harnstoff;
- den Energiestoffwechsel des Herzens beeinflussende Verbindungen, wie beispielhaft und vorzugsweise Etomoxir, Dichloracetat, Ranolazin oder Trimetazidin;
- anti-obstruktiv wirkende Mittel, wie sie z.B. zur Therapie der chronisch-obstruktiven Lungenerkrankung (COPD) oder eines Asthma bronchiale eingesetzt werden, beispielhaft und vorzugsweise aus der Gruppe der inhalativ oder systemisch angewendeten β-adrenergen Rezeptor-Agonisten (β-Mimetika) und der inhalativ angewendeten anti-muscarinergen Substanzen;
- entzündungshemmende, immunmodulierende, immunsuppressive und/oder zytotoxische Mittel, beispielhaft und vorzugsweise aus der Gruppe der systemisch oder inhalativ angewendeten Corticosteroide sowie Acetylcystein, Montelukast, Azathioprin, Cyclophosphamid, Hydroxycarbamid, Azithromycin, Pirfenidon oder Etanercept;
- antifibrotisch wirkende Mittel, wie beispielhaft und vorzugsweise Adenosin-A2b-Rezeptor-Antagonisten, Sphingosin-1-phosphat-Rezeptor 3 (S1P3)-Antagonisten, Autotaxin-Inhibitoren, Lysophosphatidsäure-Rezeptor 1 (LPA-1)- und Lysophosphatidsäure-Rezeptor 2 (LPA-2)-Antagonisten, Lysyloxidase (LOX)-Inhibitoren, Lysyloxidase-like-2-Inhibitoren, CTGF-Inhibitoren, IL-13-Antagonisten, αᵥβ₆-Integrin-Antagonisten, TGF-β-Antagonisten, Inhibitoren des Wnt-Signalwegs oder CCR2-Antagonisten;
- antithrombotisch wirkende Mittel, beispielhaft und vorzugsweise aus der Gruppe der Thrombozytenaggregationshemmer, der Antikoagulantien und der profibrinolytischen Substanzen;
- den Blutdruck senkende Wirkstoffe, beispielhaft und vorzugsweise aus der Gruppe der Calcium-Antagonisten, Angiotensin All-Antagonisten, ACE-Hemmer, Vasopeptidase-Inhibitoren, Endothelin-Antagonisten, Renin-Inhibitoren, α-Rezeptoren-Blocker, β-Rezeptoren-Blocker, Mineralocorticoid-Rezeptor-Antagonisten sowie der Diuretika;
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-α-, PPAR-γ- und/oder PPAR-δ-Agonisten, Cholesterin-Absorptionshemmer, Lipase-Inhibitoren, polymeren Gallensäureadsorber, Gallensäure-Reabsorptionshemmer und Lipoprotein(a)-Antagonisten; und/oder
- Chemotherapeutika, wie sie z.B. zur Therapie von Neubildungen (Neoplasien) der Lunge oder anderer Organe eingesetzt werden.

Bei einer bevorzugten Ausführungsform der Erfindung werden die erfindungsgemäßen Verbindungen in Kombination mit einem β-adrenergen Rezeptor-Agonisten, wie beispielhaft und vorzugsweise Albuterol, Isoproterenol, Metaproterenol, Terbutalin, Fenoterol, Formoterol, Reproterol, Salbutamol oder Salmeterol, verabreicht.

Bei einer bevorzugten Ausführungsform der Erfindung werden die erfindungsgemäßen Verbindungen in Kombination mit einer anti-muscarinergen Substanz, wie beispielhaft und vorzugsweise Ipratropiumbromid, Tiotropiumbromid oder Oxitropiumbromid, verabreicht.

Bei einer bevorzugten Ausführungsform der Erfindung werden die erfindungsgemäßen Verbindungen in Kombination mit einem Corticosteroid, wie beispielhaft und vorzugsweise Prednison, Prednisolon, Methylprednisolon, Triamcinolon, Dexamethason, Beclomethason, Betamethason, Flunisolid, Budesonid oder Fluticason, verabreicht.

Unter antithrombotisch wirkenden Mittel werden vorzugsweise Verbindungen aus der Gruppe der Thrombozytenaggregationshemmer, der Antikoagulantien und der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, α-Rezeptoren-Blocker, β-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem α₁-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem β-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan, Embursartan, Irbesartan, Olmesartan, Eprosartan oder Azilsartan oder einem dualen Angiotensin AII-Antagonisten/NEP-Inhibitor, wie beispielsweise und vorzugsweise LCZ696 (Valsartan/Sacubitril), verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton, Eplerenon oder Finerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-α-, PPAR-γ- und/oder PPAR-δ-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-γ-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Besonders bevorzugt sind Kombinationen der erfindungsgemäßen Verbindungen mit einem oder mehreren weiteren Wirkstoffen ausgewählt aus der Gruppe bestehend aus PDE 5-Inhibitoren, sGC-Aktivatoren, sGC-Stimulatoren, Prostacyclin-Analoga, IP-Rezeptor-Agonisten, Endothelin-Antagonisten, die Signaltransduktionskaskade inhibierenden Verbindungen und Pirfenidon.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat oder Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Dosieraerosole), Nasentropfen, -lösungen oder -sprays, lingual, sublingual oder buccal zu applizierende Tabletten, Filme/Oblaten oder Kapseln, Suppositorien, Ohrenoder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale und die parenterale Applikation, insbesondere die orale, die intravenöse und die intrapulmonale (inhalative) Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0,001 bis 1 mg/kg, vorzugsweise etwa 0,01 bis 0,5 mg/kg Körpergewicht zur Erzielung wirksamer Ergebnisse zu verabreichen. Bei oraler Applikation beträgt die Dosierung etwa 0,01 bis 100 mg/kg, vorzugsweise etwa 0,01 bis 20 mg/kg und ganz besonders bevorzugt 0,1 bis 10 mg/kg Körpergewicht. Bei intrapulmonaler Applikation beträgt die Menge im Allgemeinen etwa 0,1 bis 50 mg je Inhalation.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Weiterer Gegenstand der vorliegenden Erfindung sind Arzneimittel pharmazeutische Zusammensetzungen, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, vaginal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan, intravitreal oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Augentropfen, Augensalben, Augenbäder, okulare Inserte, Ohrentropfen, -sprays, -pulver, -spülungen, -tampons, , Vaginalkapseln, wässrige Suspensionen (Lotionen, Schüttelmixturen), lipophile Suspensionen, Emulsionen, Mikroemulsionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit pharmazeutisch geeigneten Hilfsstoffen geschehen. Zu diesen Hilfsstoffen zählen u.a.
- Füll- und Trägerstoffe (beispielsweise Cellulose, mikrokristalline Cellulose wie z.B. Avicel^{®}, Laktose, Mannitol, Stärke, Calciumphosphate wie z.B. Di-Cafos^{®}),
- Salbengrundlagen (beispielsweise Vaselin, Paraffine, Triglyceride, Wachse, Wollwachs, Wollwachsalkohole, Lanolin, hydrophile Salbe, Polyethylenglycole),
- Suppositoriengrundlagen (zum Beispiel Polyethylenglycole, Kakaobutter, Hartfett),
- Lösungsmittel (z.B. Wasser, Ethanol, Isopropanol, Glycerol, Propylenglycol, mittelkettige Triglyceride fette Öle, flüssige Polyethylenglycole, Paraffine),
- Tenside, Emulgatoren, Dispergier- oder Netzmittel (beispielsweise Natriumdodecylsulfat, Lecithin, Phospholipide, Fettalkohole wie z.B. Lanette^{®}, Sorbitanfettsäureester wie z.B. Span^{®}, Polyoxy-ethylen-Sorbitanfettsäureester wie z.B. Tween^{®}, Polyoxyethylen-Fettsäureglyceride wie z.B. Cremophor^{®}, Polyoxethlyen-Fettsäureester, Polyoxethlyen-Fettalkoholether, Glycerolfettsäureester, Poloxamere wie z.B. Pluronic^{®}),
- Puffersubstanzen sowie Säuren und Basen (beispielsweise Phosphate, Carbonate, Citronensäure, Essigsäure, Salzsäure, Natronlauge, Ammoniumcarbonat, Trometamol, Triethanolamin),
- Isotonisierungsmittel (beispielsweise Glucose, Natriumchlorid),
- Adsorptionsmittel (beispielsweise hochdisperse Siliciumdioxide),
- Viskositätserhöhende Mittel, Gelbildner, Verdickungs- bzw. Bindemittel (beispielsweise Polyvinylpyrrolidon, Methylcellulose, Hydroxypropylmethylcellulose, Hydroxypropylcellulose, Carboxymethylcellulose-Natrium, Stärke, Carbomere, Polyacrylsäuren wie z.B. Carbopol^{®}, Alginate, Gelatine),
- Sprengmittel (beispielsweise modifizierte Stärke, Carboxymethylcellulose-Natrium, Natriumstärkeglycolat wie z.B. Explotab^{®}, quervernetztes Polyvinylpyrrolidon, Croscarmellose-Natrium wie z.B. AcDiSol^{®}),
- Fließregulier-, Schmier-, Gleit- und Formtrennmittel (beispielsweise Magnesiumstearat, Stearinsäure, Talkum, hochdisperse Siliciumdioxide wie z.B. Aerosil^{®}),
- Überzugsmittel (beispielsweise Zucker, Schellac) sowie Filmbildemittel für sich schnell oder modifiziert auflösende Filme bzw. Diffusionsmembranen (beispielsweise Polyvinylpyrrolidone wie z.B. Kollidon^{®}, Polyvinylalkohol, Hydroxypropylmethylcellulose, Hydroxypropylcellulose, Ethylcellulose, Hydroxypropylmethylcellulosephthalat, Celluloseacetat, Celluloseacetatphthtalat, Polyacrylate, Polymethacrylate wie z.B. Eudragit^{®}),
- Kapselmaterialien (z.B. Gelatine, Hydroxypropylmethylcellulose),
- Synthetische Polymere (beispielsweise Polylactide, Polyglycolide, Polyacrylate, Polymethacrylate wie z.B Eudragit^{®}, Polyvinylpyrrolidone wie z.B. Kollidon^{®}, Polyvinylalcohole, Polyvinylacetate, Polyethylenoxide, Polyethylenglycole und deren Copolymere und Blockcopolymere),
- Weichmacher (beispielsweise Polyethylenglycole, Propylenglykol, Glycerol, Triacetin, Triacetylcitrat, Dibutylphthalat),
- Penetrationsenhancer,
- Stabilisatoren (z.B. Antioxidantien wie beispielsweise Ascorbinsäure, Ascorbylpalmitat, Natriumascorbat, Butylhydroxyanisol, Butylhydroxytoluol, Propylgallat),
- Konservierungsmittel (beispielsweise Parabene, Sorbinsäure, Thiomersal, Benzalkoniumchlorid, Chlorhexidinacetat, Natriumbenzoat),
- Farbstoffe (z.B. anorganische Pigmente wie beispielsweise Eisenoxide, Titandioxid),
- Aromen, Süßungsmittel, Geschmacks- und / oder Geruchskorrigentien.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.1 bis 6 mg/kg zur Erzielung wirksamer Ergebnisse zu verabreichen. Bei oraler Applikation beträgt die Dosierung etwa 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

**Abkürzungen und Akronyme:**

| | |
|---|---|
| Ac | Acetyl |
| aq. | wässrig, wässrige Lösung |
| boc | tert.-Butoxycarbonyl |
| br.d | breites Dublett (NMR) |
| br.m | breites Multiplett (NMR) |
| br.s | breites Singulett (NMR) |
| br.t | breites Triplett (NMR) |
| c | Konzentration |
| cat. | katalytisch |
| d | Dublett (NMR) |
| de | Diastereomerenüberschuss |
| DC | Dünnschichtchromatographie |
| DCI | direkte chemische Ionisation (bei MS) |
| dd | Dublett von Dubletten (NMR) |
| ddd | Dublett von Dubletten von Dubletten (NMR) |
| dest. | destilliert |
| DIAD | Diisopropylzaodicarboxylat |
| DIEA | *N*,*N*-Diisopropylethylamin |
| DMAP | 4-*N*,*N*-Dimethylaminopyridin |
| DMF | Dimethylformamid |
| DMSO | Dimethylsulfoxid |
| DSC | Differential Scanning Thermography |
| dt | Dublett von Tripletten (NMR) |
| d. Th. | der Theorie (bei Ausbeute) |
| EDC | *N'*-(3-Dimethylaniinopropyl)-*N*-ethylcarbodiimid-Hydrochlorid |
| ee | Enantiomerenüberschuss |
| ent | enantiomerenrein, Enantiomer |
| eq. | Äquivalent(e) |
| ESI | Elektrospray-Ionisation (bei MS) |
| Et | Ethyl |
| GC-MS | Gaschromatographie-gekoppelte Massenspektrometrie |
| h | Stunde(n) |
| HATU | *O*-(7-Azabenzotriazol-1-yl)-*N*,*N*,*N'*,*N'*-tetramethyluronium-hexafluorophosphat |
| HBTU | *O*-(Benzotriazol-1-yl)-*N*,*N*,*N'*,*N'*-tetramethyluronium-hexafluorphosphat |
| HOBt | 1-Hydroxy-1*H*-benzotriazol-Hydrat |
| HPLC | Hochdruck-, Hochleistungsflüssigchromatographie |
| ID | Innendurchmesser |
| iPr | iso-Propyl |
| J | Kopplungskonstante (NMR) |
| konz. | Konzentriert |
| LC-MS | Flüssigchromatographie-gekoppelte Massenspektrometrie |
| m | Multiplett (NMR) |
| Me | Methyl |
| min | Minute(n) |
| MPLC | Mitteldruckflüssigchromatographie |
| MS | Massenspektrometrie |
| MTBE | Methyl-*tert*.-butylether |
| NMR | Kernresonanzspektrometrie |
| Pd/C | Palladium auf Aktivkohle |
| Ph | Phenyl |
| PyBOP | Benzotriazol-1 -yloxy-tris(pyrrolidino)phosphonium-hexafluorophosphat |
| quant. | quantitativ (bei Ausbeute) |
| rac | racemisch, Racemat |
| RT | Raumtemperatur |
| Rₜ | Retentionszeit (bei HPLC) |
| Schmp. | Schmelzpunkt |
| SFC | Überkritische, superkritische Flüssigkeitschromatographie |
| t | Triplett (NMR) |
| tBu | *tert*.-Butyl |
| tert. | Tertiär |
| TFA | Trifluoressigsäure |
| TFAA | Trifluoressigsäureanhydrid |
| THF | Tetrahydrofuran |
| TPPO | Triphenylphosphinoxid |
| UV | Ultraviolett -Spektrometrie |
| vgl. | vergleiche |
| v/v | Volumen zu Volumen-Verhältnis (einer Lösung) |

**HPLC-, GC-MS und LC-MS-Methoden**

### Methode 1:

Instrument: Waters ACQUITY SQD UPLC System; Säule: Waters Acquity UPLC HSS T3 1.8 µ 50 x 1 mm; Eluent A: 1 I Wasser + 0.25 ml 99%ige Ameisensäure , Eluent B: 1 I Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 208 - 400 nm.

### Methode 2:

Gerätetyp MS: Thermo Scientific FT-MS; Gerätetyp UHPLC+: Thermo Scientific UltiMate 3000; Säule: Waters, HSST3, 2.1 x 75 mm, C18 1.8 µm; Eluent A: 1 l Wasser + 0.01% Ameisensäure; Eluent B: 1 l Acetonitril + 0.01% Ameisensäure; Gradient: 0.0 min 10% B → 2.5 min 95% B → 3.5 min 95% B; Ofen: 50°C; Fluss: 0.90 ml/min; UV- Detektion: 210 nm/ Optimum Integration Path 210-300 nm

### Methode 3:

Instrument: Agilent MS Quad 6150;HPLC: Agilent 1290; Säule: Waters Acquity UPLC HSS T3 1.8 µ 50 x 2.1 mm; Eluent A: 1 l Wasser + 0.25 ml 99%ige Ameisensäure , Eluent B: 1 l Acetonitril + 0.25 ml 99%ige Ameisensäure; Gradient: 0.0 min 90% A → 0.3 min 90% A → 1.7 min 5% A → 3.0 min 5% A Ofen: 50°C; Fluss: 1,20 ml/min; UV-De tektion: 205 - 305 nm.

### Methode 4:

Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

### Weitere Angaben:

Bei Aufreinigungen von erfindungsgemäßen Verbindungen per Chromatographie, vor allem per Säulenchromatographie, werden vorgepackte Kieselgel-Kartuschen, wie z. B. Biotage SNAP Kartuschen, KP-Sil^{®} oder KP-NH^{®} in Kombination mit einem Biotage-System (SP4^{®} oder Isolera Four^{®}) verwendet. Als Laufmittel finden Gradienten aus Hexan/Ethylacetat oder Dichlormethan/Methanol Anwendung.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen ausreichend basische bzw. saure Funktionalitäten enthalten. Ein solches Salz kann durch verschiedene dem Fachmann bekannte Methoden in die entsprechende freie Base bzw. Säure überführt werden.

Des Weiteren können Amidine als freie Verbindungen oder anteilig (abhängig von der Präparation bei Beteiligung von Essigsäure) als Acetat-Salze oder Acetat-Solvate vorliegen.

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x Salzsäure", "x CF₃COOH", "x Na+" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

Weiterhin können die erfindungsgemäßen sekundären Amide als Rotationsisomere/ Isomerengemische, insbesondere bei NMR-Untersuchungen, vorliegen. Reinheitsangaben beziehen sich in der Regel auf entsprechende Peak-Integrationen im LC/MS-Chromatogramm, können aber zusätzlich auch unter Zuhilfenahme des ¹H-NMR-Spektrums ermittelt worden sein. Wenn keine Reinheit angegeben ist, handelt es sich in der Regel um eine 100%-Reinheit laut automatischer Peak-Integration im LC/MS-Chromatogramm oder die Reinheit wurde nicht explizit ermittelt.

Angaben zu Ausbeuten in % d. Th. sind in der Regel reinheitskorrigiert, sofern eine Reinheit <100% angegeben ist. Bei lösungsmittelhaltigen oder verunreinigten Chargen kann die Ausbeute formal ">100%" betragen; in diesen Fällen ist die Ausbeute nicht lösungsmittel- bzw. reinheitskorrigiert.

Alle Angaben in ¹H-NMR-Spektren geben die Chemischen Verschiebungen δ[ppm] = in ppm an.

Die in den folgenden Paragraphen angegebenen Multiplizitäten von Protonensignalen in ¹H-NMR-Spektren geben die jeweils beobachtete Signalform wieder und berücksichtigen keine Signalphänomene höherer Ordnung. In der Regel bezieht sich die Angabe zur chemischen Verschiebung auf das Zentrum des betreffenden Signals. Bei breiten Multipletts erfolgt die Angabe eines Intervalls. Durch Lösungsmittel oder Wasser verdeckte Signale wurden entweder tentativ zugeordnet oder sind nicht aufgeführt. Stark verbreiterte Signale - z.B. verursacht durch schnelle Rotation von Molekülteilen oder aufgrund von austauschenden Protonen - wurden ebenfalls tentativ zugeordnet (oft als breites Multiplett oder breites Singulett bezeichnet) oder sind nicht aufgeführt.

Schmelzpunkte und Schmelzbereiche, soweit angegeben, sind nicht korrigiert.

Die ¹H-NMR-Daten ausgewählter Synthese-Intermediate und Ausführungsbeispiele werden in Form von ¹H-NMR-Peaklisten notiert. Zu jedem Signalpeak wird erst der δ[ppm] = -Wert in ppm und dann die Signalintensität in runden Klammem aufgeführt. Die δ[ppm] = -Wert-Signalintensitäts-Zahlenpaare von verschiedenen Signalpeaks werden durch Kommata voneinander getrennt aufgelistet. Die Peakliste eines Beispieles hat daher die Form: δ[ppm] = ₁ (Intensität₁), δ[ppm] = ₂ (lntensität₂), ... , δ[ppm] = ᵢ (Intensitätᵢ), ... , δ[ppm] = ₙ (Intensitätₙ).

Die Intensität scharfer Signale korreliert mit der Höhe der Signale in einem gedruckten Beispiel eines NMR-Spektrums in cm und zeigt im Vergleich mit anderen Signalen die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden. Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden. Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen. Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%). Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen. Ein Experte, der die Peaks der Zielverbindungen mit bekannten Verfahren (MestreC, ACD-Simulation, oder unter Verwendung von empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation. Eine detaillierte Beschreibung der Darstellung von NMR-Daten in Form von Peaklisten kann der Publikation "Citation of NMR Peaklist Data within Patent Applications" entnommen werden (vgl. Research Disclosure Database Number 605005, 2014, 1. August 2014 oder http://www.researchdisclosure.com/searching-disclosures). In der Peak Picking Routine, die in der Research Disclosure Database Number 605005 beschrieben ist, kann der Parameter "MinimumHeight" zwischen 1% und 4% eingestellt werden. Abhängig von der Art der chemischen Struktur und/oder abhängig von der Konzentration der zu vermessenden Verbindung kann es sinnvoll sein, den Parameter "MinimumHeight" auf Werte <1% einzustellen.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

Bei den im Folgenden beschriebenen Intermediaten und Ausführungsbeispielen bedeutet eine im IUPAC-Namen des betreffenden Beispiels aufgeführte Bezeichnung "5*RS*" in Verbindung mit der Angabe "Racemat", dass es sich hierbei um ein racemisches Gemisch des 5*R*-Enantiomeren (→1. Buchstabe nach der Positionsziffer in "5*RS*") mit dem entsprechenden 5S-Enantiomeren (→2. Buchstabe nach der Positionsziffer) in handelt. Die Bezeichnung "5*RS*" in Verbindung mit den Angaben "Enantiomer 1" und "Enantiomer 2" bedeutet, dass es sich hierbei um die beiden Enantiomere in separierter, isolierter Form handelt, wobei eine Zuordnung der Absolutkonfiguration (5*R* oder 5*S*) zu diesen Enantiomeren nicht vorgenommen wurde. Ähnliche Bezeichnungen wie "5SR ", die sich aus der veränderten Priorität und/oder Reihenfolge von Namensbestandteilen aufgrund der IUPAC-Nomenklatur-Regeln ergeben, sind nach dieser Anleitung auf analoge Weise zu interpretieren

Bei den im Folgenden beschriebenen Intermediaten und Ausführungsbeispielen bedeutet eine im IUPAC-Namen des betreffenden Beispiels aufgeführte Bezeichnung "5*RS*,7*RS*" in Verbindung mit der Angabe "Racemat", dass es sich hierbei um ein racemisches Gemisch des 5*R*,7*R*-Enantiomeren (→ jeweils 1. Buchstabe nach der Positionsziffer in "5*RS,*7*RS* ") mit dem entsprechenden 5S,7S -Enantiomeren (→ jeweils 2. Buchstabe nach der Positionsziffer) handelt. Die Bezeichnung "5*RS*,7*RS*" in Verbindung mit den Angaben "Enantiomer 1" und "Enantiomer 2" bedeutet, dass es sich hierbei um die beiden Enantiomere in separierter, isolierter Form handelt, wobei eine Zuordnung der Absolutkonfiguration (5*R,*7*R* oder 5S,7S) zu diesen Enantiomeren nicht vorgenommen wurde. Ähnliche Bezeichnungen wie "5*SR,*7*SR* ", die sich aus der veränderten Priorität und/oder Reihenfolge von Namensbestandteilen aufgrund der IUPAC-Nomenklatur-Regeln ergeben, sind nach dieser Anleitung auf analoge Weise zu interpretieren.

Die (5S)-Konfiguration wurde aufgrund von Kristallstrukturaufklärung für Beispiel 26, 108, 113, 157, 237, 358 und 454 zugeordnet. In Analogie dazu wurden für die Beispiele 183-188, 190-219, 275-279, 342-402, 404-415, 418-563 die (5S)-Konfiguration zugeordnet.

### Ausgangsverbindungen und Intermediate:

### Intermediat 1

### Methyl-3-fluor-2-(trifluormethyl)isonicotinat

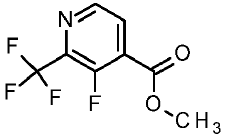

3-Fluor-2-(trifluormethyl)isonicotinsäure (1.00 g, 4.78 mmol) wurde in Methanol (10 ml) gelöst und mit Schwefelsäure (310 µl, 5.7 mmol) versetzt. Das Reaktionsgemisch wurde für 30 Minuten auf 60 °C erhitzt und anschließend über Nacht bei Raumtemperatur gerührt. Das Reaktionsgemisch wurde im Vakuum eingeengt, der Rückstand in Ethylacetat gelöst und mit gesättigter Natriumhydrogencarbonat-Lösung gewaschen. Die organische Phase wurde über Natriumsulfat getrocknet, filtriert und das Filtrat eingeengt. Es wurden 890 mg (70 % Reinheit, 58 % d. Th.) der Titelverbindung erhalten.

LC-MS (Methode 3): Rₜ = 1.15 min; MS (ESIpos): m/z = 224 [M+H]⁺

### Intermediat 2

### [3-Fluor-2-(trifluormethyl)pyridin-4-yl]methanol

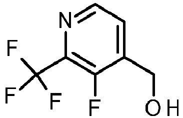

Methyl-3-fluor-2-(trifluormethyl)isonicotinat (890 mg, Reinheit 70%, 3.99 mmol) wurde in Methanol (5.0 ml) gelöst und bei 0°C portionsweise mit Natriumborhydrid (166 mg, 4.39 mmol) versetzt. Das Reaktionsgemisch wurde nach Rühren über Nacht bei Raumtemperatur mit gesättigter wässriger Ammoniumchlorid-Lösung versetzt und das Methanol im Vakuum entfernt. Der Rückstand wurde mit Ethylacetat und Wasser versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 420 mg (54 % d. Th.) der Titelverbindung erhalten. Die Verbindung wurde direkt weiter umgestzt.

### Intermediat 3

### 4-(Chlormethyl)-3-fluor-2-(trifluormethyl)pyridin Hydrochlorid

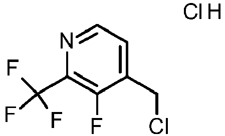

[3-Fluor-2-(trifluormethyl)pyridin-4-yl]methanol (420 mg, 2.15 mmol) wurde in Dichlormethan (10 ml) gelöst und bei Raumtemperatur mit Thionyldichlorid (310 µl, 4.3 mmol) versetzt. Nachdem das Reaktionsgemisch über Nacht bei Raumtemperatur gerührt wurde, wurde erneut Thionyldichlorid (310 µl, 4.3 mmol) und ein Tropfen Dimethylformamid zugegeben. Das Reaktionsgemisch wurde für eine Stunde bei Raumtemperatur gerührt und anschließend erneut mit Thionyldichlorid (620 µl, 8.6 mmol) und einem Tropfen Dimethylformamid versetzt. Nach erneutem Rühren für 2 Stunden bei Raumtemperatur wurde das Lösungsmittel unter Vakuum entfernt. Es wurden 379 mg (70 % d. Th.) der Titelverbindung erhalten.

GC-MS (Methode 4): Rₜ = 2.74 min; MS (ESIpos): m/z = 213 [M+H]⁺

### Intermediat 4

### (6-Hydrazinopyrazin-2-yl)(pyrrolidin-1-yl)methanon

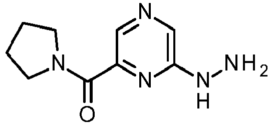

Eine Lösung aus (6-Chlorpyrazin-2-yl)(pyrrolidin-1-yl)methanon (710 mg, 3.35 mmol), Hydrazinhydrat (1.6 ml, 34 mmol) und Ethanol (7.1 ml) wurde für 2 h unter Rückfluss gerührt. Das Lösungsmittel wurde im Vakuum entfernt. Der Rückstand wurde in gesättigter wässriger Natriumhydrogencarbonat-Lösung aufgenommen und dann mit Dichlormethan extrahiert. Die wässrige Phase wurde mit Natriumchlorid gesättigt und erneut mit Dichlormethan und Ethylacetat extrahiert. Die organischen Phasen wurden vereinigt, über Magnesiumsulfat getrocknet und im Vakuum eingeengt und es wurden 275 mg (36 % d. Th.) der Titelverbindung erhalten.

¹H-NMR (400 MHz, DMSO-d6) δ [ppm]: 8.19-8.10 (m, 2H), 7.95 (s, 1H), 4.28 (s, 2H), 3.67-3.54 (m, 2H), 3.46 (t, 2H), 1.88-1.76 (m, 4H).

### Intermediat 5

### 5-(Pyrrolidin-1-ylcarbonyl)[1,2,4]triazolo[4,3-a]pyrazin-3(2H)-on

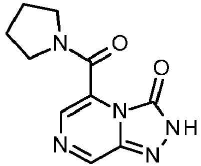

(6-Hydrazinopyrazin-2-yl)(pyrrolidin-1-yl)methanon (94.0 mg, 454 µmol) wurde in THF (1.0 ml) vorgelegt und mit Di-1H-imidazol-1-ylmethanon (88.3 mg, 544 µmol) versetzt und über Nacht bei Raumtemperatur gerührt. Das Lösungsmittel wurde in Vakuum entfernt und der Rückstand über Säulenchromatographie gereinigt (Kieselgel, Dichlormethan/Methanol 90/10). Die Produkthaltigen Fraktionen wurden im Vakuum eingeengt und es wurden 83.5 mg (76 % d. Th.) der Titelverbindung erhalten.

¹H-NMR (400 MHz, DMSO-d6) δ [ppm]: 1.801 (2.45), 1.816 (4.36), 1.832 (4.48), 1.843 (4.50), 1.857 (5.35), 1.873 (5.56), 1.891 (3.10), 1.906 (0.80), 3.163 (2.40), 3.175 (2.34), 3.218 (0.57), 3.246 (4.88), 3.262 (10.52), 3.278 (5.58), 3.441 (5.00), 3.458 (8.29), 3.475 (4.14), 4.075 (0.53), 4.088 (0.53), 5.754 (1.55), 7.025 (0.48), 7.455 (16.00), 8.878 (13.88), 13.046 (1.65).

### Intermediat 6

### tert-Butyl-(5RS)-3-oxo-5-(pyrrolidin-1-ylcarbonyl)-2,5,6,8-tetrahydro[1,2,4]triazolo[4,3-a]pyrazin-7(3H)-carboxylat (Racemat)

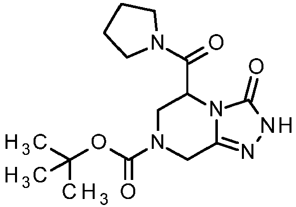

Unter Argon wurde Palladium auf Kohle (200 mg, 10%) vorgelegt und 5-(Pyrrolidin-1-ylcarbonyl)[1,2,4]triazolo[4,3-a]pyrazin-3(2H)-on (250 mg, 1.07 mmol) in 1,4-Dioxan (15 ml) zugegeben. Nach Zugabe von Di-tert-butyldicarbonat (250 µl, 1.1 mmol) wurde das Reaktionsgemisch über Nacht bei Raumtemperatur in einer Wasserstoffatmosphäre (3 bar) gerührt. Die Suspension wurde mit Ethylacetat verdünnt und über Kieselgur filtriert und mit Dichlormethan/Methanol 90/10 nachgewaschen. Das Reaktionsgemisch wurde eingeengt und es wurden 351 mg (92 % d. Th.) der Titelverbindung erhalten.

LC-MS (Methode 1): Rₜ = 0.60 min; MS (ESIneg): m/z = 336 [M-H]⁻

¹H-NMR (400 MHz, DMSO-d6) δ [ppm]: 1.110 (0.50), 1.349 (7.03), 1.468 (16.00), 1.574 (1.67), 1.784 (0.62), 1.807 (0.48), 1.935 (0.71), 1.951 (0.79), 3.335 (0.50), 3.352 (0.42), 3.479 (0.41), 3.567 (1.03), 4.286 (0.42), 4.322 (0.41), 4.723 (0.75), 11.548 (1.35).

### Intermediat 7

### tert-Butyl-(5RS)-3-oxo-5-(pyrrolidin-1-ylcarbonyl)-2,5,6,8-tetrahydro[1,2,4]triazolo[4,3-a]pyrazin-7(3H)-carboxylat (Enantiomer 1)

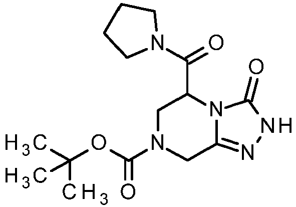

(tert-Butyl-(5RS)-3-oxo-5-(pyrrolidin-1-ylcarbonyl)-2,5,6,8-tetrahydro[1,2,4]triazolo[4,3-a]pyrazin-7(3H)-carboxylat (Racemat) wurde durch chirale präparative Flüssigchromatographie getrennt [Probenvorbereitung: 155 mg gelöst in 4 ml Ethanol und 2 mL Dichlormethan; Injektionsvolumen: 0.3 ml; Säule: Daicel Chirapak^{®} IF 5µm, 250 x 20 mm; Laufmittel: n-Heptan/Ethanol: isokratisch 50% Ethanol, Fluß: 15.0 ml/min; Temperatur 30°C; UV Detektion: 220 nm]. Nach der Trennung wurden 47.0 mg von Enantiomer 1, welches zuerst eluierte, und 54.0 mg von Enantiomer 2, welches später eluierte, isoliert.

### Enantiomer 1:

Analytische chirale HPLC: Rₜ = 6.84 min, e.e. = 100% [Säule: Daicel Chiralpak^{®} IF 5µm 250 x 4.6 mm; Laufmittel: n-Heptan/Ethanol 1.1; Fluß: 1 ml/min; UV Detektion: 220 nm] LC-MS (Methode 1): Rₜ = 0.59 min; MS (ESIpos): m/z = 338 [M+H]⁺

¹H-NMR (400 MHz, DMSO-d6) δ [ppm]: -0.008 (0.64), 0.008 (0.52), 1.349 (16.00), 1.760 (0.54), 1.772 (0.81), 1.789 (1.09), 1.807 (0.92), 1.825 (0.65), 1.844 (0.61), 1.859 (0.56), 1.936 (1.59), 1.951 (1.69), 3.280 (0.68), 3.335 (0.49), 3.353 (0.64), 3.462 (0.81), 3.479 (0.92), 3.517 (0.65), 3.554 (0.52), 3.648 (0.84), 4.051 (0.60), 4.093 (0.70), 4.286 (0.97), 4.322 (0.93), 4.723 (1.73), 4.781 (0.68), 4.823 (0.58), 11.547 (2.68).

### Intermediat 8

### tert-Butyl-(5RS)-3-oxo-5-(pyrrolidin-1-ylcarbonyl)-2,5,6,8-tetrahydro[1,2,4]triazolo[4,3-a]pyrazin-7(3H)-carboxylat (Enantiomer 2)

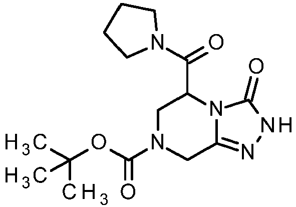

tert-Butyl-(5RS)-3-oxo-5-(pyrrolidin-1-ylcarbonyl)-2,5,6,8-tetrahydro[1,2,4]triazolo[4,3-a]pyrazin-7(3H)-carboxylat (Racemat) wurde durch chirale präparative Flüssigchromatographie getrennt [Probenvorbereitung: 155 mg gelöst in 4 ml Ethanol und 2 mL Dichlormethan; Injektionsvolumen: 0.3 ml; Säule: Daicel Chirapak^{®} IF 5µm, 250 x 20 mm; Laufmittel: n-Heptan/Ethanol: isokratisch 50% Ethanol, Fluß: 15.0 ml/min; Temperatur 30°C; UV Detektion: 220 nm]. Nach der Trennung wurden 47.0 mg von Enantiomer 1, welches zuerst eluierte, und 54.0 mg von Enantiomer 2, welches später eluierte, isoliert.

### Enantiomer 2:

Analytische chirale HPLC: Rₜ = 10.28 min, e.e. = 99% [Säule: Daicel Chiralpak^{®} IF 5µm 250 x 4.6 mm; Laufmittel: n-Heptan/Ethanol 1.1; Fluß: 1 ml/min; UV Detektion: 220 nm]

LC-MS (Methode 1): Rₜ = 0.59 min; MS (ESIneg): m/z = 336 [M-H]-¹H-NMR (400 MHz, DMSO-d6) δ [ppm]: 0.008 (0.93), 1.349 (16.00), 1.512 (0.94), 1.788 (1.33), 1.806 (0.99), 1.826 (0.73), 1.951 (1.74), 3.335 (0.96), 3.352 (0.82), 3.481 (0.94), 3.558 (0.52), 3.648 (0.86), 4.052 (0.59), 4.080 (0.62), 4.094 (0.72), 4.286 (0.98), 4.323 (0.92), 4.724 (1.70), 4.782 (0.71), 4.824 (0.58), 11.547 (1.12).

### Intermediat 9

### 6-Chlorpyrazin-2-carbonylchlorid

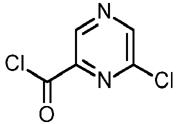

Thionylchlorid (23 ml, 320 mmol) wurde vorgelegt und 6-Chlorpyrazin-2-carbonsäure (5.00 g, 31.5 mmol) wurde langsam zugegeben. Das Reaktionsgemisch wurde für 2 Stunden bei 90°C gerührt. Die flüchtigen Bestandteile wurden im Vakuum entfernt und der Rückstand wurde zweimal in Toluol aufgenommen und eingeengt. Der Rückstand wurde ohne weitere Reinigung direkt weiter umgesetzt.

### Intermediat 10

### (6-Chlorpyrazin-2-yl)[(3S)-3-fluorpyrrolidin-1-yl]methanon

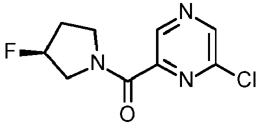

6-Chlorpyrazin-2-carbonylchlorid (5.58 g, 31.5 mmol) wurde in Dichlormethan (50 ml) gelöst und mit (3S)-3-Fluorpyrrolidinhydrochlorid (4.36 g, 34.7 mmol) versetzt. Anschließend wurde N,N-Diisopropylethylamin (16 ml, 95 mmol) zugetropft (exotherm). Das Reaktionsgemisch wurde über Nacht bei Raumtemperatur gerührt und anschließend mit Wasser und Dichlormethan verdünnt. Die Phasen wurden getrennt und die wässrige Phase wurde mit Dichlormethan extrahiert. Die vereinigten organischen Phasen wurden über Magnesiumsulfat getrocknet, filtriert und das Filtrat eingeengt. Es wurden 6.68 g (92 % d. Th.) der Titelverbindung erhalten.

LC-MS (Methode 2): Rₜ = 1.11 min; MS (ESIpos): m/z = 230 [M+H]⁺

¹H-NMR (400 MHz, DMSO-d6) δ [ppm]: 1.250 (6.35), 1.263 (6.11), 2.004 (0.46), 2.012 (0.52), 2.019 (0.53), 2.028 (1.10), 2.040 (1.69), 2.049 (1.65), 2.055 (1.81), 2.064 (2.32), 2.077 (2.04), 2.091 (1.81), 2.100 (1.10), 2.107 (1.03), 2.116 (1.01), 2.126 (0.50), 2.148 (2.83), 2.153 (2.86), 2.170 (5.19), 2.184 (4.15), 2.196 (5.12), 2.219 (4.96), 2.234 (2.71), 2.252 (1.26), 2.270 (0.65), 3.124 (0.50), 3.542 (2.11), 3.561 (2.39), 3.572 (4.20), 3.590 (4.45), 3.600 (3.17), 3.619 (2.88), 3.663 (1.51), 3.672 (1.51), 3.700 (3.01), 3.708 (4.91), 3.726 (2.09), 3.736 (4.54), 3.753 (4.84), 3.767 (7.80), 3.780 (3.21), 3.797 (8.57), 3.804 (8.14), 3.827 (6.72), 3.851 (4.81), 3.877 (6.98), 3.886 (3.98), 3.900 (2.61), 3.914 (4.49), 3.948 (1.60), 3.978 (10.49), 5.317 (4.25), 5.322 (4.22), 5.330 (4.07), 5.338 (2.03), 5.452 (4.33), 5.463 (3.82), 8.947 (12.80), 8.953 (12.24), 8.983 (15.72), 8.986 (16.00).

### Intermediat 11

### [(3S)-3-Fluorpyrrolidin-1-yl](6-hydrazinopyrazin-2-yl)methanon

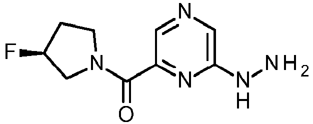

Eine Lösung aus (6-Chlorpyrazin-2-yl)[(3S)-3-fluorpyrrolidin-1-yl]methanon (1.00 g, 4.35 mmol) in THF (25 ml) wurde auf 3 Mikrowellengefäße (30 ml) verteilt und jeweils mit Hydrazin (11.7 ml, 1.0 M, 11.7 mmol) versetzt. Die Reaktionsgemische wurden für 4 Stunden bei 100°C unter Mikrowelleneinstrahlung gerührt. Es wurde festes Natriumhydrogencarbonat und Magnesiumsulfat zugegeben und für 5 Minuten bei Raumtemperatur gerührt. Die Feststoffe wurden abfiltriert und mit Essigester nachgewaschen. Die organische Phase wurde eingeengt. Es wurden 934 mg (80 % Reinheit, 76 % d. Th.) der Titelverbindung erhalten.

¹H-NMR (400 MHz, DMSO-d6) δ [ppm]: -0.150 (0.46), -0.008 (3.67), 0.008 (3.57), 0.146 (0.49), 1.157 (4.10), 1.174 (8.20), 1.192 (4.19), 1.234 (0.41), 1.355 (8.09), 1.742 (0.51), 1.988 (16.00), 2.021 (1.24), 2.037 (0.90), 2.045 (1.26), 2.072 (0.67), 2.080 (0.83), 2.092 (0.98), 2.100 (0.74), 2.120 (2.18), 2.128 (2.38), 2.134 (2.10), 2.164 (2.87), 2.176 (2.97), 2.183 (3.61), 2.216 (1.02), 2.327 (0.51), 2.366 (0.44), 2.523 (1.60), 2.669 (0.54), 2.710 (0.48), 3.479 (1.09), 3.496 (1.23), 3.508 (2.12), 3.526 (2.19), 3.537 (1.49), 3.554 (1.35), 3.571 (0.47), 3.589 (0.52), 3.600 (1.19), 3.609 (0.88), 3.617 (0.46), 3.637 (1.53), 3.645 (1.62), 3.707 (2.75), 3.733 (3.25), 3.742 (3.02), 3.754 (3.60), 3.761 (2.67), 3.770 (3.87), 3.781 (3.95), 3.798 (3.26), 3.809 (2.20), 3.826 (2.13), 3.853 (1.93), 3.859 (2.39), 3.880 (3.03), 3.895 (1.94), 3.903 (2.59), 3.958 (2.37), 3.976 (1.22), 3.993 (2.77), 4.002 (2.97), 4.020 (6.05), 4.038 (3.75), 4.056 (1.85), 4.298 (11.88), 4.534 (0.64), 4.545 (0.64), 5.272 (1.94), 5.284 (1.33), 5.295 (1.95), 5.303 (1.20), 5.329 (0.45), 5.405 (1.89), 5.428 (1.86), 6.870 (0.71), 8.008 (8.72), 8.032 (8.87), 8.085 (0.91), 8.146 (10.29), 8.151 (10.35), 8.215 (4.30), 8.946 (1.40), 8.952 (1.37), 8.981 (1.65), 8.985 (1.72).

### Intermediat 12

### 5-{[(3S)-3-Fluorpyrrolidin-1-yl]carbonyl}[1,2,4]triazolo[4,3-a]pyrazin-3(2H)-on

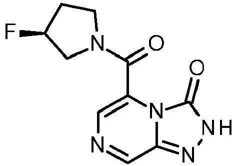

[(3S)-3-Fluorpyrrolidin-1-yl](6-hydrazinopyrazin-2-yl)methanon (930 mg, 80 % Reinheit, 3.30 mmol) wurde in THF (20 ml) gelöst und mit Di-1H-imidazol-1-ylmethanon (562 mg, 3.47 mmol) versetzt. Das Reaktionsgemisch wurde 1 Stunde bei Raumtemperatur und 15 Minuten bei 50°C gerührt. Das Reaktionsgemisch wurde eingeengt und über Säulenchromatographie (Kieselgel, Laufmittel: Dichlormethan/Methanol-Gradient) gereinigt. So wurden 487mg (56 % d. Th.) der Titelverbindung erhalten.

LC-MS (Methode 1): Rₜ = 0.25 min; MS (ESIpos): m/z = 252 [M+H]⁺

¹H-NMR (500 MHz, DMSO-d6) δ [ppm]: 1.168 (0.73), 2.037 (0.49), 2.058 (0.47), 2.093 (0.77), 2.105 (0.82), 2.132 (1.10), 2.142 (0.97), 2.175 (0.55), 2.191 (0.45), 2.216 (0.60), 2.227 (0.48), 3.385 (0.64), 3.400 (0.68), 3.483 (0.58), 3.504 (1.07), 3.519 (1.20), 3.541 (1.02), 3.640 (0.45), 3.647 (0.40), 3.669 (1.22), 3.676 (1.36), 3.684 (1.57), 3.733 (1.20), 3.743 (2.86), 3.752 (1.47), 3.773 (0.72), 5.260 (0.94), 5.365 (1.58), 5.469 (0.76), 5.752 (16.00), 7.480 (3.13), 7.505 (1.68), 8.893 (2.81), 8.900 (7.48), 13.038 (0.55).

### Intermediat 13

### tert-Butyl-(5RS)-5-{[(3S)-3-fluorpyrrolidin-1-yl]carbonyl}-3-oxo-2,5,6,8-tetrahydro[1,2,4]triazolo[4,3-a]pyrazin-7(3H)-carboxylat (Diastereomerengemisch; 2 Isomere)

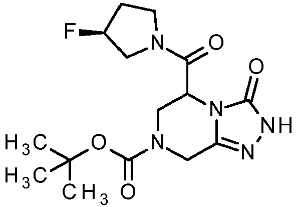

Unter Argon wurde Palladium auf Kohle (357 mg, 10%) vorgelegt und 55-{[(3S)-3-Fluorpyrrolidin-1-yl]carbonyl}[1,2,4]triazolo[4,3-a]pyrazin-3(2H)-on (480 mg, 1.91 mmol) in 1,4-Dioxan (25 ml) zugegeben. Nach Zugabe von Di-tert-butyldicarbonat (440 µl, 1.9 mmol) wurde das Reaktionsgemisch über Nacht bei Raumtemperatur in einer Wasserstoffatmosphäre (1 bar) gerührt. Es wurde Palladium auf Kohle (357 mg, 10%) zugegeben und erneut für 20 Stunden bei Raumtemperatur in einer Wasserstoffatmosphäre (1 bar) gerührt. Die Suspension wurde mit Ethylacetat verdünnt und über Kieselgur filtriert und mit Ethylacetat nachgewaschen. Das Reaktionsgemisch wurde eingeengt und über Säulenchromatographie (Kieselgel, Laufmittel: Dichlormethan/Methanol-Gradient) gereinigt. So wurden 486 mg (72 % d. Th.) der Titelverbindung erhalten.

LC-MS (Methode 1): Rₜ = 0.59 min; MS (ESIneg): m/z = 354 [M-H]⁻

¹H-NMR (400 MHz, DMSO-d6) δ [ppm]: -0.008 (0.80), 0.008 (0.64), 1.343 (16.00), 2.120 (0.45), 2.150 (0.49), 2.229 (0.47), 2.245 (0.47), 2.278 (0.54), 2.523 (0.42), 3.455 (0.46), 3.499 (0.70), 3.529 (1.29), 3.563 (1.16), 3.627 (0.54), 3.661 (0.60), 3.787 (0.60), 3.797 (0.56), 3.867 (0.80), 3.995 (0.48), 4.048 (0.78), 4.069 (0.51), 4.090 (0.92), 4.258 (0.88), 4.294 (0.78), 4.306 (0.57), 4.323 (0.62), 4.342 (0.56), 4.359 (0.53), 4.640 (0.56), 4.723 (0.46), 4.784 (0.66), 4.828 (1.15), 4.851 (1.42), 4.858 (1.30), 5.396 (0.59), 5.529 (0.51), 5.753 (1.98), 11.574 (3.14). (Mischung von Diastereomeren)

### Intermediat 14

### 2-(4-Methylbenzyl)-5-(pyrrolidin-1-ylcarbonyl)[1,2,4]triazolo[4,3-a]pyrazin-3(2H)-on

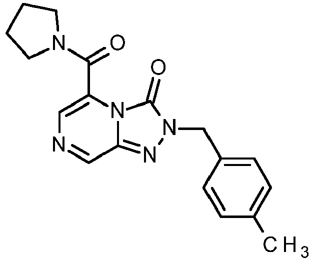

5-(Pyrrolidin-1-ylcarbonyl)[1,2,4]triazolo[4,3-a]pyrazin-3(2H)-on (50.0 mg, 214 µmol) wurde in Acetonitril (2.0 ml) vorgelegt. Cäsiumcarbonat (105 mg, 322 µmol) und 1-(Brommethyl)-4-methylbenzol (41.7 mg, 225 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnesiumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 36.6 mg (49 % d. Th.) der Titelverbindung erhalten.

LC-MS (Methode 1): Rₜ = 0.77 min; MS (ESIpos): m/z = 338 [M+H]⁺

¹H-NMR (400 MHz, DMSO-d6) δ [ppm]: 1.806 (1.05), 1.821 (1.85), 1.837 (1.78), 1.851 (1.76), 1.866 (2.18), 1.882 (2.41), 1.900 (1.37), 2.276 (16.00), 2.326 (0.59), 3.269 (2.31), 3.285 (4.98), 3.302 (3.31), 3.311 (3.20), 3.452 (2.43), 3.469 (4.04), 3.486 (2.00), 5.081 (3.37), 7.149 (3.09), 7.169 (5.92), 7.209 (6.56), 7.229 (3.41), 7.524 (6.43), 8.890 (5.13).

### Intermediat 15

### 5-{[(3S)-3-Fluorpyrrolidin-1-yl]carbonyl}-2-{[6-(trifluormethyl)pyridin-3-yl]methyl}[1,2,4]triazolo[4,3-a]pyrazin-3(2H)-on

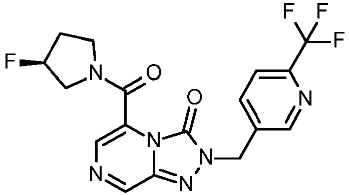

5-{[(3S)-3-Fluorpyrrolidin-1-yl]carbonyl}[1,2,4]triazolo[4,3-a]pyrazin-3(2H)-on (90.0 mg, 358 µmol) wurde in Acetonitril (2.7 ml) vorgelegt. Caesiumcarbonat (350 mg, 1.07 mmol) und 5-(Chlormethyl)-2-(trifluormethyl)pyridinhydrochlorid (91.4 mg, 394 µmol) wurden anschließend zugegeben. Das Reaktionsgemisch wurde nach Rühren über Nacht bei Raumtemperatur im Vakuum eingeengt. Der Rückstand wurde über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 86.0 mg (59 % d. Th.) der Titelverbindung erhalten.

LC-MS (Methode 2): Rₜ = 1.25 min; MS (ESIpos): m/z = 411 [M+H]⁺

¹H-NMR (400 MHz, DMSO-d6) δ [ppm]: -0.149 (0.83), -0.008 (6.09), 0.008 (5.53), 0.146 (0.74), 1.243 (0.83), 1.259 (0.83), 1.273 (0.51), 2.143 (0.78), 2.292 (0.74), 2.323 (1.52), 2.327 (2.07), 2.366 (1.80), 2.523 (5.07), 2.665 (1.48), 2.670 (2.03), 2.710 (1.57), 3.483 (16.00), 3.759 (2.21), 5.108 (0.55), 5.259 (0.78), 5.333 (5.35), 5.491 (0.41), 6.945 (6.27), 7.073 (6.69), 7.201 (6.27), 7.569 (1.98), 7.592 (0.92), 7.904 (2.58), 7.925 (3.37), 8.033 (1.57), 8.053 (1.06), 8.770 (1.98), 8.925 (1.52), 8.932 (2.90).

### Ausführungsbeispiele:

### Beispiel 1

### tert-Butyl-(5RS)-2-(4-methylbenzyl)-3-oxo-5-(pyrrolidin-1-ylcarbonyl)-2,5,6,8-tetrahydro[1,2,4]triazolo[4,3-a]pyrazin-7(3H)-carboxylat (Racemat)

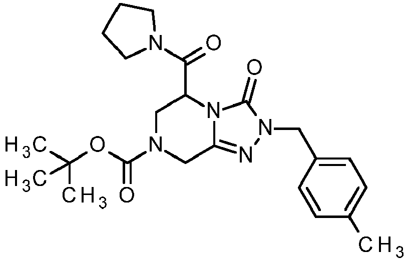

tert-Butyl-(5RS)-3-oxo-5-(pyrrolidin-1-ylcarbonyl)-2,5,6,8-tetrahydro[1,2,4]triazolo[4,3-a]pyrazin-7(3H)-carboxylat (Racemat) (175 mg, 519 µmol) und Cäsiumcarbonat (254 mg, 778 µmol) wurden in Acetonitril (5.0 ml) vorgelegt und 1-(Brommethyl)-4-methylbenzol (101 mg, 545 µmol) wurde anschließend zugegeben. Nach Rühren über Nacht bei Raumtemperatur wurde das Reaktionsgemisch mit Acetonitril (5.0 ml) verdünnt und für 1 h unter Rückfluss gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnesiumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 122 mg (53 % d. Th.) der Titelverbindung erhalten.

LC-MS (Methode 2): Rₜ = 1.71 min; MS (ESIpos): m/z = 442 [M+H]⁺

¹H-NMR (400 MHz, DMSO-d6) δ [ppm]: -0.008 (0.42), 1.346 (11.49), 1.775 (0.44), 1.791 (0.58), 1.809 (0.48), 1.940 (0.93), 1.956 (0.98), 2.274 (11.81), 3.480 (0.55), 3.668 (0.48), 4.113 (0.41), 4.299 (0.56), 4.335 (0.53), 4.745 (0.41), 4.784 (3.68), 4.792 (3.75), 4.809 (1.22), 4.830 (0.64), 7.135 (16.00).

### Beispiel 2

### (5RS)-2-(4-Methylbenzyl)-5-(pyrrolidin-1-ylcarbonyl)-5,6,7,8-tetrahydro[1,2,4]triazolo[4,3-a]pyrazin-3(2H)-on (Racemat)

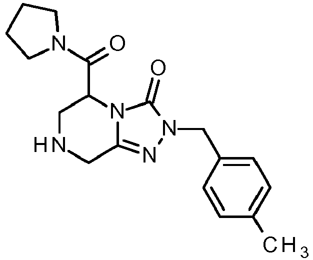

Tert-Butyl-(5RS)-2-(4-methylbenzyl)-3-oxo-5-(pyrrolidin-1-ylcarbonyl)-2,5,6,8-tetrahydro[1,2,4]triazolo[4,3-a]pyrazin-7(3H)-carboxylat (Racemat) (114 mg, 258 µmol) wurde in Dichlormethan (2.0 ml) gelöst und bei Raumtemperatur mit Trifluoressigsäure (1.0 ml) versetzt. Nachdem das Reaktionsgemisch für 30 min bei Raumtemperatur gerührt wurde, wurde Dichlormethan zugegeben und gesättigte wässrige Natriumhydrogencarbonatlösung zugegeben. Die Phasen wurden getrennt und die wässrige Phase wurde mit Dichlormethan extrahiert. Die vereinigten organischen Phasen wurden über Magnesiumsulfat getrocknet, filtriert und das Filtrat eingeengt. Es wurden 86.0 mg (96 % d. Th.) der Titelverbindung erhalten.

LC-MS (Methode 1): Rₜ = 0.57 min; MS (ESIpos): m/z = 342 [M+H]⁺

¹H-NMR (400 MHz, DMSO-d6) δ [ppm]: -0.008 (0.41), 1.537 (0.43), 1.772 (1.31), 1.789 (2.29), 1.806 (1.76), 1.823 (0.52), 1.887 (0.53), 1.903 (1.57), 1.920 (1.96), 1.937 (1.18), 2.271 (10.88), 2.384 (0.60), 3.044 (0.65), 3.054 (0.66), 3.079 (0.94), 3.089 (0.93), 3.214 (0.86), 3.227 (0.95), 3.248 (0.73), 3.261 (1.15), 3.272 (0.76), 3.289 (1.31), 3.329 (0.73), 3.347 (1.23), 3.359 (0.45), 3.365 (0.65), 3.377 (0.66), 3.464 (0.78), 3.471 (0.58), 3.482 (0.48), 3.489 (0.97), 3.506 (0.43), 3.616 (0.48), 3.630 (1.22), 3.641 (0.53), 3.650 (0.58), 3.657 (0.82), 3.671 (2.59), 3.706 (2.47), 3.747 (0.82), 4.591 (0.88), 4.603 (1.51), 4.614 (0.85), 4.716 (0.45), 4.754 (3.17), 4.766 (3.13), 4.804 (0.45), 7.126 (16.00).

### Beispiel 3

### tert-Butyl-(5RS)-2-{[3-chlor-4-(trifluormethyl)pyridin-2-yl]methyl}-5-{[(3S)-3-fluorpyrrolidin-1-yl]carbonyl}-3-oxo-2,5,6,8-tetrahydro[1,2,4]triazolo[4,3-a]pyrazin-7(3H)-carboxylat (Diastereomerengemisch; 2 Isomere)

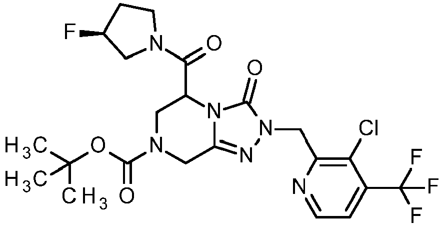

tert-Butyl-(5RS)-5-{[(3S)-3-fluorpyrrolidin-1-yl]carbonyl}-3-oxo-2,5,6,8-tetrahydro[1,2,4]triazolo[4,3-a]pyrazin-7(3H)-carboxylat (Diastereomerengemisch; 2 Isomere) (421 mg, 1.18 mmol) wurde in Acetonitril (10 ml) vorgelegt. Cäsiumcarbonat (965 mg, 2.96 mmol) und 3-Chlor-2-(chlormethyl)-4-(trifluormethyl)pyridinhydrochlorid (434 mg, 80 % Reinheit, 1.30 mmol) wurden zugegeben. Das Reaktionsgemisch wurde über Nacht bei Raumtemperatur gerührt. Nach 1 Stunde bei 80°C wird das Re aktionsgemisch über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 150 mg (92 % Reinheit, 21 % d. Th.) der Titelverbindung erhalten

LC-MS (Methode 3): Rₜ = 1.20 min; MS (ESIpos): m/z = 493 M-56+

¹H-NMR (400 MHz, DMSO-d6) δ [ppm]: -0.008 (3.72), 0.008 (1.32), 1.344 (16.00), 1.399 (1.62), 1.477 (0.70), 2.155 (0.51), 2.249 (1.02), 2.284 (0.51), 2.327 (0.61), 2.366 (0.44), 2.519 (2.96), 2.524 (3.11), 3.073 (4.33), 3.530 (4.91), 3.585 (0.89), 3.624 (0.91), 3.803 (0.78), 3.879 (0.68), 4.088 (0.70), 4.129 (0.80), 4.271 (0.80), 4.308 (0.85), 4.355 (0.60), 4.763 (0.71), 4.828 (0.64), 4.852 (0.63), 4.977 (0.89), 4.985 (0.80), 5.146 (0.93), 5.152 (1.05), 5.187 (2.96), 5.192 (3.28), 5.214 (2.36), 5.218 (2.46), 5.226 (2.06), 5.259 (0.79), 5.267 (0.82), 5.404 (0.61), 5.477 (0.51), 5.534 (0.48), 7.854 (3.88), 7.867 (3.92), 7.883 (0.62), 8.740 (2.93), 8.752 (2.80), 8.771 (0.43). (Mischung von Diastereomeren)

### Beispiel 4

### (5RS)-2-{[3-Chlor-4-(trifluormethyl)pyridin-2-yl]methyl}-5-{[(3S)-3-fluorpyrrolidin-1-yl]carbonyl}-5,6,7,8-tetrahydro[1,2,4]triazolo[4,3-a]pyrazin-3(2H)-on (Diastereomerengemisch; 2 Isomere)

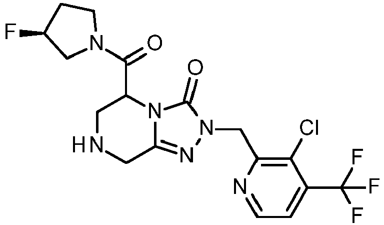

tert-Butyl-(5RS)-2-{[3-chlor-4-(trifluormethyl)pyridin-2-yl]methyl}-5-{[(3S)-3-fluorpyrrolidin-1-yl]carbonyl}-3-oxo-2,5,6,8-tetrahydro[1,2,4]triazolo[4,3-a]pyrazin-7(3H)-carboxylat (Diastereomerengemisch; 2 Isomere) (143 mg, 92 % Reinheit, 240 µmol) wurde in Dichlormethan (1.8 ml) und Trifluoressigsäure (900 µl) gelöst und für 30 Minuten bei Raumtemperatur gerührt. Die flüchtigen Bestandteile wurden im Vakuum entfernt und der Rückstand wurde über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 99.6 mg (93 % d. Th.) der Titelverbindung erhalten.

LC-MS (Methode 2): Rₜ = 1.07 min; MS (ESIpos): m/z = 449 [M+H]⁺

¹H-NMR (400 MHz, DMSO-d6) δ [ppm]: -0.008 (3.04), 0.008 (2.76), 1.953 (0.79), 1.980 (0.88), 2.012 (0.44), 2.065 (0.84), 2.073 (1.06), 2.090 (2.39), 2.106 (2.04), 2.126 (1.84), 2.140 (2.29), 2.155 (1.71), 2.218 (2.00), 2.232 (1.82), 2.250 (2.15), 2.263 (2.20), 2.297 (0.69), 2.327 (0.89), 2.366 (0.95), 2.524 (2.07), 2.670 (0.69), 2.710 (0.63), 2.988 (1.09), 2.999 (1.12), 3.022 (1.35), 3.033 (1.37), 3.063 (1.47), 3.074 (2.44), 3.086 (2.48), 3.097 (2.60), 3.110 (3.12), 3.121 (3.40), 3.196 (1.76), 3.210 (1.94), 3.232 (1.52), 3.248 (2.72), 3.285 (6.60), 3.355 (3.62), 3.373 (2.95), 3.383 (2.15), 3.401 (1.31), 3.408 (1.36), 3.417 (1.24), 3.480 (1.14), 3.509 (2.07), 3.516 (2.14), 3.540 (1.96), 3.550 (1.67), 3.564 (1.39), 3.574 (3.17), 3.589 (1.95), 3.607 (4.76), 3.636 (2.65), 3.647 (4.32), 3.659 (2.95), 3.678 (8.56), 3.688 (11.87), 3.714 (11.37), 3.720 (10.26), 3.744 (2.34), 3.756 (2.95), 3.763 (4.15), 3.792 (1.11), 3.803 (1.15), 3.813 (1.86), 3.823 (1.87), 3.898 (2.84), 3.950 (1.22), 3.970 (2.61), 3.996 (1.85), 4.026 (1.14), 4.057 (0.85), 4.578 (1.39), 4.591 (2.66), 4.603 (1.36), 4.637 (1.85), 4.649 (3.21), 4.661 (1.84), 4.671 (1.75), 4.683 (2.72), 4.694 (1.66), 4.714 (1.76), 4.726 (3.15), 4.737 (1.69), 5.122 (3.17), 5.128 (2.79), 5.163 (12.90), 5.169 (14.29), 5.181 (12.67), 5.190 (12.97), 5.222 (2.29), 5.230 (3.41), 5.262 (2.27), 5.271 (2.17), 5.345 (1.45), 5.396 (2.22), 5.477 (1.35), 5.510 (1.02), 7.844 (15.37), 7.856 (16.00), 8.347 (0.56), 8.736 (11.56), 8.748 (11.27). (Mischung von Diastereomeren)

### Beispiel 5

### tert-Butyl-(5RS)-5-{[(3S)-3-fluorpyrrolidin-1-yl]carbonyl}-2-[4-fluor-3-(trifluormethyl)benzyl]-3-oxo-2,5,6,8-tetrahydro[1,2,4]triazolo[4,3-a]pyrazin-7(3H)-carboxylat (Diastereomerengemisch; 2 Isomere)

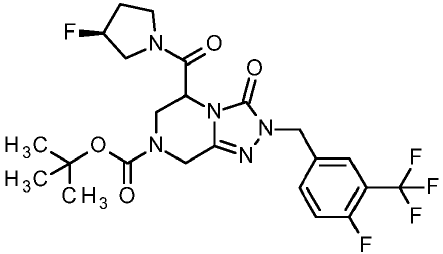

tert-Butyl-(5RS)-5-{[(3S)-3-fluorpyrrolidin-1-yl]carbonyl}-3-oxo-2,5,6,8-tetrahydro[1,2,4]triazolo[4,3-a]pyrazin-7(3H)-carboxylat (Diastereomerengemisch; 2 Isomere) (421 mg, 1.18 mmol) und Cäsiumcarbonat (965 mg, 2.96 mmol) wurden in Acetonitril (10 ml) suspendiert und 4-(Brommethyl)-1-fluor-2-(trifluormethyl)benzol (335 mg, 1.30 mmol) wurde zugegeben. Das Reaktionsgemisch wurde über Nacht bei Raumtemperatur gerührt und anschließend mit Ethylacetat verdünnt. Die organische Phase wurde mit Wasser gewaschen. Die wässrige Phase wurde anschließend mit Ethylacetat extrahiert. Die vereinigten organischen Phasen wurden über Magnesiumsulfat getrocknet, filtriert und das Filtrat eingeengt. Es wurden 608 mg (92 % d. Th.) der Titelverbindung erhalten.

LC-MS (Methode 2): Rₜ = 1.84 min; MS (ESIpos): m/z = 476 [M+H]⁺

¹H-NMR (400 MHz, DMSO-d6) δ [ppm]: 0.808 (0.43), 0.824 (0.42), 0.833 (0.58), 0.840 (0.97), 0.854 (0.59), 1.106 (4.99), 1.343 (16.00), 1.423 (1.13), 1.514 (3.80), 2.126 (0.41), 2.156 (0.43), 2.235 (0.42), 2.283 (0.48), 3.077 (1.57), 3.453 (0.42), 3.557 (0.85), 3.577 (0.82), 3.613 (0.79), 3.646 (0.57), 3.683 (0.56), 3.806 (0.80), 3.881 (0.48), 4.021 (0.41), 4.085 (0.62), 4.128 (0.76), 4.283 (0.68), 4.320 (0.63), 4.353 (0.49), 4.368 (0.52), 4.389 (0.40), 4.743 (0.48), 4.795 (0.85), 4.835 (0.78), 4.963 (6.80), 5.405 (0.55), 5.535 (0.43), 7.490 (1.02), 7.512 (1.86), 7.538 (1.65), 7.599 (1.19), 7.697 (1.49), 7.713 (1.49). (Mischung von Diastereomeren)

### Beispiel 6

### (5RS)-5-{[(3S)-3-Fluorpyrrolidin-1-yl]carbonyl}-2-[4-fluor-3-(trifluormethyl)benzyl]-5,6,7,8-tetrahydro[1,2,4]triazolo[4,3-a]pyrazin-3(2H)-on (Diastereomerengemisch; 2 Isomere)

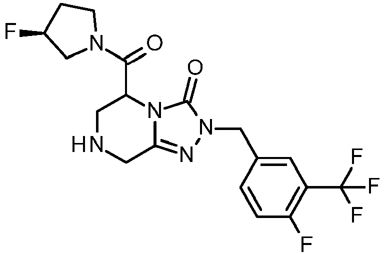

tert-Butyl-(5RS)-5-{[(3S)-3-fluorpyrrolidin-1-yl]carbonyl}-2-[4-fluor-3-(trifluormethyl)benzyl]-3-oxo-2,5,6,8-tetrahydro[1,2,4]triazolo[4,3-a]pyrazin-7(3H)-carboxylat (Diastereomerengemisch; 2 Isomere) (600 mg, 95 % Reinheit, 1.07 mmol) wurde in Dichlormethan (8.0 ml) gelöst und mit Trifluoressigsäure (4.0 ml) versetzt. Das Reaktionsgemisch wurde für 30 Minuten bei Raumtemperatur gerührt. Anschließend wurden alle flüchtigen Bestandteile im Vakuum entfernt und der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 355 mg (77 % d. Th.) der Titelverbindung erhalten.

LC-MS (Methode 2): Rₜ = 1.23 min; MS (ESIpos): m/z = 432 [M+H]⁺

¹H-NMR (400 MHz, DMSO-d6) δ [ppm]: -0.008 (2.40), 0.008 (1.89), 1.958 (0.49), 1.984 (0.56), 2.064 (0.57), 2.073 (0.75), 2.093 (1.37), 2.115 (1.11), 2.129 (1.07), 2.142 (1.25), 2.155 (0.96), 2.198 (0.89), 2.218 (1.05), 2.234 (1.12), 2.248 (1.28), 2.263 (1.23), 2.328 (0.64), 2.366 (0.56), 2.523 (1.76), 2.670 (0.59), 2.710 (0.44), 2.997 (0.57), 3.008 (0.59), 3.033 (0.70), 3.043 (0.71), 3.084 (0.90), 3.095 (1.43), 3.107 (1.22), 3.119 (1.39), 3.130 (2.17), 3.142 (1.91), 3.187 (1.24), 3.199 (1.36), 3.221 (0.90), 3.238 (1.60), 3.252 (1.73), 3.266 (2.51), 3.279 (3.79), 3.351 (1.93), 3.362 (1.82), 3.379 (1.60), 3.391 (0.98), 3.409 (0.98), 3.466 (0.50), 3.475 (0.54), 3.502 (0.69), 3.514 (1.15), 3.523 (1.19), 3.534 (1.66), 3.552 (0.85), 3.568 (1.56), 3.604 (2.80), 3.625 (0.54), 3.643 (2.29), 3.651 (3.11), 3.684 (4.29), 3.695 (6.56), 3.726 (5.31), 3.733 (6.32), 3.760 (1.67), 3.768 (2.19), 3.774 (2.43), 3.792 (0.73), 3.802 (1.06), 3.811 (1.30), 3.884 (1.28), 3.937 (0.73), 3.959 (1.85), 3.985 (0.77), 4.019 (0.69), 4.051 (0.53), 4.570 (0.66), 4.582 (1.15), 4.594 (0.65), 4.630 (0.94), 4.642 (1.45), 4.653 (0.88), 4.680 (1.03), 4.692 (1.59), 4.702 (1.01), 4.719 (1.07), 4.731 (1.73), 4.742 (1.03), 4.930 (16.00), 5.264 (1.20), 5.272 (1.21), 5.345 (0.84), 5.403 (1.23), 5.476 (0.82), 5.511 (0.50), 7.481 (2.52), 7.504 (4.42), 7.530 (4.05), 7.579 (2.54), 7.585 (2.66), 7.592 (2.96), 7.606 (1.73), 7.612 (1.62), 7.683 (3.32), 7.699 (3.31), 8.217 (0.49). (Mischung von Diastereomeren)

### Beispiel 7

### (5RS)-5-{[(3S)-3-Fluorpyrrolidin-1-yl]carbonyl}-2-[4-fluor-3-(trifluormethyl)benzyl]-5,6,7,8-tetrahydro[1,2,4]triazolo[4,3-a]pyrazin-3(2H)-on (Isomer 1)

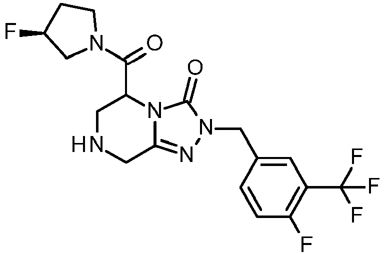

(5RS)-5-{[(3S)-3-Fluorpyrrolidin-1-yl]carbonyl}-2-[4-fluor-3-(trifluormethyl)benzyl]-5,6,7,8-tetrahydro[1,2,4]triazolo[4,3-a]pyrazin-3(2H)-on (Diastereomerengemisch; 2 Isomere) wurde durch chirale präparative Flüssigchromatographie getrennt [Probenvorbereitung: 340 mg gelöst in 5 ml Ethanol; Injektionsvolumen: 0.25 ml; Säule: Daicel Chirapak^{®} IE 5µm, 250 x 20 mm; Laufmittel: n-Heptan/Ethanol: isokratisch 40% Ethanol, Fluß: 20.0 ml/min; Temperatur 40°C; UV Detektion: 210 nm]. Nach der Trennung wurd en 121 mg von Isomer 1, welches zuerst eluierte, und 165 mg von Isomer 2, welches später eluierte, isoliert.

### Isomer 1:

Analytische chirale HPLC: Rₜ = 2.24 min, d.e. = 100% [Säule: Daicel Chiralpak^{®} IF-3 3µm 50 x 4.6 mm; Laufmittel: n-Heptan/Ethanol 1:1; Fluß: 1 ml/min; UV Detektion: 220 nm]

LC-MS (Methode 2): Rₜ = 1.24 min; MS (ESIpos): m/z = 432 [M+H]⁺

¹H-NMR (400 MHz, DMSO-d6) δ [ppm]: -0.008 (1.93), 0.008 (1.10), 1.038 (0.72), 1.046 (0.44), 1.056 (1.35), 1.064 (0.80), 1.073 (0.72), 1.082 (0.42), 1.959 (0.42), 1.983 (0.47), 2.063 (0.47), 2.073 (0.55), 2.091 (1.08), 2.107 (1.12), 2.139 (1.23), 2.157 (0.87), 2.213 (1.02), 2.234 (1.16), 2.263 (1.40), 2.328 (1.33), 2.366 (1.10), 2.519 (2.24), 2.524 (2.10), 2.670 (0.47), 2.710 (0.44), 3.003 (0.78), 3.038 (0.95), 3.100 (1.02), 3.136 (1.40), 3.280 (3.75), 3.354 (0.99), 3.368 (0.89), 3.377 (0.95), 3.404 (1.02), 3.412 (0.99), 3.426 (0.44), 3.438 (0.53), 3.456 (0.47), 3.466 (0.78), 3.474 (0.80), 3.501 (1.08), 3.510 (0.95), 3.626 (0.99), 3.650 (4.00), 3.668 (2.58), 3.680 (4.63), 3.685 (4.51), 3.705 (4.19), 3.733 (4.11), 3.759 (2.69), 3.780 (1.54), 3.811 (2.48), 3.884 (2.58), 4.337 (0.40), 4.570 (1.46), 4.582 (2.33), 4.594 (1.35), 4.631 (1.97), 4.641 (2.88), 4.653 (1.76), 4.888 (0.57), 4.927 (16.00), 4.968 (0.57), 5.263 (1.46), 5.388 (1.48), 5.394 (1.52), 5.511 (0.99), 7.481 (2.48), 7.503 (4.19), 7.529 (3.77), 7.579 (2.46), 7.585 (2.60), 7.591 (2.77), 7.606 (1.65), 7.612 (1.48), 7.677 (3.56), 7.695 (3.47).

### Beispiel 8

### (5RS)-5-{[(3S)-3-Fluorpyrrolidin-1-yl]carbonyl}-2-[4-fluor-3-(trifluormethyl)benzyl]-5,6,7,8-tetrahydro[1,2,4]triazolo[4,3-a]pyrazin-3(2H)-on (Isomer 2)

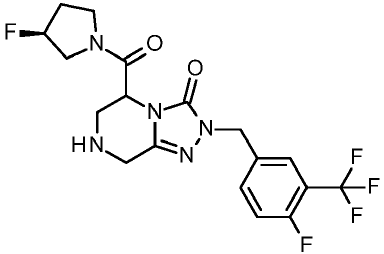

(5RS)-5-{[(3S)-3-Fluorpyrrolidin-1-yl]carbonyl}-2-[4-fluor-3-(trifluormethyl)benzyl]-5,6,7,8-tetrahydro[1,2,4]triazolo[4,3-a]pyrazin-3(2H)-on (Diastereomerengemisch; 2 Isomere) wurde durch chirale präparative Flüssigchromatographie getrennt [Probenvorbereitung: 340 mg gelöst in 5 ml Ethanol; Injektionsvolumen: 0.25 ml; Säule: Daicel Chirapak^{®} IE 5µm, 250 x 20 mm; Laufmittel: n-Heptan/Ethanol: isokratisch 40% Ethanol, Fluß: 20.0 ml/min; Temperatur 40°C; UV Detektion: 210 nm]. Nach der Trennung wurd en 121 mg von Isomer 1, welches zuerst eluierte, und 165 mg von Isomer 2, welches später eluierte, isoliert.

### Enantiomer 2:

Analytische chirale HPLC: Rₜ = 4.20 min, d.e. = 100% [Säule: Daicel Chiralpak^{®} IF-3 3µm 50 x 4.6 mm; Laufmittel: n-Heptan/Ethanol 1:1; Fluß: 1 ml/min; UV Detektion: 220 nm]

LC-MS (Methode 2): Rₜ = 1.24 min; MS (ESIpos): m/z = 432 [M+H]⁺

¹H-NMR (400 MHz, DMSO-d6) δ [ppm]: -0.008 (0.87), 0.008 (0.68), 1.113 (0.70), 1.958 (0.40), 1.984 (0.46), 2.065 (0.51), 2.093 (1.29), 2.116 (1.04), 2.126 (0.93), 2.144 (1.01), 2.198 (1.01), 2.219 (0.87), 2.234 (0.80), 2.248 (1.18), 2.328 (0.68), 2.333 (0.66), 2.366 (0.70), 2.432 (0.76), 2.519 (1.10), 2.524 (0.87), 3.100 (0.97), 3.135 (1.75), 3.193 (1.02), 3.232 (0.99), 3.244 (1.18), 3.260 (0.84), 3.281 (0.87), 3.352 (1.01), 3.363 (1.56), 3.380 (1.50), 3.391 (0.99), 3.409 (0.78), 3.515 (1.40), 3.523 (1.63), 3.534 (2.35), 3.552 (1.14), 3.563 (2.14), 3.568 (2.22), 3.574 (1.88), 3.588 (1.18), 3.605 (3.95), 3.647 (0.93), 3.663 (1.06), 3.693 (2.68), 3.703 (2.24), 3.732 (3.19), 3.770 (1.50), 3.792 (0.87), 3.800 (0.78), 3.937 (1.02), 3.959 (2.68), 3.986 (1.12), 4.018 (0.97), 4.050 (0.78), 4.680 (1.46), 4.692 (2.26), 4.702 (1.44), 4.720 (1.56), 4.731 (2.47), 4.742 (1.48), 4.932 (16.00), 4.970 (0.53), 5.273 (1.25), 5.344 (1.20), 5.403 (1.21), 5.476 (1.18), 7.482 (2.01), 7.504 (3.59), 7.530 (3.23), 7.579 (2.13), 7.585 (2.26), 7.592 (2.51), 7.606 (1.46), 7.613 (1.37), 7.684 (3.09), 7.701 (3.15).

### Beispiel 9

### tert-Butyl-(5RS)-5-{[(3S)-3-fluorpyrrolidin-1-yl]carbonyl}-2-(4-methylbenzyl)-3-oxo-2,5,6,8-tetrahydro[1,2,4]triazolo[4,3-a]pyrazin-7(3H)-carboxylat (Diastereomerengemisch; 2 Isomere)

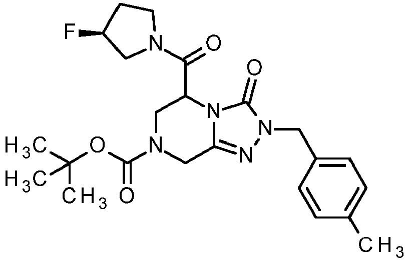

tert-Butyl-(5RS)-5-{[(3S)-3-fluorpyrrolidin-1-yl]carbonyl}-3-oxo-2,5,6,8-tetrahydro[1,2,4]triazolo[4,3-a]pyrazin-7(3H)-carboxylat (Diastereomerengemisch; 2 Isomere) (200 mg, 563 µmol) und Cäsiumcarbonat (275 mg, 844 µmol) wurden in Acetonitril (5.0 ml) suspendiert und 1-(Brommethyl)-4-methylbenzol (109 mg, 591 µmol) wurde zugegeben. Das Reaktionsgemisch wurde für 4.5 stunden bei Raumtemperatur gerührt und anschließend mit Ethylacetat verdünnt. Die organische Phase wurde mit Wasser gewaschen. Die wässrige Phase wurde anschließend mit Ethylacetat extrahiert. Die vereinigten organischen Phasen wurden über Magnesiumsulfat getrocknet, filtriert und das Filtrat eingeengt. Es wurden 246 mg (91 % d. Th.) der Titelverbindung erhalten.

LC-MS (Methode 1): Rₜ = 0.91 min; MS (ESIpos): m/z = 460 [M+H]⁺

¹H-NMR (500 MHz, DMSO-d6) δ [ppm]: 1.160 (0.62), 1.174 (1.24), 1.188 (0.64), 1.340 (11.14), 1.987 (2.36), 2.244 (0.48), 2.274 (16.00), 2.294 (1.51), 2.299 (1.12), 3.498 (0.41), 3.523 (0.46), 3.565 (0.70), 3.599 (0.61), 3.808 (0.60), 3.868 (0.42), 4.022 (0.73), 4.036 (0.70), 4.071 (0.54), 4.105 (0.64), 4.272 (0.62), 4.301 (0.51), 4.320 (0.41), 4.349 (0.46), 4.673 (0.79), 4.751 (0.47), 4.762 (0.43), 4.783 (2.41), 4.793 (4.44), 4.797 (4.00), 4.823 (0.70), 4.829 (0.70), 4.923 (0.48), 4.929 (0.54), 4.940 (0.82), 4.946 (0.75), 5.414 (0.55), 7.135 (12.68), 7.139 (9.65). (Mischung von Diastereomeren)

### Beispiel 10

### (5RS)-5-{[(3S)-3-Fluorpyrrolidin-1-yl]carbonyl}-2-(4-methylbenzyl)-5,6,7,8-tetrahydro[1,2,4]triazolo[4,3-a]pyrazin-3(2H)-on (Diastereomerengemisch; 2 Isomere)

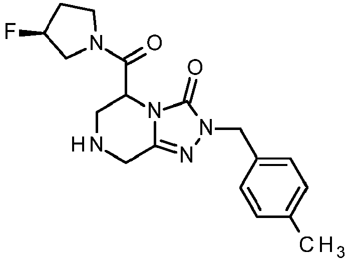

tert-Butyl-(5RS)-5-{[(3S)-3-fluorpyrrolidin-1-yl]carbonyl}-2-(4-methylbenzyl)-3-oxo-2,5,6,8-tetrahydro[1,2,4]triazolo[4,3-a]pyrazin-7(3H)-carboxylat (Diastereomerengemisch; 2 Isomere) (240 mg, 522 µmol) wurde in Dichlormethan (4.0 ml) und Trifluoressigsäure (2.0 ml) gelöst. Das Reaktionsgemisch wurde für 20 Minuten bei Raumtemperatur gerührt und anschließend mit Dichlormethan verdünnt. Die organische Phase wurde mit gesättigter wässriger Natriumhydrogencarbonat-Lösung gewaschen. Die wässrige Phase wurde mit Dichlormethan extrahiert. Die vereinigten organischen Phasen wurden über Magnesiumsulfat getrocknet, filtriert und das Filtrat eingeengt. Es wurden 170 mg (90 % Reinheit, 82 % d. Th.) der Titelverbindung erhalten.

LC-MS (Methode 2): Rₜ = 1.00 min; MS (ESIpos): m/z = 360 [M+H]⁺

¹H-NMR (400 MHz, DMSO-d6) δ [ppm]: -0.008 (0.58), 0.008 (0.52), 2.094 (0.45), 2.113 (0.46), 2.251 (0.80), 2.272 (14.07), 3.120 (0.50), 3.155 (0.82), 3.229 (0.42), 3.271 (0.56), 3.284 (0.77), 3.316 (3.54), 3.362 (0.66), 3.379 (0.72), 3.390 (0.41), 3.407 (0.44), 3.539 (0.46), 3.547 (0.50), 3.574 (0.68), 3.609 (0.85), 3.662 (0.42), 3.674 (0.62), 3.687 (0.97), 3.715 (1.30), 3.729 (1.43), 3.761 (2.57), 3.802 (0.96), 3.831 (0.40), 3.905 (0.43), 3.977 (0.42), 4.651 (0.47), 4.689 (0.52), 4.722 (0.56), 4.735 (0.67), 4.747 (0.42), 4.760 (1.75), 4.772 (3.47), 4.816 (0.40), 5.385 (0.43), 7.109 (0.77), 7.131 (16.00). (Mischung von Diastereomeren)

### Beispiel 11

### (5RS)-5-{[(3S)-3-Fluorpyrrolidin-1-yl]carbonyl}-7-methyl-2-(4-methylbenzyl)-5,6,7,8-tetrahydro[1,2,4]triazolo[4,3-a]pyrazin-3(2H)-on (Diastereomerengemisch; 2 Isomere)

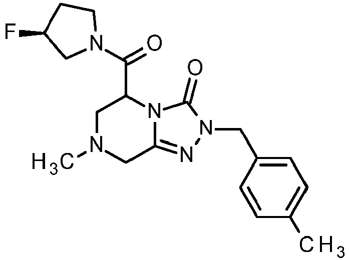

(5RS)-5-{[(3S)-3-Fluorpyrrolidin-1-yl]carbonyl}-2-(4-methylbenzyl)-5,6,7,8-tetrahydro[1,2,4]triazolo[4,3-a]pyrazin-3(2H)-on (Diastereomerengemisch; 2 Isomere) (80.0 mg, 90 % Reinheit, 200 µmol)wurde in THF (2.0 ml) vorgelegt und mit N,N-Diisopropylethylamin (70 µl, 400 µmol) versetzt. Nach Zugabe von lodmethan (19 µl, 300 µmol) wurde über Nacht bei Raumtemperatur gerührt. Nach Zugabe von lodmethan (8.5 µl, 150 µmol) und N,N-Diisopropylethylamin (35 µl, 200 µmol) wurden 3 h bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 42.7 mg (57 % d. Th.) der Titelverbindung erhalten.

LC-MS (Methode 1): Rₜ = 0.66 min; MS (ESIpos): m/z = 374 [M+H]⁺

¹H-NMR (500 MHz, DMSO-d6) δ [ppm]: -0.007 (0.50), 2.144 (0.45), 2.233 (0.44), 2.274 (16.00), 2.296 (5.31), 2.307 (12.12), 2.855 (0.45), 2.868 (0.70), 2.876 (1.59), 2.886 (1.52), 2.947 (0.44), 2.958 (0.47), 3.216 (0.84), 3.246 (1.05), 3.257 (0.82), 3.271 (0.76), 3.288 (1.35), 3.303 (1.90), 3.314 (2.42), 3.352 (0.71), 3.381 (0.44), 3.396 (0.55), 3.404 (0.42), 3.448 (0.71), 3.483 (0.94), 3.496 (0.98), 3.514 (1.85), 3.526 (1.47), 3.545 (1.21), 3.571 (0.49), 3.600 (0.41), 3.669 (0.60), 3.751 (0.67), 3.817 (0.43), 3.875 (0.47), 3.961 (0.49), 4.688 (0.55), 4.738 (0.83), 4.749 (0.46), 4.766 (3.61), 4.771 (4.75), 4.813 (0.42), 4.823 (0.88), 4.833 (0.43), 4.841 (0.45), 4.851 (0.92), 4.861 (0.41), 5.383 (0.44), 5.390 (0.51), 7.110 (0.96), 7.114 (0.86), 7.130 (11.55), 7.146 (0.98). (Mischung von Diastereomeren)

### Beispiel 12

### (5RS)-5-{[(3S)-3-Fluorpyrrolidin-1-yl]carbonyl}-7-methyl-2-(4-methylbenzyl)-5,6,7,8-tetrahydro[1,2,4]triazolo[4,3-a]pyrazin-3(2H)-on (Isomer 1)

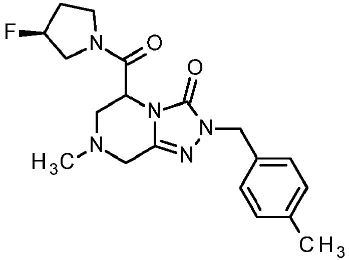

(5RS)-5-{[(3S)-3-Fluorpyrrolidin-1-yl]carbonyl}-7-methyl-2-(4-methylbenzyl)-5,6,7,8-tetrahydro[1,2,4]triazolo[4,3-a]pyrazin-3(2H)-on (Diastereomerengemisch; 2 Isomere) wurde durch chirale präparative Flüssigchromatographie getrennt [Probenvorbereitung: 185.5 mg gelöst in 8 ml warmem Ethanol; Injektionsvolumen: 0.5 ml; Säule: Daicel Chirapak^{®} IC 5µm, 250 x 20 mm; Laufmittel: n-Heptan/Ethanol: isokratisch 70% Ethanol, Fluß: 15.0 ml/min; Temperatur 25°C; UV Detektion: 210 nm]. Nach der Trenn ung wurden 86.2 mg von Isomer 1, welches zuerst eluierte, und 54.2 mg von Isomer 2, welches später eluierte, isoliert.

### Isomer 1:

Analytische chirale HPLC: Rₜ = 3.23 min, d.e. = 100% [Säule: Daicel Chiralpak^{®} IC-3 3µm 50 x 4.6 mm; Laufmittel: n-Heptan/Ethanol 1:1 ; Fluß: 1 ml/min; UV Detektion: 220 nm]

LC-MS (Methode 2): Rₜ = 1.20 min; MS (ESIpos): m/z = 374 [M+H]⁺

¹H-NMR (400 MHz, DMSO-d6) δ [ppm]: 0.008 (0.72), 2.095 (0.48), 2.113 (0.58), 2.141 (0.45), 2.188 (0.47), 2.238 (0.58), 2.274 (16.00), 2.296 (7.60), 2.308 (8.02), 2.852 (0.47), 2.875 (2.24), 2.887 (2.54), 2.944 (0.72), 2.957 (0.78), 2.975 (0.41), 3.212 (1.16), 3.250 (2.02), 3.291 (1.54), 3.379 (0.58), 3.397 (0.57), 3.493 (1.61), 3.512 (2.95), 3.531 (1.71), 3.550 (1.59), 3.565 (0.63), 3.582 (1.17), 3.600 (0.62), 3.608 (0.77), 3.938 (0.42), 3.963 (0.79), 3.984 (0.69), 4.772 (7.89), 4.812 (0.66), 4.824 (1.35), 4.837 (1.02), 4.851 (1.40), 4.864 (0.62), 5.269 (0.53), 5.341 (0.53), 5.405 (0.54), 5.474 (0.51), 7.109 (0.63), 7.131 (14.87), 7.150 (0.72).

### Beispiel 13

### (5RS)-5-{[(3S)-3-Fluorpyrrolidin-1-yl]carbonyl}-7-methyl-2-(4-methylbenzyl)-5,6,7,8-tetrahydro[1,2,4]triazolo[4,3-a]pyrazin-3(2H)-on (Isomer 2)

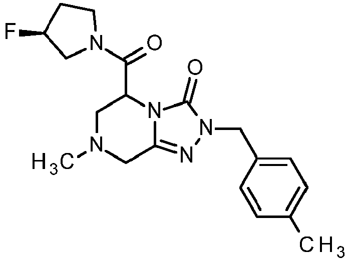

(5RS)-5-{[(3S)-3-Fluorpyrrolidin-1-yl]carbonyl}-7-methyl-2-(4-methylbenzyl)-5,6,7,8-tetrahydro[1,2,4]triazolo[4,3-a]pyrazin-3(2H)-on (Diastereomerengemisch; 2 Isomere) wurde durch chirale präparative Flüssigchromatographie getrennt [Probenvorbereitung: 185.5 mg gelöst in 8 ml warmem Ethanol; Injektionsvolumen: 0.5 ml; Säule: Daicel Chirapak^{®} IC 5µm, 250 x 20 mm; Laufmittel: n-Heptan/Ethanol: isokratisch 70% Ethanol, Fluß: 15.0 ml/min; Temperatur 25°C; UV Detektion: 210 nm]. Nach der Trenn ung wurden 86.2 mg von Isomer 1, welches zuerst eluierte, und 54.2 mg von Isomer 2, welches später eluierte, isoliert.

### Isomer 2:

Analytische chirale HPLC: Rₜ = 2.08 min, d.e. = 100% [Säule: Daicel Chiralpak^{®} IC-3 3µm 50 x 4.6 mm; Laufmittel: n-Heptan/Ethanol 1:1 ; Fluß: 1 ml/min; UV Detektion: 220 nm]

LC-MS (Methode 1): Rₜ = 0.72 min; MS (ESIpos): m/z = 374 [M+H]^{+ 1}H-NMR (400 MHz, DMSO-d6) δ [ppm]: -0.008 (0.54), 0.008 (0.52), 2.096 (0.44), 2.114 (0.42), 2.131 (0.53), 2.233 (0.45), 2.274 (15.49), 2.307 (16.00), 2.722 (0.42), 2.737 (0.43), 2.754 (0.50), 2.768 (0.50), 2.839 (0.49), 2.852 (0.52), 2.870 (0.72), 2.884 (0.72), 2.988 (0.68), 3.002 (0.75), 3.020 (0.58), 3.031 (0.69), 3.040 (0.58), 3.059 (0.42), 3.267 (1.18), 3.289 (0.58), 3.355 (1.41), 3.445 (1.39), 3.481 (2.05), 3.494 (0.47), 3.518 (1.15), 3.645 (0.61), 3.662 (1.32), 3.688 (0.96), 3.695 (0.73), 3.728 (0.96), 3.752 (0.86), 3.810 (0.97), 3.883 (1.03), 4.674 (0.52), 4.688 (1.10), 4.702 (0.54), 4.725 (0.83), 4.738 (1.60), 4.752 (0.96), 4.765 (7.33), 5.256 (0.56), 5.381 (0.68), 5.388 (0.70), 7.106 (0.84), 7.128 (11.71), 7.150 (0.96).

### Beispiel 14

### (5RS)-7-(Cyclopropylcarbonyl)-2-(4-methylbenzyl)-5-(pyrrolidin-1-ylcarbonyl)-5,6,7,8-tetrahydro[1,2,4]triazolo[4,3-a]pyrazin-3(2H)-on (Racemate)

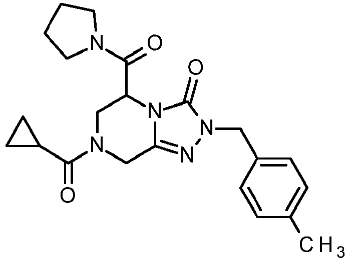

(5RS)-2-(4-Methylbenzyl)-5-(pyrrolidin-1-ylcarbonyl)-5,6,7,8-tetrahydro[1,2,4]triazolo[4,3-a]pyrazin-3(2H)-on (Racemate) (50.0 mg, 85 % Reinheit, 124 µmol) wurde in Dichlormethan (1.0 ml) und N,N-Diisopropylethylamin (43 µl, 250 µmol) gelöst und mit Cyclopropancarbonylchlorid (12 µl, 140 µmol) versetzt. Das Reaktionsgemisch wurde 1 Stunde bei Raumtemperatur gerührt Anschließend wurden alle flüchtigen Bestandteile im Vakuum entfernt und der Rückstand wurde in Acetonitril/ Wasser aufgenommen und über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 43.5 mg (85 % d. Th.) der Titelverbindung erhalten.

LC-MS (Methode 2): Rₜ = 1.33 min; MS (ESIpos): m/z = 410 [M+H]⁺

¹H-NMR (400 MHz, DMSO-d6) δ [ppm]: -0.008 (0.76), 0.008 (0.57), 0.605 (0.69), 0.690 (1.35), 0.743 (0.83), 1.768 (1.02), 1.785 (1.09), 1.801 (0.90), 1.923 (1.86), 1.938 (1.94), 1.954 (1.21), 2.274 (16.00), 3.261 (0.69), 3.640 (1.20), 3.655 (1.80), 3.926 (0.41), 3.960 (0.44), 4.045 (0.57), 4.087 (0.62), 4.617 (0.93), 4.654 (0.87), 4.795 (4.48), 4.834 (0.57), 4.913 (0.88), 5.063 (0.66), 5.106 (0.81), 7.135 (11.00).

### Beispiel 15

### (5RS)-7-(Cyclopropylcarbonyl)-2-(4-methylbenzyl)-5-(pyrrolidin-1-ylcarbonyl)-5,6,7,8-tetrahydro[1,2,4]triazolo[4,3-a]pyrazin-3(2H)-on (Enantiomer 1)

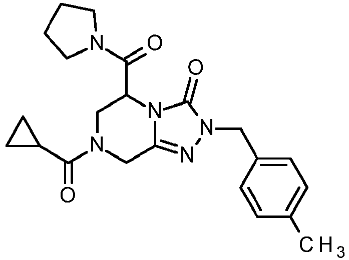

(5RS)-7-(Cyclopropylcarbonyl)-2-(4-methylbenzyl)-5-(pyrrolidin-1-ylcarbonyl)-5,6,7,8-tetrahydro[1,2,4]triazolo[4,3-a]pyrazin-3(2H)-on (Racemat) wurde durch chirale präparative Flüssigchromatographie getrennt [Probenvorbereitung: 117.3 mg gelöst in 4 ml Ethanol; Injektionsvolumen: 1.0 ml; Säule: Daicel Chirapak^{®} IA 5µm, 250 x 20 mm; Laufmittel: Ethanol: isokratisch 100% Ethanol, Fluß: 15.0 ml/min; Temperatur 55°C; UV Detektion: 210 nm]. Nach der Trennung wurden 45.1 mg von Enantiomer 1, welches zuerst eluierte, und 46.0 mg von Enantiomer 2, welches später eluierte, isoliert.

### Enantiomer 1:

Analytische chirale HPLC: Rₜ = 6.46 min, e.e. = 100% [Säule: Daicel Chiralpak^{®} IA 3µm 250 x 4.6 mm; Laufmittel: Ethanol; Fluß: 1 ml/min; UV Detektion: 220 nm]

LC-MS (Methode 2): Rₜ = 1.34 min; MS (ESIpos): m/z = 410 [M+H]⁺

¹H-NMR (400 MHz, DMSO-d6) δ [ppm]: 0.606 (0.70), 0.691 (1.39), 0.745 (0.87), 1.769 (1.04), 1.786 (1.12), 1.802 (0.93), 1.924 (1.90), 1.939 (1.99), 2.274 (16.00), 2.523 (0.43), 3.261 (0.68), 3.641 (1.23), 3.656 (1.85), 3.928 (0.43), 3.958 (0.46), 4.045 (0.59), 4.087 (0.64), 4.617 (0.95), 4.655 (0.89), 4.796 (4.60), 4.834 (0.60), 4.914 (0.91), 5.064 (0.68), 5.106 (0.84), 7.135 (11.22).

### Beispiel 16

### (5RS)-7-(Cyclopropylcarbonyl)-2-(4-methylbenzyl)-5-(pyrrolidin-1-ylcarbonyl)-5,6,7,8-tetrahydro[1,2,4]triazolo[4,3-a]pyrazin-3(2H)-on (Enantiomer 2)

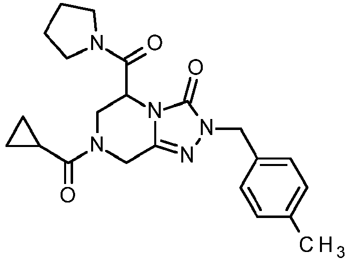

(5RS)-7-(Cyclopropylcarbonyl)-2-(4-methylbenzyl)-5-(pyrrolidin-1-ylcarbonyl)-5,6,7,8-tetrahydro[1,2,4]triazolo[4,3-a]pyrazin-3(2H)-on (Racemate) wurde durch chirale präparative Flüssigchromatographie getrennt [Probenvorbereitung: 117.3 mg gelöst in 4 ml Ethanol; Injektionsvolumen: 1.0 ml; Säule: Daicel Chirapak^{®} IA 5µm, 250 x 20 mm; Laufmittel: Ethanol: isokratisch 100% Ethanol, Fluß: 15.0 ml/min; Temperatur 55°C; UV Detektion: 210 nm]. Nach der Trennung wurden 45.1 mg von Enantiomer 1, welches zuerst eluierte, und 46.0 mg von Enantiomer 2, welches später eluierte, isoliert.

### Enantiomer 2:

Analytische chirale HPLC: Rₜ = 28.6 min, e.e. = 99% [Säule: Daicel Chiralpak^{®} IA 3µm 250 x 4.6 mm; Laufmittel: Ethanol; Fluß: 1 ml/min; UV Detektion: 220 nm]

LC-MS (Methode 2): Rₜ = 1.34 min; MS (ESIpos): m/z = 410 [M+H]⁺

¹H-NMR (400 MHz, DMSO-d6) δ [ppm]: 0.606 (0.70), 0.691 (1.36), 0.745 (0.84), 1.768 (1.03), 1.786 (1.10), 1.801 (0.91), 1.923 (1.86), 1.938 (1.95), 1.953 (1.23), 2.274 (16.00), 3.260 (0.70), 3.640 (1.21), 3.655 (1.81), 3.927 (0.41), 3.959 (0.44), 4.044 (0.57), 4.086 (0.62), 4.617 (0.93), 4.655 (0.86), 4.795 (4.51), 4.834 (0.57), 4.913 (0.88), 5.064 (0.67), 5.106 (0.82), 7.135 (11.10).

### Beispiel 17

### (5RS)-2-(4-Methylbenzyl)-3-oxo-5-(pyrrolidin-1-ylcarbonyl)-N-(2,2,2-trifluorethyl)-2,5,6,8-tetrahydro[1,2,4]triazolo[4,3-a]pyrazin-7(3H)-carboxamid (Racemat)

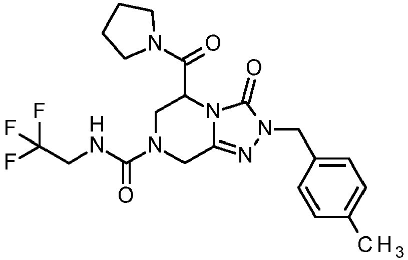

(5RS)-2-(4-Methylbenzyl)-5-(pyrrolidin-1-ylcarbonyl)-5,6,7,8-tetrahydro[1,2,4]triazolo[4,3-a]pyrazin-3(2H)-on (Racemate) (25.0 mg, 85 % Reinheit, 62.2 µmol) wurde in Dichlormethan (500 µl) gelöst und anschließend wurde 1,1,1-Trifluor-2-isocyanatoethan (9.34 mg, 74.7 µmol) zugegeben. Das Reaktionsgemisch wurde nach Rühren für zwei Stunden bei Raumtemperatur eingeengt. Der Rückstand wurde in Acetonitril/ Wasser aufgenommen und über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 21.2 mg (73 % d. Th.) der Titelverbindung erhalten.

LC-MS (Methode 2): Rₜ = 1.40 min; MS (ESIpos): m/z = 467 [M+H]⁺

¹H-NMR (400 MHz, DMSO-d6) δ [ppm]: -0.008 (0.44), 0.008 (0.43), 1.750 (0.63), 1.757 (0.43), 1.769 (1.07), 1.788 (1.24), 1.807 (0.90), 1.823 (0.43), 1.918 (0.83), 1.935 (1.17), 1.945 (1.06), 1.952 (0.87), 1.960 (0.74), 2.274 (11.22), 3.174 (0.44), 3.186 (0.66), 3.200 (0.73), 3.219 (0.48), 3.271 (0.55), 3.289 (1.23), 3.335 (0.60), 3.541 (0.65), 3.547 (0.58), 3.565 (0.98), 3.582 (0.44), 3.614 (0.58), 3.621 (0.86), 3.631 (1.46), 3.646 (0.72), 3.657 (1.18), 3.669 (1.07), 3.686 (0.44), 3.711 (0.64), 3.725 (0.43), 3.734 (0.45), 3.866 (0.46), 3.875 (0.40), 3.890 (0.57), 4.243 (1.68), 4.285 (2.32), 4.326 (0.67), 4.333 (0.67), 4.764 (1.65), 4.790 (7.26), 4.806 (1.60), 7.133 (16.00), 7.384 (0.65), 7.399 (1.33), 7.414 (0.65).

### Beispiel 18

### (5RS)-7-Methyl-2-(4-methylbenzyl)-5-(pyrrolidin-1-ylcarbonyl)-5,6,7,8-tetrahydro[1,2,4]triazolo[4,3-a]pyrazin-3(2H)-on (Racemat)

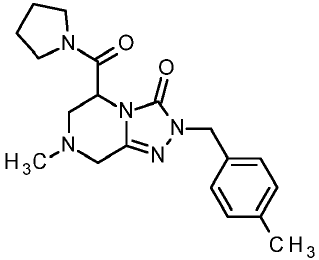

(5RS)-2-(4-Methylbenzyl)-5-(pyrrolidin-1-ylcarbonyl)-5,6,7,8-tetrahydro[1,2,4]triazolo[4,3-a]pyrazin-3(2H)-on (Racemate) (40.0 mg, 117 µmol) wurde in THF (1.0 ml) vorgelegt und mit N,N-Diisopropylethylamin (41 µl, 230 µmol) versetzt. Nach Zugabe von lodmethan (11 µl, 180 µmol) wurde über Nacht bei Raumtemperatur gerührt. Nach Zugabe von lodmethan (5.5 µl, 90 µmol) und N,N-Diisopropylethylamin (21 µl, 115 µmol) wurden 6 h bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 26.2 mg (63 % d. Th.) der Titelverbindung erhalten.

LC-MS (Methode 2): Rₜ = 1.17 min; MS (ESIpos): m/z = 356 [M+H]⁺

¹H-NMR (400 MHz, DMSO-d6) δ [ppm]: 1.766 (1.05), 1.777 (1.06), 1.785 (1.87), 1.792 (1.51), 1.803 (1.62), 1.823 (0.73), 1.877 (0.46), 1.893 (1.21), 1.909 (1.61), 1.918 (1.50), 1.927 (1.17), 1.934 (1.03), 2.273 (16.00), 2.301 (14.63), 2.816 (0.82), 2.829 (0.85), 2.848 (1.32), 2.861 (1.32), 2.934 (1.25), 2.947 (1.39), 2.965 (0.85), 2.978 (0.79), 3.228 (0.47), 3.253 (2.35), 3.273 (1.38), 3.290 (3.69), 3.312 (2.35), 3.328 (1.25), 3.347 (1.70), 3.358 (0.69), 3.365 (0.90), 3.376 (0.97), 3.394 (0.46), 3.430 (0.51), 3.447 (1.11), 3.454 (0.87), 3.470 (3.86), 3.489 (0.64), 3.508 (2.04), 3.619 (0.60), 3.636 (1.07), 3.644 (0.74), 3.652 (0.77), 3.659 (0.89), 3.676 (0.45), 4.717 (1.23), 4.730 (2.71), 4.743 (1.29), 4.764 (8.79), 7.104 (0.89), 7.126 (14.85), 7.149 (0.89).

### Beispiel 19

### (5RS)-7-Methyl-2-(4-methylbenzyl)-5-(pyrrolidin-1-ylcarbonyl)-5,6,7,8-tetrahydro[1,2,4]triazolo[4,3-a]pyrazin-3(2H)-on (Enantiomer 1)

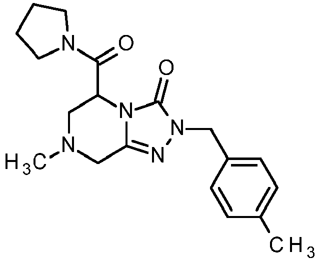

(5RS)-7-Methyl-2-(4-methylbenzyl)-5-(pyrrolidin-1-ylcarbonyl)-5,6,7,8-tetrahydro[1,2,4]triazolo[4,3-a]pyrazin-3(2H)-on (Racemat) wurde durch chirale präparative Flüssigchromatographie getrennt [Probenvorbereitung: 84 mg gelöst in 4 ml warmen Ethanol; Injektionsvolumen: 0.25 ml; Säule: Daicel Chirapak^{®} IB 5µm, 250 x 20 mm; Laufmittel: n-Heptan/Ethanol: isokratisch 50% Ethanol, Fluß: 15.0 ml/min; Temperatur 25°C; UV Detektion: 210 nm]. Nach der Trennung wurden 30.3 mg von Enantiomer 1, welches zuerst eluierte, und 29.8 mg von Enantiomer 2, welches später eluierte, isoliert.

### Enantiomer 1:

Analytische chirale HPLC: Rₜ = 3.15 min, e.e. = 100% [Säule: Daicel Chiralpak^{®} IB-3 3µm 250 x 4.6 mm; Laufmittel: n-Heptan/Ethanol 1:1; Fluß: 1 ml/min; UV Detektion: 220 nm]

LC-MS (Methode 2): Rₜ = 1.19 min; MS (ESIpos): m/z = 356 [M+H]⁺

¹H-NMR (500 MHz, DMSO-d6) δ [ppm]: 1.236 (0.67), 1.324 (0.62), 1.770 (1.48), 1.784 (2.55), 1.796 (2.36), 1.806 (1.77), 1.895 (1.70), 1.908 (2.54), 1.920 (2.28), 1.931 (1.47), 2.273 (16.00), 2.301 (14.24), 2.821 (1.06), 2.831 (1.13), 2.846 (1.55), 2.856 (1.47), 2.938 (1.52), 2.948 (1.58), 2.963 (1.09), 2.973 (1.00), 3.235 (0.75), 3.258 (3.39), 3.271 (1.83), 3.287 (3.63), 3.334 (1.53), 3.349 (2.02), 3.365 (3.12), 3.387 (0.66), 3.436 (0.68), 3.450 (1.48), 3.472 (3.80), 3.503 (2.20), 3.625 (0.79), 3.638 (1.40), 3.656 (1.19), 3.669 (0.58), 4.720 (1.56), 4.730 (2.92), 4.740 (1.65), 4.764 (9.06), 7.109 (1.81), 7.127 (13.42), 7.145 (1.65).

### Beispiel 20

### (5RS)-7-Methyl-2-(4-methylbenzyl)-5-(pyrrolidin-1-ylcarbonyl)-5,6,7,8-tetrahydro[1,2,4]triazolo[4,3-a]pyrazin-3(2H)-on (Enantiomer 2)

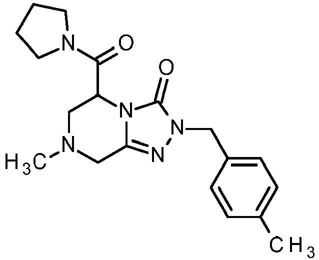

(5RS)-7-Methyl-2-(4-methylbenzyl)-5-(pyrrolidin-1-ylcarbonyl)-5,6,7,8-tetrahydro[1,2,4]triazolo[4,3-a]pyrazin-3(2H)-on (Racemat) wurde durch chirale präparative Flüssigchromatographie getrennt [Probenvorbereitung: 84 mg gelöst in 4 ml warmen Ethanol; Injektionsvolumen: 0.25 ml; Säule: Daicel Chirapak^{®} IB 5µm, 250 x 20 mm; Laufmittel: n-Heptan/Ethanol: isokratisch 50% Ethanol, Fluß: 15.0 ml/min; Temperatur 25°C; UV Detektion: 210 nm]. Nach der Trennung wurden 30.3 mg von Enantiomer 1, welches zuerst eluierte, und 29.8 mg von Enantiomer 2, welches später eluierte, isoliert.

### Enantiomer 2:

Analytische chirale HPLC: Rₜ = 4.94 min, e.e. = 100% [Säule: Daicel Chiralpak^{®} IB-3 3µm 250 x 4.6 mm; Laufmittel: n-Heptan/Ethanol 1:1; Fluß: 1 ml/min; UV Detektion: 220 nm]

LC-MS (Methode 2): Rₜ = 1.20 min; MS (ESIpos): m/z = 356 [M+H]⁺

¹H-NMR (400 MHz, DMSO-d6) δ [ppm]: -0.008 (1.38), 0.008 (1.39), 1.139 (1.67), 1.157 (3.43), 1.175 (1.76), 1.767 (1.05), 1.786 (1.86), 1.793 (1.51), 1.804 (1.65), 1.824 (0.77), 1.894 (1.16), 1.910 (1.53), 1.919 (1.45), 1.927 (1.16), 1.935 (1.05), 2.273 (16.00), 2.302 (14.41), 2.328 (0.52), 2.523 (1.38), 2.670 (0.43), 2.817 (0.81), 2.830 (0.83), 2.848 (1.26), 2.861 (1.28), 2.897 (0.48), 2.915 (1.92), 2.934 (2.38), 2.948 (1.51), 2.966 (0.85), 2.980 (0.83), 3.228 (0.41), 3.253 (2.23), 3.273 (1.32), 3.291 (3.68), 3.347 (1.96), 3.359 (0.85), 3.365 (1.05), 3.376 (1.07), 3.395 (0.54), 3.430 (0.54), 3.448 (1.14), 3.454 (0.91), 3.471 (3.74), 3.490 (0.68), 3.508 (2.05), 3.620 (0.62), 3.636 (1.07), 3.652 (0.77), 3.659 (0.87), 3.677 (0.46), 4.718 (1.20), 4.731 (2.63), 4.744 (1.26), 4.764 (8.52), 5.753 (0.50), 7.104 (0.85), 7.126 (13.02), 7.150 (1.30), 7.154 (1.38).

### Beispiel 21

### (5RS)-N-Cyclopropyl-2-(4-methylbenzyl)-3-oxo-5-(pyrrolidin-1-ylcarbonyl)-2,5,6,8-tetrahydro[1,2,4]triazolo[4,3-a]pyrazin-7(3H)-carboxamid (Racemat)

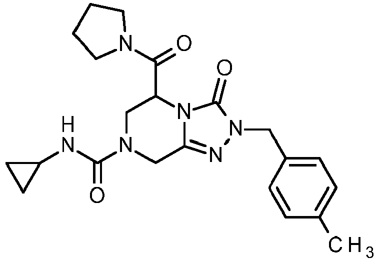

(5RS)-2-(4-Methylbenzyl)-5-(pyrrolidin-1-ylcarbonyl)-5,6,7,8-tetrahydro[1,2,4]triazolo[4,3-a]pyrazin-3(2H)-on (Racemate) (50.0 mg, 85 % Reinheit, 124 µmol) wurde in Dichlormethan (1.0 ml) gelöst und anschließend wurde Isocyanatocyclopropan (10 µl, 150 µmol) zugegeben. Das Reaktionsgemisch wurde nach Rühren für zwei Stunden bei Raumtemperatur eingeengt. Der Rückstand wurde in Acetonitril/ Wasser aufgenommen und über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 37.2 mg (70 % d. Th.) der Titelverbindung erhalten.

LC-MS (Methode 2): Rₜ = 1.24 min; MS (ESIpos): m/z = 425 [M+H]⁺

¹H-NMR (400 MHz, DMSO-d6) δ [ppm]: -0.008 (0.62), 0.008 (0.52), 0.313 (2.64), 0.321 (2.49), 0.339 (0.48), 0.499 (0.60), 0.512 (1.45), 0.518 (1.75), 0.529 (1.62), 0.535 (1.54), 0.551 (0.45), 1.761 (0.75), 1.766 (0.56), 1.779 (1.34), 1.798 (1.59), 1.816 (1.08), 1.832 (0.48), 1.919 (1.02), 1.927 (1.00), 1.935 (1.36), 1.942 (1.29), 1.952 (0.97), 1.958 (0.90), 2.272 (12.76), 2.418 (0.42), 2.425 (0.70), 2.434 (0.90), 2.442 (0.88), 2.451 (0.66), 2.458 (0.41), 3.160 (0.55), 3.175 (0.75), 3.189 (0.84), 3.208 (0.53), 3.284 (0.65), 3.331 (0.99), 3.349 (0.43), 3.520 (0.78), 3.526 (0.69), 3.545 (1.20), 3.556 (1.06), 3.567 (1.13), 3.593 (1.47), 3.604 (1.20), 3.610 (1.15), 3.627 (0.66), 3.635 (0.65), 4.124 (2.07), 4.166 (2.35), 4.209 (0.88), 4.215 (0.91), 4.245 (0.80), 4.252 (0.80), 4.705 (2.04), 4.720 (1.83), 4.729 (1.08), 4.747 (1.66), 4.776 (6.62), 6.830 (1.87), 6.836 (1.86), 7.107 (0.52), 7.129 (16.00), 7.152 (0.52)

### Beispiel 22

### (5RS)-N-Cyclopropyl-2-(4-methylbenzyl)-3-oxo-5-(pyrrolidin-1-ylcarbonyl)-2,5,6,8-tetrahydro[1,2,4]triazolo[4,3-a]pyrazin-7(3H)-carboxamid (Enantiomer 1)

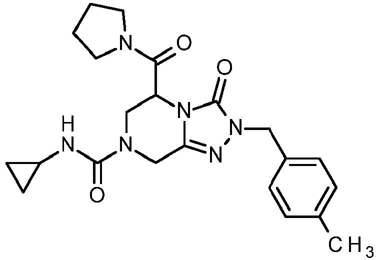

(5RS)-N-Cyclopropyl-2-(4-methylbenzyl)-3-oxo-5-(pyrrolidin-1-ylcarbonyl)-2,5,6,8-tetrahydro[1,2,4]triazolo[4,3-a]pyrazin-7(3H)-carboxamid (Racemat) wurde durch chirale präparative Flüssigchromatographie getrennt [Probenvorbereitung: 113.4 mg gelöst in 8 ml warmen Ethanol; Injektionsvolumen: 0.5 ml; Säule: Daicel Chirapak^{®} AD-H 5µm, 250 x 20 mm; Laufmittel: n-Heptan/Ethanol: isokratisch 50% Ethanol, Fluß: 15.0 ml/min; Temperatur 25°C; UV Detektion: 210 nm]. Nach der Trennung wurden 15.3 mg von Enantiomer 1, welches zuerst eluierte, und 45.2 mg von Enantiomer 2, welches später eluierte, isoliert.

### Enantiomer 1:

Analytische chirale HPLC: Rₜ = 1.91 min, e.e. = 100% [Säule: Daicel Chiralpak^{®} AD-3 3µm 50 x 4.6 mm; Laufmittel: n-Heptan/Ethanol 1:1; Fluß: 1 ml/min; UV Detektion: 220 nm]

LC-MS (Methode 2): Rₜ = 1.24 min; MS (ESIpos): m/z = 425 [M+H]⁺

¹H-NMR (400 MHz, DMSO-d6) δ [ppm]: -0.008 (0.78), 0.007 (0.76), 0.313 (2.71), 0.321 (2.58), 0.339 (0.51), 0.499 (0.60), 0.512 (1.48), 0.518 (1.81), 0.530 (1.69), 0.536 (1.62), 0.552 (0.49), 1.761 (0.76), 1.766 (0.58), 1.780 (1.36), 1.798 (1.62), 1.816 (1.11), 1.832 (0.51), 1.919 (1.05), 1.927 (1.06), 1.935 (1.42), 1.943 (1.36), 1.953 (1.04), 1.959 (0.96), 2.272 (12.80), 2.425 (0.70), 2.434 (0.90), 2.442 (0.89), 2.451 (0.67), 2.458 (0.41), 3.160 (0.55), 3.175 (0.76), 3.189 (0.83), 3.208 (0.53), 3.284 (0.62), 3.331 (1.16), 3.349 (0.49), 3.520 (0.80), 3.526 (0.72), 3.544 (1.24), 3.556 (1.11), 3.567 (1.17), 3.593 (1.48), 3.604 (1.24), 3.610 (1.19), 3.627 (0.71), 3.635 (0.71), 4.124 (2.04), 4.166 (2.33), 4.209 (0.91), 4.215 (0.94), 4.245 (0.83), 4.252 (0.83), 4.706 (2.07), 4.720 (1.88), 4.729 (1.13), 4.747 (1.70), 4.776 (6.69), 6.829 (1.89), 6.834 (1.90), 7.107 (0.51), 7.129 (16.00), 7.152 (0.61).

### Beispiel 23

### (5RS)-N-Cyclopropyl-2-(4-methylbenzyl)-3-oxo-5-(pyrrolidin-1-ylcarbonyl)-2,5,6,8-tetrahydro[1,2,4]triazolo[4,3-a]pyrazin-7(3H)-carboxamid (Enantiomer 2)

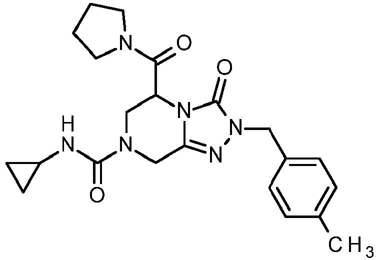

(5RS)-N-Cyclopropyl-2-(4-methylbenzyl)-3-oxo-5-(pyrrolidin-1-ylcarbonyl)-2,5,6,8-tetrahydro[1,2,4]triazolo[4,3-a]pyrazin-7(3H)-carboxamid (Racemat) wurde durch chirale präparative Flüssigchromatographie getrennt [Probenvorbereitung: 113.4 mg gelöst in 8 ml warmen Ethanol; Injektionsvolumen: 0.5 ml; Säule: Daicel Chirapak^{®} AD-H 5µm, 250 x 20 mm; Laufmittel: n-Heptan/Ethanol: isokratisch 50% Ethanol, Fluß: 15.0 ml/min; Temperatur 25°C; UV Detektion: 210 nm]. Nach der Trennung wurden 15.3 mg von Enantiomer 1, welches zuerst eluierte, und 45.2 mg von Enantiomer 2, welches später eluierte, isoliert.

### Enantiomer 2:

Analytische chirale HPLC: Rₜ = 2.93 min, e.e. = 100% [Säule: Daicel Chiralpak^{®} AD-3 3µm 50 x 4.6 mm; Laufmittel: n-Heptan/Ethanol 1:1; Fluß: 1 ml/min; UV Detektion: 220 nm]

LC-MS (Methode 2): Rₜ = 1.24 min; MS (ESIpos): m/z = 425 [M+H]⁺

¹H-NMR (400 MHz, DMSO-d6) δ [ppm]: 0.313 (2.59), 0.321 (2.45), 0.339 (0.45), 0.499 (0.56), 0.518 (1.69), 0.529 (1.57), 0.535 (1.52), 0.551 (0.46), 1.761 (0.69), 1.779 (1.28), 1.798 (1.52), 1.816 (1.05), 1.832 (0.46), 1.919 (0.99), 1.936 (1.35), 1.953 (0.97), 2.272 (12.59), 2.425 (0.71), 2.434 (0.91), 2.441 (0.89), 2.450 (0.68), 2.458 (0.43), 3.161 (0.52), 3.174 (0.73), 3.189 (0.82), 3.207 (0.50), 3.284 (0.59), 3.331 (1.02), 3.349 (0.43), 3.520 (0.76), 3.526 (0.69), 3.544 (1.18), 3.556 (1.02), 3.567 (1.07), 3.593 (1.40), 3.604 (1.17), 3.610 (1.11), 3.627 (0.66), 3.635 (0.65), 4.124 (1.90), 4.166 (2.17), 4.216 (0.88), 4.245 (0.78), 4.705 (1.97), 4.720 (1.81), 4.729 (1.05), 4.747 (1.59), 4.776 (6.34), 6.828 (1.82), 6.833 (1.80), 7.107 (0.53), 7.129 (16.00), 7.151 (0.57).

### Beispiel 24

### (5RS)-2-(4-Methylbenzyl)-3-oxo-5-(pyrrolidin-1-ylcarbonyl)-N-[4-(trifluormethyl)phenyl]-2,5,6,8-tetrahydro[1,2,4]triazolo[4,3-a]pyrazin-7(3H)-carboxamid (Racemat)

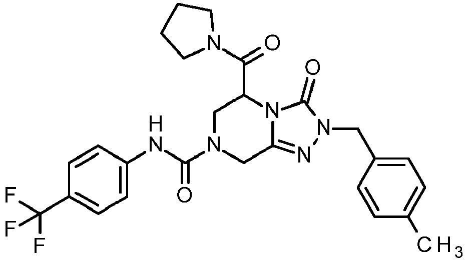

(5RS)-2-(4-Methylbenzyl)-5-(pyrrolidin-1-ylcarbonyl)-5,6,7,8-tetrahydro[1,2,4]triazolo[4,3-a]pyrazin-3(2H)-on (Racemat) (50.0 mg, 85 % Reinheit, 124 µmol) wurde in Dichlormethan (1.0 ml) gelöst und mit 1-Isocyanato-4-(trifluormethyl)benzol (21 µl, 150 µmol) versetzt. Das Reaktionsgemisch wurde eine Stunde bei Raumtemperatur gerührt. Der entstehende Niederschlag wurde abfiltriert und mit Petrolether gewaschen und im Vakuum getrocknet. Es wurden 46.3 mg (70 % d. Th.) der Titelverbindung erhalten.

LC-MS (Methode 2): Rₜ = 1.81 min; MS (ESIpos): m/z = 529 [M+H]⁺

¹H-NMR (400 MHz, DMSO-d6) δ [ppm]: 0.839 (0.51), 0.855 (0.58), 1.236 (0.57), 1.614 (0.43), 1.632 (0.55), 1.727 (0.52), 1.743 (0.56), 1.758 (0.44), 1.902 (0.70), 1.919 (1.13), 1.935 (0.99), 1.949 (0.63), 2.276 (9.71), 2.957 (0.51), 2.970 (0.45), 3.203 (0.71), 3.214 (0.41), 3.221 (0.44), 3.232 (0.61), 3.559 (0.56), 3.583 (0.81), 3.645 (0.66), 3.662 (0.48), 3.701 (0.57), 3.711 (0.64), 3.738 (0.68), 3.748 (0.59), 4.311 (1.25), 4.354 (1.41), 4.545 (0.71), 4.581 (0.66), 4.804 (4.73), 4.857 (1.18), 5.003 (1.19), 5.045 (1.07), 7.144 (16.00), 7.595 (13.12), 9.111 (2.21).

### Beispiel 25

### (5RS)-2-{[3-Chlor-4-(trifluormethyl)pyridin-2-yl]methyl}-5-{[(3S)-3-fluorpyrrolidin-1-yl]carbonyl}-7-(2,2,2-trifluorethyl)-5,6,7,8-tetrahydro[1,2,4]triazolo[4,3-a]pyrazin-3(2H)-on (Diastereomerengemisch; 2 Isomere)

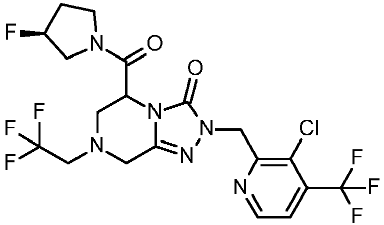

(5RS)-2-{[3-Chlor-4-(trifluormethyl)pyridin-2-yl]methyl}-5-{[(3S)-3-fluorpyrrolidin-1-yl]carbonyl}-5,6,7,8-tetrahydro[1,2,4]triazolo[4,3-a]pyrazin-3(2H)-on (Diastereomerengemisch; 2 Isomere) (20.0 mg, 44.6 µmol) wurde in DMF (1.0 ml) und N,N-Diisopropylethylamin (23 µl, 130 µmol) gelöst mit 2,2,2-Trifluorethyltrifluormethansulfonat (12.4 mg, 53.5 µmol) versetzt. Das Reaktionsgemisch wurde für 6 Stunden bei Raumtemperatur gerührt. Es wurde erneut 2,2,2-Trifluorethyltrifluormethansulfonat (12.4 mg, 53.5 µmol) zugegeben und über Nacht nachgerührt. Es wurden 2,2,2-Trifluorethyltrifluormethansulfonat (24.8 mg, 107.0 µmol) zugegeben und für 24 Stunden nachgerührt. Anschließend wurden alle flüchtigen Bestandteile im Vakuum entfernt und der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 10.5 mg (44 % d. Th.) der Titelverbindung erhalten.

LC-MS (Methode 2): Rₜ = 1.60 min; MS (ESIpos): m/z = 531 [M+H]⁺

¹H-NMR (400 MHz, DMSO-d6) δ [ppm]: 1.236 (1.26), 2.102 (1.14), 2.235 (0.98), 3.087 (0.46), 3.121 (0.58), 3.145 (0.81), 3.158 (0.97), 3.194 (1.47), 3.232 (0.99), 3.244 (0.98), 3.345 (2.85), 3.370 (2.02), 3.383 (3.78), 3.408 (3.47), 3.444 (1.53), 3.490 (3.16), 3.514 (3.43), 3.540 (2.16), 3.555 (2.21), 3.663 (1.68), 3.687 (1.94), 3.704 (2.38), 3.726 (4.16), 3.751 (2.90), 3.761 (3.26), 3.823 (6.62), 3.862 (2.86), 3.895 (1.41), 3.951 (0.68), 3.974 (1.16), 3.999 (1.08), 4.032 (0.48), 4.057 (0.50), 4.091 (0.46), 4.738 (0.62), 4.752 (1.18), 4.767 (0.67), 4.796 (0.81), 4.809 (1.53), 4.823 (0.76), 4.879 (1.49), 4.893 (1.41), 4.906 (1.75), 5.190 (16.00), 5.265 (1.53), 5.396 (1.61), 5.493 (0.73), 5.753 (1.54), 7.851 (7.25), 7.863 (7.53), 8.737 (6.28), 8.749 (6.10). (Mischung von Diastereomeren)

### Beispiel 26

### (5RS)-N-(3,4-Dichlorphenyl)-2-(4-methylbenzyl)-3-oxo-5-(pyrrolidin-1-ylcarbonyl)-2,5,6,8-tetrahydro[1,2,4]triazolo[4,3-a]pyrazin-7(3H)-carboxamid (Racemat)

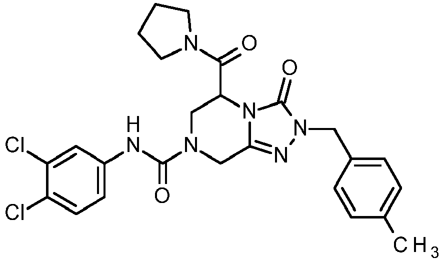

(5RS)-2-(4-Methylbenzyl)-5-(pyrrolidin-1-ylcarbonyl)-5,6,7,8-tetrahydro[1,2,4]triazolo[4,3-a]pyrazin-3(2H)-on (Racemat) (50.0 mg, 85 % Reinheit, 124 µmol) wurde in Dichlormethan (1.0 ml) gelöst und mit 1,2-Dichlor-4-isocyanatobenzol (28.1 mg, 149 µmol) versetzt. Das Reaktionsgemisch wurde für 1 Stunde bei Raumtemperatur gerührt. Anschließend wurden alle flüchtigen Bestandteile im Vakuum entfernt und der Rückstand wurde in Acetonitril/ Wasser aufgenommen und über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und über Säulenchromatographie (Kieselgel, Laufmittel: Dichlormethan/Methanol-Gradient) gereinigt. So wurden 42.0 mg (64 % d. Th.) der Titelverbindung erhalten.

LC-MS (Methode 2): Rₜ = 1.87 min; MS (ESIpos): m/z = 529 [M+H]⁺

¹H-NMR (400 MHz, DMSO-d6) δ [ppm]: 1.637 (0.41), 1.655 (0.55), 1.746 (0.49), 1.761 (0.54), 1.909 (0.69), 1.926 (1.11), 1.942 (0.99), 1.956 (0.61), 2.275 (9.25), 2.967 (0.48), 2.980 (0.43), 3.215 (0.70), 3.233 (0.41), 3.245 (0.59), 3.556 (0.53), 3.563 (0.47), 3.581 (0.79), 3.642 (0.63), 3.658 (0.47), 3.665 (0.42), 3.692 (0.56), 3.703 (0.62), 3.729 (0.64), 3.740 (0.58), 4.292 (1.25), 4.333 (1.39), 4.506 (0.70), 4.542 (0.63), 4.801 (4.59), 4.848 (1.13), 4.965 (1.15), 5.007 (1.03), 7.142 (16.00), 7.333 (1.00), 7.339 (1.01), 7.355 (1.27), 7.361 (1.34), 7.475 (2.43), 7.497 (1.78), 7.731 (2.15), 7.738 (2.10), 9.023 (1.85).

### Beispiel 27

### (5RS)-N-(2,2-Difluorethyl)-2-(4-methylbenzyl)-3-oxo-5-(pyrrolidin-1-ylcarbonyl)-2,5,6,8-tetrahydro[1,2,4]triazolo[4,3-a]pyrazin-7(3H)-carboxamid (Racemat)

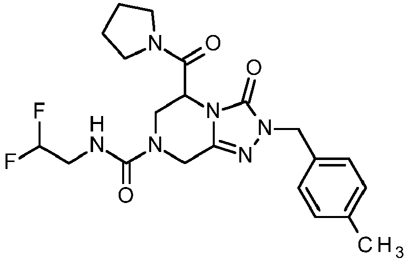

(5RS)-2-(4-Methylbenzyl)-5-(pyrrolidin-1-ylcarbonyl)-5,6,7,8-tetrahydro[1,2,4]triazolo[4,3-a]pyrazin-3(2H)-on (Racemat) (25.0 mg, 85 % Reinheit, 62.2 µmol) wurde in Dichlormethan (500 µl) gelöst und mit 1,1-Difluor-2-isocyanatoethan (11.4 mg, 70 % Reinheit, 74.7 µmol) versetzt. Das Reaktionsgemisch wurde für 2 Stunden bei Raumtemperatur gerührt. Anschließend wurden alle flüchtigen Bestandteile im Vakuum entfernt und der Rückstand wurde in Acetonitril/ Wasser aufgenommen und über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 21.0 mg (75 % d. Th.) der Titelverbindung erhalten.

LC-MS (Methode 2): Rₜ = 1.30 min; MS (ESIpos): m/z = 449 [M+H]⁺

¹H-NMR (400 MHz, DMSO-d6) δ [ppm]: -0.008 (0.69), 0.008 (0.60), 1.762 (0.70), 1.780 (1.21), 1.791 (0.99), 1.798 (0.96), 1.807 (0.80), 1.919 (0.78), 1.936 (1.07), 1.946 (0.98), 1.953 (0.78), 1.961 (0.68), 2.273 (10.94), 3.179 (0.47), 3.193 (0.60), 3.208 (0.75), 3.226 (0.46), 3.280 (0.66), 3.298 (1.95), 3.313 (14.34), 3.345 (0.80), 3.383 (0.42), 3.394 (0.50), 3.538 (0.61), 3.545 (0.54), 3.563 (0.91), 3.580 (0.41), 3.608 (0.51), 3.627 (1.24), 3.640 (0.60), 3.653 (0.99), 3.664 (0.92), 4.230 (1.62), 4.247 (0.69), 4.254 (0.72), 4.272 (1.93), 4.283 (0.66), 4.290 (0.62), 4.725 (1.46), 4.768 (2.74), 4.785 (5.27), 5.750 (0.48), 5.881 (0.45), 5.891 (0.95), 5.901 (0.46), 6.032 (0.45), 7.132 (16.00), 7.188 (0.60), 7.202 (1.19), 7.216 (0.58).

### Beispiel 28

### (5RS)-N-tert-Butyl-2-(4-methylbenzyl)-3-oxo-5-(pyrrolidin-1-ylcarbonyl)-2,5,6,8-tetrahydro[1,2,4]triazolo[4,3-a]pyrazin-7(3H)-carboxamid (Racemat)

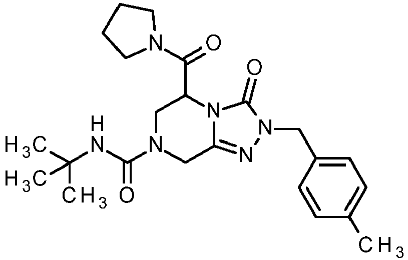

(5RS)-2-(4-Methylbenzyl)-5-(pyrrolidin-1-ylcarbonyl)-5,6,7,8-tetrahydro[1,2,4]triazolo[4,3-a]pyrazin-3(2H)-on (Racemat) (25.0 mg, 85 % Reinheit, 62.2 µmol) wurde in Dichlormethan (500 µl) gelöst und mit 2-Isocyanato-2-methylpropan (7.40 mg, 74.7 µmol) versetzt. Das Reaktionsgemisch wurde für 2 Stunde bei Raumtemperatur gerührt. Es wurde 2-Isocyanato-2-methylpropan (7.40 mg, 74.7 µmol) zugegeben und über Nacht bei Raumtemperatur gerührt. Anschließend wurden alle flüchtigen Bestandteile im Vakuum entfernt und der Rückstand wurde in Acetonitril/ Wasser aufgenommen und über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 18.5 mg (67 % d. Th.) der Titelverbindung erhalten.

LC-MS (Methode 2): Rₜ = 1.53 min; MS (ESIpos): m/z = 441 [M+H]⁺

¹H-NMR (400 MHz, DMSO-d6) δ [ppm]: 1.202 (16.00), 1.775 (0.41), 1.793 (0.47), 1.939 (0.51), 1.949 (0.46), 2.274 (5.17), 3.345 (0.56), 3.549 (0.53), 3.558 (0.66), 3.585 (0.41), 4.111 (0.78), 4.153 (0.87), 4.711 (0.40), 4.720 (0.70), 4.780 (2.69), 4.813 (0.63), 6.035 (1.16), 7.135 (8.57).

### Beispiel 29

### (5RS)-2-{[3-Chlor-4-(trifluormethyl)pyridin-2-yl]methyl}-5-{[(3S)-3-fluorpyrrolidin-1-yl]carbonyl}-7-isobutyl-5,6,7,8-tetrahydro[1,2,4]triazolo[4,3-a]pyrazin-3(2H)-on (Diastereomerengemisch; 2 Isomere)

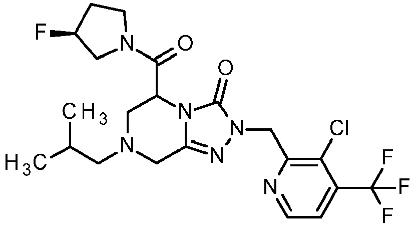

(5RS)-2-{[3-Chlor-4-(trifluormethyl)pyridin-2-yl]methyl}-5-{[(3S)-3-fluorpyrrolidin-1-yl]carbonyl}-5,6,7,8-tetrahydro[1,2,4]triazolo[4,3-a]pyrazin-3(2H)-on (Diastereomerengemisch; 2 Isomere) (70.0 mg, 156 µmol) und 2-Methylpropanal (28 µl, 310 µmol) wurden in THF (1.5 ml) gelöst und mit Natriumtriacetoxyborhydrid (132 mg, 624 µmol) versetzt. Es wurde Essigsäure (67 µl) zugegeben und für 1 Stunde bei Raumtemperatur gerührt. Es wurde erneut 2-Methylpropanal (28 µl, 310 µmol) zugegeben und für eine weitere Stunde bei Raumtemperatur gerührt. Das Reaktionsgemisch wurde über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 64.0 mg (81 % d. Th.) der Titelverbindung erhalten.

LC-MS (Methode 2): Rₜ = 1.79 min; MS (ESIpos): m/z = 505 [M+H]⁺

¹H-NMR (400 MHz, DMSO-d6) δ [ppm]: 0.759 (7.26), 0.775 (16.00), 0.789 (12.19), 0.804 (7.51), 0.812 (9.17), 0.817 (8.97), 0.827 (15.02), 0.840 (10.10), 1.722 (0.65), 1.737 (1.47), 1.754 (2.20), 1.771 (2.35), 1.788 (1.84), 1.805 (1.09), 1.823 (0.44), 1.950 (0.50), 1.976 (0.44), 2.093 (1.23), 2.120 (1.19), 2.148 (1.71), 2.160 (2.14), 2.178 (3.41), 2.191 (4.10), 2.199 (3.16), 2.209 (3.98), 2.219 (4.66), 2.236 (4.90), 2.248 (3.38), 2.266 (2.19), 2.328 (0.44), 2.670 (0.45), 2.884 (1.65), 2.924 (3.94), 2.936 (2.79), 2.988 (0.90), 3.001 (0.98), 3.027 (1.37), 3.037 (1.35), 3.058 (1.53), 3.070 (1.72), 3.088 (0.76), 3.102 (0.66), 3.266 (0.77), 3.332 (2.08), 3.357 (3.13), 3.372 (3.12), 3.383 (1.74), 3.412 (2.83), 3.421 (2.92), 3.463 (1.62), 3.485 (1.21), 3.512 (3.87), 3.530 (2.00), 3.551 (3.90), 3.569 (2.96), 3.589 (1.76), 3.607 (2.45), 3.636 (1.02), 3.661 (1.26), 3.679 (2.60), 3.709 (1.22), 3.742 (1.75), 3.765 (1.72), 3.787 (1.13), 3.821 (0.84), 3.883 (1.72), 3.946 (0.80), 3.969 (1.45), 3.992 (0.81), 4.034 (0.48), 4.062 (0.58), 4.092 (0.44), 4.688 (0.81), 4.700 (1.75), 4.713 (0.84), 4.738 (0.93), 4.752 (1.90), 4.765 (0.86), 4.821 (0.81), 4.833 (1.79), 4.849 (1.35), 4.861 (2.33), 4.872 (1.09), 5.136 (1.02), 5.184 (13.68), 5.226 (1.09), 5.258 (1.71), 5.338 (0.77), 5.391 (1.71), 5.470 (0.74), 5.502 (0.70), 7.848 (9.33), 7.861 (9.62), 8.737 (7.79), 8.750 (7.58). (Mischung von Diastereomeren)

### Beispiel 30

### (5RS)-7-Isobutyl-2-(4-methylbenzyl)-5-(pyrrolidin-1-ylcarbonyl)-5,6,7,8-tetrahydro[1,2,4]triazolo[4,3-a]pyrazin-3(2H)-on (Racemat)

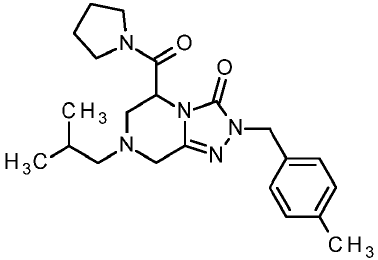

(5RS)-2-(4-Methylbenzyl)-5-(pyrrolidin-1-ylcarbonyl)-5,6,7,8-tetrahydro[1,2,4]triazolo[4,3-a]pyrazin-3(2H)-on (Racemat) (40.0 mg, 117 µmol) und 2-Methylpropanal (21 µl, 230 µmol) wurden in THF (1.0 ml) vorgelegt und mit Natriumtriacetoxyborhydrid (99.3 mg, 469 µmol) und Essigsäure (50 µl) versetzt. Nach 1 h bei Raumtemperatur wurde mit Wasser und Ethylacetat zugegeben und über Nacht bei Raumtemperatur gerührt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnesiumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 38.2 mg (82 % d. Th.) der Titelverbindung erhalten.

LC-MS (Methode 2): Rₜ = 1.76 min; MS (ESIpos): m/z = 398 [M+H]⁺

¹H-NMR (400 MHz, DMSO-d6) δ [ppm]: 0.770 (4.70), 0.787 (4.84), 0.815 (4.66), 0.831 (4.76), 1.732 (0.66), 1.739 (0.42), 1.751 (1.06), 1.769 (0.96), 1.786 (0.79), 1.802 (0.69), 1.818 (0.58), 1.882 (0.67), 1.897 (0.97), 1.911 (0.79), 1.926 (0.52), 2.137 (0.45), 2.157 (0.44), 2.167 (0.97), 2.188 (0.92), 2.206 (0.98), 2.223 (0.94), 2.236 (0.47), 2.253 (0.46), 2.274 (9.70), 2.919 (1.13), 2.930 (1.69), 2.941 (1.08), 3.220 (0.56), 3.232 (0.54), 3.239 (0.47), 3.252 (0.40), 3.311 (1.19), 3.324 (1.67), 3.347 (0.91), 3.362 (1.93), 3.376 (0.64), 3.408 (0.64), 3.414 (0.45), 3.426 (0.40), 3.433 (0.67), 3.523 (1.63), 3.561 (1.15), 3.653 (0.52), 3.662 (0.41), 3.669 (0.40), 3.676 (0.41), 4.714 (0.68), 4.726 (1.60), 4.738 (0.69), 4.765 (4.67), 7.134 (16.00).

### Beispiel 31

### (5RS)-7-Cyclobutyl-2-(4-methylbenzyl)-5-(pyrrolidin-1-ylcarbonyl)-5,6,7,8-tetrahydro[1,2,4]triazolo[4,3-a]pyrazin-3(2H)-on (Racemat)

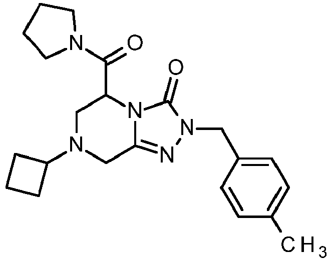

(5RS)-2-(4-Methylbenzyl)-5-(pyrrolidin-1-ylcarbonyl)-5,6,7,8-tetrahydro[1,2,4]triazolo[4,3-a]pyrazin-3(2H)-on (Racemat) (50.0 mg, 146 µmol) und Cyclobutanon (22 µl, 290 µmol) wurden in THF (1.0 ml) vorgelegt und mit Natriumtriacetoxyborhydrid (124 mg, 586 µmol) und Essigsäure (63 µl, 1.1 mmol) versetzt. Die Reaktionsmischung wurde 1 Stunde bei Raumtemperatur gerührt und anschließend mit Wasser verdünnt. Das Reaktionsgemisch wurde über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 37.8 mg (65 % d. Th.) der Titelverbindung erhalten.

LC-MS (Methode 2): Rₜ = 1.61 min; MS (ESIpos): m/z = 396 [M+H]⁺

¹H-NMR (400 MHz, DMSO-d6) δ [ppm]: 1.576 (0.54), 1.582 (0.61), 1.602 (1.20), 1.618 (1.39), 1.626 (1.19), 1.644 (0.95), 1.673 (0.47), 1.696 (0.80), 1.720 (1.05), 1.742 (1.09), 1.761 (1.60), 1.780 (2.15), 1.797 (2.21), 1.813 (1.76), 1.829 (0.67), 1.870 (0.45), 1.887 (1.15), 1.903 (1.65), 1.916 (1.49), 1.930 (1.27), 1.946 (1.04), 1.962 (0.80), 1.972 (0.79), 1.998 (0.82), 2.009 (0.70), 2.017 (0.72), 2.274 (15.01), 2.697 (0.88), 2.712 (0.95), 2.729 (1.13), 2.743 (1.12), 2.913 (1.09), 2.926 (1.25), 2.945 (1.19), 2.970 (1.29), 2.989 (0.85), 3.233 (0.50), 3.250 (0.73), 3.263 (1.20), 3.283 (2.39), 3.296 (1.64), 3.322 (4.29), 3.336 (1.43), 3.355 (1.72), 3.374 (1.17), 3.383 (4.02), 3.405 (1.44), 3.421 (2.04), 3.430 (1.31), 3.447 (0.56), 3.648 (0.56), 3.664 (0.96), 3.673 (0.71), 3.680 (0.73), 3.688 (0.83), 3.705 (0.42), 4.695 (1.12), 4.708 (2.32), 4.722 (1.11), 4.763 (8.25), 7.106 (0.82), 7.128 (16.00), 7.151 (0.76).

### Beispiel 32

### (5RS)-2-(4-Methylbenzyl)-5-(pyrrolidin-1-ylcarbonyl)-7-[4-(trifluormethyl)benzoyl]-5,6,7,8-tetrahydro[1,2,4]triazolo[4,3-a]pyrazin-3(2H)-on (Racemat)

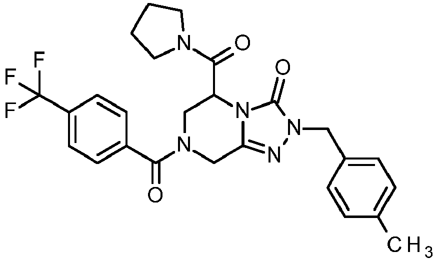

(5RS)-2-(4-Methylbenzyl)-5-(pyrrolidin-1-ylcarbonyl)-5,6,7,8-tetrahydro[1,2,4]triazolo[4,3-a]pyrazin-3(2H)-on (Racemat) (50.0 mg, 85 % Reinheit, 124 µmol) wurde in Dichlormethan (1.0 ml) und N,N-Diisopropylethylamin (43 µl, 250 µmol) gelöst und mit 4-(Trifluormethyl)benzoylchlorid (20 µl, 140 µmol) versetzt. Die Reaktionsmischung wurde 1 Stunde bei Raumtemperatur gerührt. Anschließend wurden alle flüchtigen Bestandteile im Vakuum entfernt und der Rückstand wurde in Acetonitril/ Wasser aufgenommen und über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es 51.2 mg (80 % d. Th.) der Titelverbindung erhalten.

LC-MS (Methode 2): Rₜ = 1.75 min; MS (ESlpos): m/z = 514 [M+H]⁺

¹H-NMR (400 MHz, DMSO-d6) δ [ppm]: -0.008 (1.84), 0.008 (1.61), 1.405 (1.53), 1.731 (1.57), 1.969 (0.42), 2.279 (16.00), 2.523 (0.98), 2.664 (0.87), 2.669 (0.85), 3.180 (1.36), 3.397 (0.94), 3.673 (0.49), 3.906 (2.29), 4.235 (0.87), 4.278 (1.07), 4.769 (2.18), 4.814 (4.18), 4.854 (0.75), 5.246 (0.90), 5.288 (0.84), 7.146 (13.34), 7.522 (2.71), 7.539 (3.38), 7.851 (5.25), 7.871 (4.59).

### Beispiel 33

### (5RS)-5-{[(3S)-3-Fluorpyrrolidin-1-yl]carbonyl}-2-[4-fluor-3-(trifluormethyl)benzyl]-7-(4,4,4-trifluorbutanoyl)-5,6,7,8-tetrahydro[1,2,4]triazolo[4,3-a]pyrazin-3(2H)-on (Isomer 1)

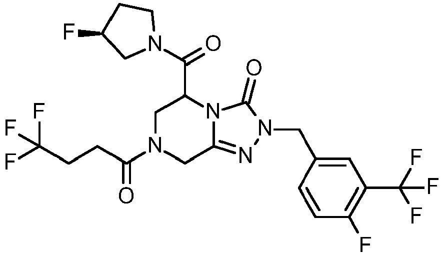

(5RS)-5-{[(3S)-3-Fluorpyrrolidin-1-yl]carbonyl}-2-[4-fluor-3-(trifluormethyl)benzyl]-5,6,7,8-tetrahydro[1,2,4]triazolo[4,3-a]pyrazin-3(2H)-on (Isomer 2) (80.0 mg, 185 µmol) wurde in Dichlormethan (2.0 ml) und N,N-Diisopropylethylamin (65 µl, 370 µmol) gelöst und mit 4,4,4-Trifluorbutanoylchlorid (32.7 mg, 204 µmol) versetzt. Das Reaktionsgemisch wurde über Nacht bei Raumtemperatur gerührt. Es wurde 4,4,4-Trifluorbutanoylchlorid (32.7 mg, 204 µmol) zugegeben und für weitere 24 Stunden bei Raumtemperatur gerührt. Das Reaktionsgemisch wurde über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 35.4 mg (34 % d. Th.) der Titelverbindung erhalten.

LC-MS (Methode 2): Rₜ = 1.71 min; MS (ESlpos): m/z = 556 [M+H]⁺

¹H-NMR (400 MHz, DMSO-d6) δ [ppm]: -0.149 (0.46), 0.146 (0.51), 1.235 (1.46), 1.982 (0.60), 2.161 (2.87), 2.179 (2.71), 2.203 (2.76), 2.218 (2.58), 2.242 (1.84), 2.326 (1.01), 2.366 (0.70), 2.447 (4.19), 2.459 (4.30), 2.613 (2.39), 2.632 (2.90), 2.709 (0.62), 2.739 (0.76), 2.778 (1.87), 2.795 (1.63), 2.819 (1.76), 2.836 (1.01), 2.859 (0.63), 3.368 (2.63), 3.385 (2.46), 3.457 (2.44), 3.488 (4.11), 3.538 (1.90), 3.584 (2.19), 3.745 (1.54), 3.762 (1.70), 3.788 (0.94), 3.900 (2.74), 3.912 (2.00), 3.925 (2.25), 3.939 (3.30), 3.951 (1.95), 3.972 (1.68), 3.997 (3.25), 4.021 (2.28), 4.072 (1.62), 4.102 (0.92), 4.135 (3.60), 4.146 (2.57), 4.177 (3.77), 4.188 (2.85), 4.290 (1.59), 4.323 (2.70), 4.358 (1.79), 4.472 (2.06), 4.515 (2.47), 4.632 (0.82), 4.680 (0.89), 4.716 (0.82), 4.890 (2.11), 4.932 (2.06), 4.962 (16.00), 5.012 (2.70), 5.034 (5.09), 5.045 (5.93), 5.075 (2.43), 5.088 (3.19), 5.268 (1.73), 5.400 (2.00), 5.519 (0.71), 5.551 (1.14), 5.753 (2.12), 7.491 (3.31), 7.513 (6.61), 7.539 (5.42), 7.599 (3.81), 7.696 (4.76), 7.711 (5.58).

### Beispiel 34

### (5RS)-7-(Cyclopropylcarbonyl)-5-{[(3S)-3-fluorpyrrolidin-1-yl]carbonyl}-2-[4-fluor-3-(trifluormethyl)benzyl]-5,6,7,8-tetrahydro[1,2,4]triazolo[4,3-a]pyrazin-3(2H)-on (Isomer 1)

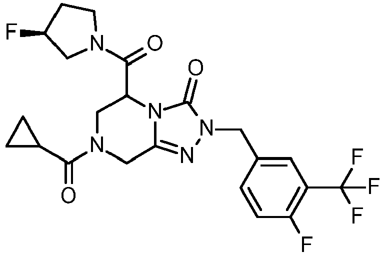

(5RS)-5-{[(3S)-3-Fluorpyrrolidin-1-yl]carbonyl}-2-[4-fluor-3-(trifluormethyl)benzyl]-5,6,7,8-tetrahydro[1,2,4]triazolo[4,3-a]pyrazin-3(2H)-on (Isomer 2) (70.0 mg, 162 µmol) wurde in Dichlormethan (1.5 ml) und N,N-Diisopropylethylamin (57 µl, 320 µmol) gelöst und mit Cyclopropancarbonylchlorid (16 µl, 180 µmol) versetzt. Das Reaktionsgemisch wurde für 2 Stunden bei Raumtemperatur gerührt. Das Reaktionsgemisch wurde über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 69.5 mg (86 % d. Th.) der Titelverbindung erhalten.

LC-MS (Methode 2): Rₜ = 1.51 min; MS (ESlpos): m/z = 500 [M+H]⁺

¹H-NMR (400 MHz, DMSO-d6) δ [ppm]: 0.590 (1.68), 0.681 (4.21), 0.765 (2.35), 1.902 (1.43), 2.020 (0.73), 2.073 (0.73), 2.165 (1.05), 2.220 (0.99), 2.273 (1.53), 2.327 (0.44), 2.670 (0.41), 3.377 (1.06), 3.394 (1.05), 3.479 (1.11), 3.512 (2.57), 3.593 (2.56), 3.775 (0.90), 3.794 (0.85), 3.936 (1.41), 3.966 (2.61), 3.987 (2.65), 4.010 (1.36), 4.027 (1.34), 4.051 (2.05), 4.087 (2.45), 4.115 (0.99), 4.592 (0.97), 4.628 (1.08), 4.706 (1.77), 4.743 (1.45), 4.967 (16.00), 5.055 (2.26), 5.118 (1.87), 5.159 (1.94), 5.273 (1.00), 5.374 (1.74), 5.405 (1.05), 5.506 (1.48), 7.492 (1.88), 7.515 (3.89), 7.540 (3.12), 7.597 (2.69), 7.702 (3.48), 7.719 (3.64).

### Beispiel 35

### (5RS)-5-{[(3S)-3-Fluorpyrrolidin-1-yl]carbonyl}-7-methyl-2-{[6-(trifluormethyl)pyridin-3-yl]methyl}-5,6,7,8-tetrahydro[1,2,4]triazolo[4,3-a]pyrazin-3(2H)-on (Diasteromeregemisch, 2 Isomere)

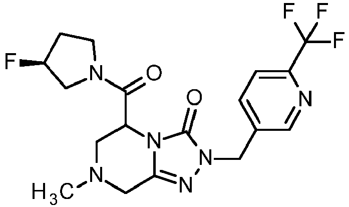

(5RS)-5-{[(3S)-3-Fluorpyrrolidin-1-yl]carbonyl}-2-{[6-(trifluormethyl)pyridin-3-yl]methyl}-5,6,7,8-tetrahydro[1,2,4]triazolo[4,3-a]pyrazin-3(2H)-on (Diasteromeregemisch, 2 Isomere) (29.0 mg, 70.0 µmol) wurde in THF (5.0 ml) vorgelegt. Formaldehyd (16 µl, 37 % in Wasser, 210 µmol) und Natriumtriacetoxyborhydird (59.3 mg, 280 µmol) wurden anschließend zugegeben. Das Reaktionsgemisch wurde nach Rühren über Nacht bei Raumtemperatur mit Wasser und Essigsäure versetzt. Das Gemisch wurde über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 5.10 mg (17 % d. Th.) der Titelverbindung erhalten.

LC-MS (Methode 1): Rₜ = 0.62 min; MS (ESlpos): m/z = 429 [M+H]⁺

¹H-NMR (500 MHz, METHANOL-d4) δ [ppm]: 1.991 (0.44), 2.156 (0.43), 2.185 (0.50), 2.193 (0.51), 2.214 (0.67), 2.225 (0.57), 2.233 (0.60), 2.240 (0.52), 2.248 (0.56), 2.258 (0.63), 2.268 (0.69), 2.281 (0.46), 2.294 (0.59), 2.316 (0.50), 2.332 (0.42), 2.350 (0.51), 2.365 (0.57), 2.381 (0.43), 2.426 (6.34), 2.435 (8.30), 2.439 (16.00), 2.452 (0.99), 2.861 (0.94), 2.899 (0.78), 2.924 (0.45), 2.935 (0.44), 2.977 (0.84), 2.988 (0.92), 2.995 (1.48), 3.003 (1.38), 3.013 (1.29), 3.018 (0.80), 3.029 (0.82), 3.032 (0.82), 3.043 (0.68), 3.084 (0.76), 3.094 (0.81), 3.110 (0.99), 3.120 (1.01), 3.132 (0.63), 3.143 (0.63), 3.157 (0.42), 3.168 (0.41), 3.359 (0.73), 3.369 (1.05), 3.390 (1.09), 3.396 (1.62), 3.426 (2.17), 3.461 (1.18), 3.474 (0.46), 3.578 (1.28), 3.583 (0.82), 3.598 (1.54), 3.606 (2.25), 3.621 (1.42), 3.629 (1.08), 3.636 (1.32), 3.652 (1.12), 3.661 (0.85), 3.672 (1.11), 3.685 (0.94), 3.692 (0.83), 3.705 (1.29), 3.715 (0.83), 3.728 (2.06), 3.761 (0.42), 3.768 (0.45), 3.785 (0.54), 3.792 (0.44), 3.817 (0.76), 3.828 (0.67), 3.840 (1.46), 3.848 (1.02), 3.862 (0.99), 3.866 (0.92), 3.882 (0.94), 3.901 (0.80), 3.927 (0.70), 4.014 (0.56), 4.033 (0.96), 4.052 (0.47), 4.715 (0.67), 4.782 (1.00), 4.793 (1.85), 4.805 (2.17), 4.872 (1.87), 4.882 (1.35), 4.925 (0.83), 4.935 (1.56), 4.945 (0.75), 5.039 (1.07), 5.071 (3.48), 5.094 (2.81), 5.101 (3.10), 5.126 (0.92), 5.133 (1.29), 5.246 (0.47), 5.255 (0.54), 5.262 (0.63), 5.294 (1.32), 5.318 (0.52), 5.352 (0.78), 5.361 (0.69), 6.807 (0.69), 6.828 (0.76), 7.788 (3.64), 7.805 (4.87), 7.822 (0.76), 7.963 (2.13), 7.979 (1.90), 8.693 (3.44), 8.766 (0.59). (Mischung von Diastereomeren)

### Beispiel 36

### (5RS)-2-[(5-Chlorpyridin-3-yl)methyl]-7-{[(1RS)-2,2-difluorcyclopropyl]methyl}-5-{[(3S)-3-fluorpyrrolidin-1-yl]carbonyll-5,6,7,8-tetrahydro[1,2,4]triazolo[4,3-a]pyrazin-3(2H)-on (Diasteromeregemisch, 4 Isomere)

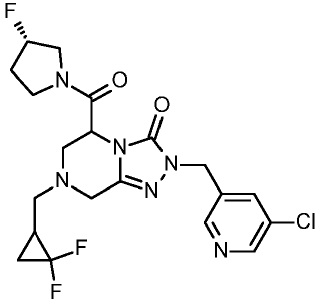

(5RS)-2-[(5-Chlorpyridin-3-yl)methyl]-5-{[(3S)-3-fluorpyrrolidin-1-yl]carbonyl}-5,6,7,8-tetrahydro[1,2,4]triazolo[4,3-a]pyrazin-3(2H)-on (Diasteromeregemisch, 2 Isomere) (100 mg, 263 µmol) wurde in THF (5.0 ml) vorgelegt und N,N-Diisopropylethylamin (180 µl, 1.1 mmol) und (2R)-2-(Brommethyl)-1,1-difluorcyclopropan (67.4 mg, 394 µmol) wurden zugegeben. Das Reaktionsgemisch wurde über Nacht bei Raumtemperatur gerührt. Es wurde erneut N,N-Diisopropylethylamin (180 µl, 1.1 mmol) und (2R)-2-(Brommethyl)-1,1-difluorcyclopropan (67.4 mg, 394 µmol) zugegeben und das Reaktionsgemisch für 24 Stunden bei 60°C und weitere 24 Stunden bei 70 °C gerührt. Anschließed wurde Reakti onsgemisch im Vakuum eingeent und der Rückstand wurde über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 16.00 mg (13 % d. Th.) der Titelverbindung erhalten.

LC-MS (Methode 3): Rₜ = 1.00 min; MS (ESlpos): m/z = 471 [M+H]⁺

### Beispiel 37

### (5RS)-2-[(5-Chlorpyridin-3-yl)methyl]-7-{[(1RS)-2,2-difluorcyclopropyl]methyl}-5-{[(3S)-3-fluorpyrrolidin-1-yl]carbonyl}-5,6,7,8-tetrahydro[1,2,4]triazolo[4,3-a]pyrazin-3(2H)-on (Isomer 1)

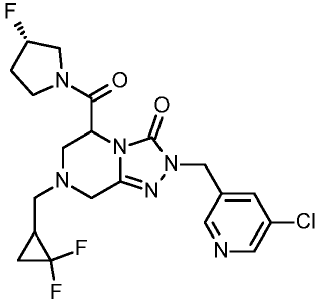

(5RS)-2-[(5-Chlorpyridin-3-yl)methyl]-7-{[(1RS)-2,2-difluorcyclopropyl]methyl}-5-{[(3S)-3-fluorpyrrolidin-1-yl]carbonyl}-5,6,7,8-tetrahydro[1,2,4]triazolo[4,3-a]pyrazin-3(2H)-on (Diasteromeregemisch, 4 Isomere) wurde durch chirale HPLC getrennt [Probenvorbereitung: 16 mg gelöst in 3 ml (Acetonitril/Ethanol, 1:1); Injektionsvolumen: 0.5 ml; Säule: Daicel Chiralpak^{®} AZ-H, 250 × 30 mm; Laufmittel: n-Heptan/Ethanol 1:1 + 0.2% Diethylamin; Fluss: 40 ml/min; Temperatur 40°C; UV Detektion: 220 nm]. Nach der Trenn ung wurden 1.0 mg von Isomer 1 isoliert.

### Isomer 1:

Analytische chirale HPLC: Rₜ = 3.1 min, d.e. = 94% [Säule: Daicel Chiralpak^{®} AZ-3 50 × 4.6 mm; Laufmittel: n-Heptan/Ethanol 1:1 + 0.2 % Diethylamin; Fluss: 1 ml/min; UV-Detektion: 220 nm].

LC-MS (Methode 2): Rt = 1.33 min; MS (ESlpos): m/z = 471 [M+H]⁺

¹H-NMR (500 MHz, DMSO-d6) δ [ppm]: -0.064 (2.42), -0.007 (9.86), 0.007 (7.44), 0.067 (4.28), 0.844 (2.05), 0.859 (2.23), 0.998 (1.12), 1.056 (2.23), 1.121 (6.33), 1.135 (12.84), 1.150 (6.14), 1.160 (5.40), 1.175 (9.30), 1.189 (5.02), 1.236 (5.58), 1.601 (1.67), 1.889 (1.30), 1.988 (16.00), 2.237 (0.93), 2.361 (2.98), 2.582 (2.42), 2.596 (2.42), 2.635 (3.91), 2.723 (1.86), 2.867 (2.79), 3.025 (1.86), 3.056 (2.05), 3.137 (1.30), 3.452 (2.98), 3.481 (3.35), 3.492 (3.16), 3.521 (4.28), 3.537 (2.98), 3.568 (2.23), 3.648 (3.91), 3.658 (3.16), 3.678 (3.16), 3.988 (1.86), 4.008 (2.05), 4.022 (3.91), 4.037 (3.72), 4.051 (1.86), 4.074 (1.12), 4.893 (2.60), 4.902 (2.98), 4.912 (3.72), 4.944 (7.26), 5.283 (1.12), 5.390 (1.30), 5.471 (1.12), 7.777 (5.40), 7.782 (5.21), 8.442 (6.33), 8.571 (5.58).

### Beispiel 38

### (5RS)-2-[(5-Chlorpyridin-3-yl)methyl]-5-{[(3S)-3-fluorpyrrolidin-1-yl]carbonyl}-7-methyl-5,6,7,8-tetrahydro[1,2,4]triazolo[4,3-a]pyrazin-3(2H)-on (Diasteromeregemisch, 2 Isomere)

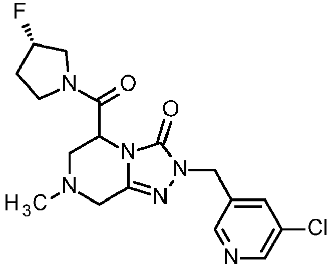

(5S)-2-[(5-Chlorpyridin-3-yl)methyl]-5-{[(3S)-3-fluorpyrrolidin-1-yl]carbonyl}-5,6,7,8-tetrahydro[1,2,4]triazolo[4,3-a]pyrazin-3(2H)-on (Diasteromeregemisch, 2 Isomere) (100 mg, 97 % Reinheit, 255 µmol) wurde in N,N-Diisopropylethylamin (180 µl, 1.0 mmol) vorgelegt und Methyliodid (24 µl, 380 µmol) wurden zugegeben. Das Reaktionsgemisch wurde über Nacht bei Raumtemperatur gerührt. Anschließed wurde Reaktionsgemisch im Vakuum eingeengt und der Rückstand wurde über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 15.0 mg (97 % Reinheit, 14 % d. Th.) der Titelverbindung erhalten.

LC-MS (Methode 1): Rₜ = 0.53 min; MS (ESlpos): m/z = 395 [M+H]⁺

¹H-NMR (400 MHz, DMSO-d6) δ [ppm]: 0.938 (0.72), 0.954 (0.68), 2.099 (0.76), 2.113 (0.76), 2.144 (0.78), 2.241 (0.74), 2.302 (6.87), 2.313 (16.00), 2.365 (0.43), 2.669 (0.70), 2.688 (0.47), 2.709 (0.43), 2.880 (1.23), 2.891 (0.98), 2.925 (1.40), 2.937 (1.97), 2.981 (0.64), 2.993 (0.62), 3.029 (0.51), 3.217 (0.96), 3.254 (1.97), 3.270 (0.94), 3.291 (2.22), 3.354 (1.37), 3.388 (0.90), 3.405 (0.74), 3.434 (0.41), 3.500 (1.54), 3.531 (1.66), 3.546 (2.03), 3.561 (1.80), 3.571 (1.62), 3.585 (1.60), 3.599 (1.46), 3.668 (1.17), 3.691 (1.03), 3.731 (0.88), 3.755 (0.84), 3.809 (0.86), 3.881 (0.70), 3.940 (0.47), 3.963 (0.82), 3.988 (0.76), 4.046 (0.45), 4.722 (0.70), 4.734 (0.41), 4.761 (0.47), 4.774 (1.00), 4.856 (0.53), 4.866 (1.11), 4.879 (1.07), 4.892 (1.33), 4.939 (7.84), 4.994 (0.49), 5.270 (0.72), 5.347 (0.53), 5.393 (0.70), 5.479 (0.55), 7.767 (3.28), 8.433 (4.27), 8.571 (3.14). (Mischung von Diastereomeren)

### Beispiel 39

### (5RS)-2-[(5-Chlorpyridin-3-yl)methyl]-5-{[(3S)-3-fluorpyrrolidin-1-yl]carbonyl}-7-methyl-5,6,7,8-tetrahydro[1,2,4]triazolo[4,3-a]pyrazin-3(2H)-on (Isomer 1)

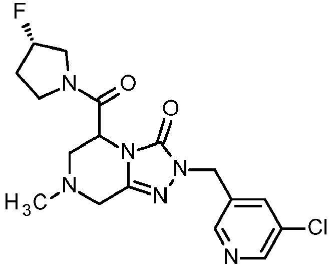

(5RS)-2-[(5-Chlorpyridin-3-yl)methyl]-5-{[(3S)-3-fluorpyrrolidin-1-yl]carbonyl}-7-methyl-5,6,7,8-tetrahydro[1,2,4]triazolo[4,3-a]pyrazin-3(2H)-on (Diasteromeregemisch, 2 Isomere) wurde durch chirale HPLC getrennt [Probenvorbereitung: 15 mg gelöst in 2 ml (Acetonitril/Ethanol, 1:1); Injektionsvolumen: 0.2 ml; Säule: Daicel Chiralpak^{®} AS-H, 250 × 20 mm; Laufmittel: n-Heptan/Ethanol 70:30 + 0.2% Diethylamin; Fluss: 20 ml/min; Temperatur 28°C; UV Detektion: 220 nm]. Nach der Trennung wurden 1.9 mg von Isomer 1, welches zuerst eluierte, und 2.2 mg von Isomer 2, welches später eluierte, isoliert.

### Isomer 1:

Analytische chirale HPLC: Rₜ = 1.2 min, d.e. = 100% [Säule: Daicel Chiralpak^{®} AS-3 50 × 4.6 mm; Laufmittel: n-Heptan/Ethanol1:1 + 0.2 % Diethylamin; Fluss: 1 ml/min; UV-Detektion: 220 nm].

¹H-NMR (500 MHz, METHANOL-d4) δ [ppm]: 1.284 (2.69), 1.291 (1.03), 1.298 (5.12), 1.313 (2.66), 2.203 (0.41), 2.213 (0.49), 2.226 (0.41), 2.230 (0.44), 2.245 (0.44), 2.266 (0.60), 2.278 (0.43), 2.285 (0.49), 2.290 (0.61), 2.313 (0.60), 2.323 (0.48), 2.347 (0.66), 2.358 (0.62), 2.415 (9.84), 2.427 (16.00), 2.938 (0.97), 2.949 (1.02), 2.964 (1.50), 2.974 (1.44), 2.983 (1.01), 2.993 (0.95), 3.019 (0.81), 3.025 (1.10), 3.033 (2.48), 3.048 (2.01), 3.061 (0.95), 3.077 (1.25), 3.088 (1.26), 3.103 (0.90), 3.113 (0.86), 3.359 (1.15), 3.390 (1.67), 3.396 (1.92), 3.427 (2.43), 3.570 (0.44), 3.584 (2.86), 3.593 (0.93), 3.602 (2.09), 3.615 (2.33), 3.623 (0.42), 3.633 (1.38), 3.652 (1.09), 3.659 (1.25), 3.669 (1.44), 3.677 (1.39), 3.684 (1.16), 3.698 (2.02), 3.723 (3.08), 3.775 (0.40), 3.848 (0.42), 4.009 (0.92), 4.028 (1.71), 4.046 (0.85), 4.060 (0.44), 4.081 (0.51), 4.106 (0.41), 4.845 (1.79), 4.866 (0.96), 4.909 (1.36), 4.920 (2.67), 4.930 (1.29), 4.955 (1.12), 4.987 (5.96), 5.000 (5.48), 5.032 (1.03), 5.256 (1.03), 5.312 (0.62), 5.360 (0.91), 5.363 (0.91), 5.417 (0.62), 7.845 (3.45), 7.850 (2.92), 8.472 (3.49), 8.490 (3.40), 8.493 (3.28).

### Beispiel 40

### (5RS)-2-[(5-Chlorpyridin-3-yl)methyl]-5-{[(3S)-3-fluorpyrrolidin-1-yl]carbonyl}-7-methyl-5,6,7,8-tetrahydro[1,2,4]triazolo[4,3-a]pyrazin-3(2H)-on (Isomer 2)

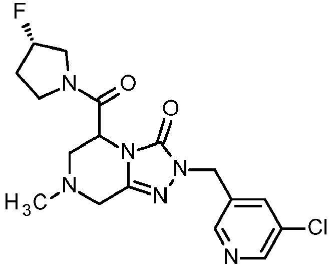

(5RS)-2-[(5-Chlorpyridin-3-yl)methyl]-5-{[(3S)-3-fluorpyrrolidin-1-yl]carbonyl}-7-methyl-5,6,7,8-tetrahydro[1,2,4]triazolo[4,3-a]pyrazin-3(2H)-on (Diasteromeregemisch, 2 Isomere) wurde durch chirale HPLC getrennt [Probenvorbereitung: 15 mg gelöst in 2 ml (Acetonitril/Ethanol, 1:1); Injektionsvolumen: 0.2 ml; Säule: Daicel Chiralpak^{®} AS-H, 250 × 20 mm; Laufmittel: n-Heptan/Ethanol 70:30 + 0.2% Diethylamin; Fluss: 20 ml/min; Temperatur 28°C; UV Detektion: 220 nm]. Nach der Trennung wurden 1.9 mg von Isomer 1, welches zuerst eluierte, und 2.2 mg von Isomer 2, welches später eluierte, isoliert.

### Isomer 2:

Analytische chirale HPLC: Rₜ = 1.43 min, d.e. = 100% [Säule: Daicel Chiralpak^{®} AS-3 50 × 4.6 mm; Laufmittel: n-Heptan/Ethanol 1:1 + 0.2 % Diethylamin; Fluss: 1 ml/min; UV-Detektion: 220 nm].

¹H-NMR (500 MHz, METHANOL-d4) δ [ppm]: 0.903 (0.44), 0.912 (0.46), 0.915 (0.44), 0.926 (0.60), 0.940 (0.52), 1.279 (1.12), 1.284 (4.20), 1.291 (1.57), 1.298 (8.14), 1.313 (4.19), 1.323 (0.56), 2.154 (0.42), 2.183 (0.42), 2.206 (0.66), 2.221 (0.44), 2.224 (0.46), 2.235 (0.48), 2.239 (0.52), 2.243 (0.55), 2.246 (0.62), 2.257 (0.60), 2.260 (0.56), 2.268 (0.47), 2.274 (0.44), 2.286 (0.63), 2.367 (0.55), 2.373 (0.52), 2.377 (0.52), 2.424 (15.48), 2.427 (16.00), 2.862 (0.90), 2.873 (0.93), 2.887 (1.09), 2.899 (1.07), 2.942 (0.83), 2.953 (0.86), 2.967 (1.12), 2.978 (1.09), 3.018 (0.93), 3.033 (2.59), 3.048 (2.53), 3.062 (0.85), 3.102 (0.90), 3.113 (0.98), 3.125 (1.26), 3.136 (1.29), 3.149 (0.79), 3.160 (0.77), 3.393 (1.32), 3.424 (2.00), 3.429 (1.93), 3.443 (0.70), 3.452 (1.09), 3.461 (2.20), 3.474 (0.63), 3.489 (0.62), 3.493 (0.52), 3.501 (0.48), 3.523 (0.54), 3.530 (0.55), 3.556 (2.51), 3.568 (0.59), 3.581 (2.43), 3.587 (1.79), 3.597 (0.67), 3.604 (0.73), 3.611 (1.65), 3.808 (1.32), 3.811 (1.29), 3.823 (1.09), 3.826 (1.21), 3.835 (1.01), 3.846 (2.14), 3.857 (1.07), 3.873 (2.03), 3.891 (1.77), 3.896 (1.85), 3.921 (1.12), 3.962 (0.67), 3.969 (0.67), 4.768 (1.26), 4.779 (2.38), 4.790 (1.44), 4.848 (3.77), 4.951 (1.17), 4.983 (6.48), 4.996 (7.08), 5.027 (1.30), 5.233 (0.50), 5.239 (0.83), 5.246 (0.47), 5.346 (1.42), 5.454 (0.83), 7.843 (3.21), 7.848 (3.22), 8.471 (2.89), 8.493 (2.94).

### Beispiel 41

### (5RS)-5-{[(3S)-3-Fluorpyrrolidin-1-yl]carbonyl}-2-{[3-fluor-2-(trifluormethyl)pyridin-4-yl]methyl}-5,6,7,8-tetrahydro[1,2,4]triazolo[4,3-a]pyrazin-3(2H)-on (Diasteromeregemisch, 2 Isomere)

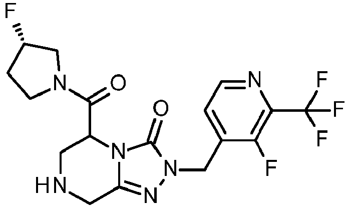

tert-Butyl-(5RS)-5-{[(3S)-3-fluorpyrrolidin-1-yl]carbonyl}-2-{[3-fluor-2-(trifluormethyl)pyridin-4-yl]methyl}-3-oxo-2,5,6,8-tetrahydro[1,2,4]triazolo[4,3-a]pyrazin-7(3H)-carboxylat (Diasteromeregemisch, 2 Isomere) (36.0 mg, 67.6 µmol) wurde in Dichlormethan (2.0 ml) gelöst und bei Raumtemperatur mit Trifluoressigsäure (400 µl, 5.2 mmol) versetzt. Nachdem das Reaktionsgemisch über Nacht bei Raumtemperatur gerührt wurde, wurde das Lösungsmittel unter Vakuum entfernt. Der Rückstand wurde in Ethylacetat gelöst und Wasser zugegeben. Die organische Phase wurde abgetrennt und die wässrige Phase dreimal mit Ethylacetat extrahiert. Die vereinigten organischen Phasen wurden über Natriumsulfat getrocknet, filtriert und das Filtrat eingeengt. Es wurden 29.0 mg (99 % d. Th.) der Titelverbindung erhalten.

LC-MS (Methode 2): Rₜ = 1.03 min; MS (ESlpos): m/z = 433 [M+H]⁺

¹H-NMR (500 MHz, DMSO-d6) δ [ppm]: -0.120 (1.29), -0.007 (16.00), 0.006 (8.84), 0.117 (1.15), 1.160 (1.85), 1.175 (3.77), 1.189 (1.96), 1.227 (2.45), 1.236 (2.34), 1.258 (0.84), 1.736 (0.70), 1.988 (6.39), 2.361 (1.71), 2.518 (3.91), 2.522 (2.86), 2.635 (1.89), 3.604 (8.98), 4.008 (0.84), 4.022 (1.54), 4.037 (1.71), 4.051 (0.70), 4.073 (0.63), 4.247 (0.63), 5.139 (1.05), 5.152 (1.36), 5.160 (1.22), 5.315 (0.42), 5.418 (0.45), 7.639 (1.19), 8.588 (1.92), 8.598 (1.85). (Mischung von Diastereomeren)

### Beispiel 42

### (5RS)-5-{[(3S)-3-Fluorpyrrolidin-1-yl]carbonyl}-2-{[6-(trifluormethyl)pyridin-3-yl]methyl}-5,6,7,8-tetrahydro[1,2,4]triazolo[4,3-a]pyrazin-3(2H)-on (Diasteromeregemisch, 2 Isomere)

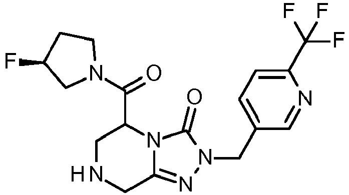

5-{[(3S)-3-Fluorpyrrolidin-1-yl]carbonyl}-2-{[6-(trifluormethyl)pyridin-3-yl]methyl}[1,2,4]triazolo [4,3-a]pyrazin-3(2H)-on (86.0 mg, 210 µmol) wurde in Ethanol (5 ml) und Methanol (1 ml) gelöst und unter Verwendung einer Hydrierapparatur (H-Cube^{®}, Pd/C 10% Palladium, 1 bar, 95°C, Fluss: 1ml/min) umgesetzt. Es wurden 58 mg (67 % d. Th.) der Titelverbindung erhalten.

LC-MS (Methode 2): Rₜ = 0.94 min; MS (ESIpos): m/z = 415 [M+H]⁺

¹H-NMR (500 MHz, DMSO-d6) δ [ppm]: 1.813 (1.19), 3.283 (0.46), 3.311 (1.04), 3.401 (2.43), 3.406 (1.50), 3.412 (3.22), 3.420 (5.01), 3.430 (5.50), 3.447 (5.07), 3.462 (6.71), 3.465 (2.94), 3.480 (4.70), 3.485 (2.04), 3.491 (1.30), 3.496 (1.84), 3.504 (1.75), 3.510 (1.83), 3.518 (1.89), 3.526 (1.32), 3.533 (0.84), 3.542 (0.45), 3.686 (1.67), 3.694 (1.47), 3.709 (3.60), 3.718 (3.20), 3.744 (3.82), 3.751 (3.63), 3.767 (1.83), 3.775 (1.55), 3.804 (1.31), 3.815 (1.42), 3.860 (0.41), 3.911 (0.64), 3.943 (14.14), 3.945 (13.65), 3.958 (0.58), 3.966 (0.59), 3.979 (1.56), 3.986 (0.43), 4.051 (0.46), 4.054 (0.44), 4.080 (0.47), 4.083 (0.45), 4.414 (0.47), 4.422 (1.05), 4.429 (0.63), 4.437 (1.97), 4.456 (3.43), 4.460 (1.97), 4.463 (1.97), 4.469 (2.08), 4.474 (1.74), 4.485 (2.52), 4.500 (15.08), 4.525 (0.43), 4.694 (0.47), 4.725 (0.51), 4.798 (0.45), 4.809 (0.69), 5.551 (0.84), 5.751 (6.23), 7.252 (0.77), 7.260 (1.11), 7.263 (1.24), 7.273 (2.04), 7.278 (2.66), 7.283 (2.57), 7.284 (2.74), 7.290 (4.96), 7.294 (3.81), 7.298 (3.76), 7.304 (5.95), 7.308 (5.23), 7.312 (3.53), 7.316 (4.24), 7.328 (5.60), 7.334 (3.69), 7.336 (3.61), 7.341 (14.08), 7.344 (16.00), 7.349 (2.55), 7.356 (5.58), 7.357 (6.30), 7.361 (2.79), 7.369 (1.04), 7.372 (1.70), 7.374 (1.14), 8.065 (2.18).

### Beispiel 43

### tert-Butyl-(5RS)-2-[(5-chlorpyridin-3-yl)methyl]-5-{[(3S)-3-fluorpyrrolidin-1-yl]carbonyl}-3-oxo-2,5,6,8-tetrahydro[1,2,4]triazolo[4,3-a]pyrazin-7(3H)-carboxylat (Diasteromeregemisch, 2 Isomere)

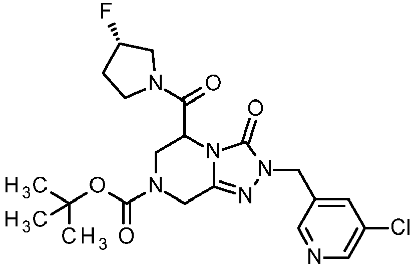

tert-Butyl-(5S)-5-{[(3S)-3-fluorpyrrolidin-1-yl]carbonyl}-3-oxo-2,5,6,8-tetrahydro[1,2,4]triazolo[4,3-a]pyrazin-7(3H)-carboxylat (Diasteromeregemisch, 2 Isomere) (589 mg, 1.66 mmol) wurde in Acetonitril (15 ml) vorgelegt. Caesiumcarbonat (1.35 g, 4.14 mmol) und 3-Chlor-5-(chlormethyl)pyridin (282 mg, 1.74 mmol) wurden anschließend zugegeben. Das Reaktionsgemisch wurde nach Rühren für 72 Stunden bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 782 mg (94 % Reinheit, 92 % d. Th.) der Titelverbindung erhalten.

LC-MS (Methode 2): Rt = 1.42 min; MS (ESlpos): m/z = 481 [M+H]⁺

### Beispiel 44

### (5RS)-2-[(5-Chlorpyridin-3-yl)methyl]-5-{[(3S)-3-fluorpyrrolidin-1-yl]carbonyl}-5,6,7,8-tetrahydro[1,2,4]triazolo[4,3-a]pyrazin-3(2H)-on (Diasteromeregemisch, 2 Isomere)

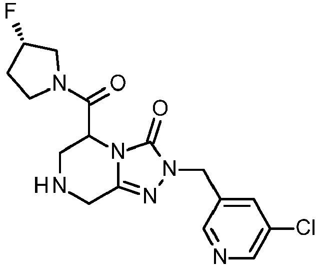

tert-Butyl-(5RS)-2-[(5-chlorpyridin-3-yl)methyl]-5-{[(3S)-3-fluorpyrrolidin-1-yl]carbonyl}-3-oxo-2,5,6,8-tetrahydro[1,2,4]triazolo[4,3-a]pyrazin-7(3H)-carboxylat (Diasteromeregemisch, 2 Isomere) (782 mg, 94 % Reinheit, 1.53 mmol) wurde in Dichlormethan (15 ml) gelöst und bei Raumtemperatur mit Trifluoressigsäure (2.4 ml, 31 mmol) versetzt. Nachdem das Reaktionsgemisch über Nacht bei Raumtemperatur gerührt wurde, wurde das Lösungsmittel unter Vakuum entfernt. Es wurden 582 mg (97 % d. Th.) der Titelverbindung erhalten.

LC-MS (Methode 2): Rₜ = 0.73 min; MS (ESlpos): m/z = 381 [M+H]⁺

### Beispiel 45

### tert-Butyl-(5RS)-5-{[(3S)-3-fluorpyrrolidin-1-yl]carbonyl}-2-{[3-fluor-2-(trifluormethyl)pyridin-4-yl]methyl}-3-oxo-2,5,6,8-tetrahydro[1,2,4]triazolo[4,3-a]pyrazin-7(3H)-carboxylat (Diasteromeregemisch, 2 Isomere)

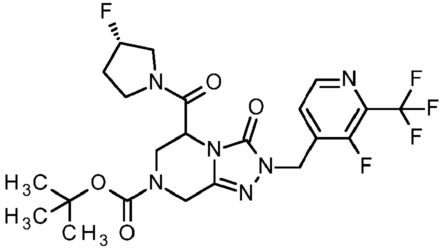

tert-Butyl-(5RS)-5-{[(3S)-3-fluorpyrrolidin-1-yl]carbonyl}-3-oxo-2,5,6,8-tetrahydro[1,2,4]triazolo[4,3-a]pyrazin-7(3H)-carboxylat (Diasteromeregemisch, 2 Isomere) (212 mg, 40 % Reinheit, 239 µmol) wurde in Acetonitril (2.0 ml) vorgelegt. Caesiumcarbonat (156 mg, 477 µmol) und 4-(Chlormethyl)-3-fluor-2-(trifluormethyl)pyridin (56.1 mg, 262 µmol) wurden anschließend zugegeben. Das Reaktionsgemisch wurde nach Rühren für 72 Stunden bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 36.0 mg (28 % d. Th.) der Titelverbindung erhalten.

LC-MS (Methode 2): Rₜ = 1.69 min; MS (ESIpos): m/z = 477 [M-tBuCO₂]⁺

### B. Bewertung der pharmakologischen Wirksamkeit

Die pharmakologische Aktivität der erfindungsgemäßen Verbindungen kann durch in vitro- und in vivo-Untersuchungen, wie sie dem Fachmann bekannt sind, nachgewiesen werden. Die nachfolgenden Anwendungsbeispiele beschreiben die biologische Wirkung der erfindungsgemäßen Verbindungen, ohne die Erfindung auf diese Beispiele zu beschränken.

**Abkürzungen und Akronyme:**

| | |
|---|---|
| ATP | Adenosintriphosphat |
| COPD | Chronisch-Obstruktive Lungenerkrankung |
| LPS | Lipopolysaccharid |
| PGP | Prolin-Glycin-Prolin |
| Poly(I:C) | Polyinosin-Polycytidylsäure |

### B-1 Biochemischer humaner Prolyl Endopeptidase (PREP) Assay zur Identifizierung von Inhibitoren der PREP-Aktivität unter Benutzung eines fluoreszent markierten Substrats

### Prinzip des Assays:

Der enzymatische Umsatz des fluoreszenten Peptidsubstrats wurde anhand der Messung der Fluoreszenzintensität beobachtet. Die Enzymaktivität wurde durch die Ermittlung der Anfangssteigung in der Fluoreszenzzunahme bestimmt. Verbindungen, die PREP inhibieren, wurden anhand der Verringerung der Anfangssteigung im Vergleich zu einem Reaktionsansatz ohne Testverbindung identifiziert.

### Aktivitätsbestimmung:

IC₅₀-Werte wurden durch die Auftragung der prozentualen PREP-Aktivität gegen die Konzentration der Testsubstanz durch Interpolation ermittelt.

### Assaybeschreibung:

Zu rekombinanter volle-Länge humaner Prolyl Endopeptidase (PREP, R&D-Systems, 4308-SE; Endkonzentration z.B. 0.4 nM, Volumen: 25 µl) in Reaktionspuffer (50 mM Tris-HCl, pH 7.5; 150 mM NaCl; 0.13 % BSA, 5 mM EDTA, 3 mM GSH, 0.005% Brij-35) wurde eine Testverbindung (in DMSO, in einem passenden Endkonzentrationsbereich von 1 nM bis 30 µM, Volumen: 1 µl) in einer Vertiefung einer 384-well-Mikrotiterplatte zugegeben. Die Reaktion wurde durch Zugabe des Substrats Z-Gly-Pro-AMC (Endkonzentration 50 µl; Z = Carboxybenzyl; AMC = 7-Amino-4-methylcoumarin, Volumen: 25 µl) gestartet. Der Fortschritt der PREP Reaktion wurde durch Messung der Fluoreszenzintensität in einem Tecan SAFIRE II Plattenspektrophotometer über einen Zeitraum von 60 min bei 32°C beobachtet (Anregungswellenlänge: 360 nm, Emissionswellenlänge: 465 nm).

In der folgenden Tabelle B-1 sind die so erhaltenen IC₅₀-Werte aus dem humanem Prolylendopeptidase Assay für individuelle Ausführungsbeispiele der Erfindung zusammengestellt (zum Teil als Mittelwerte aus mehreren unabhängigen Einzelbestimmungen):

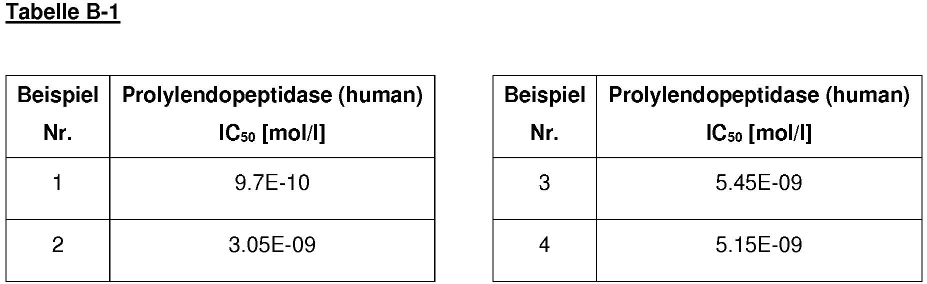

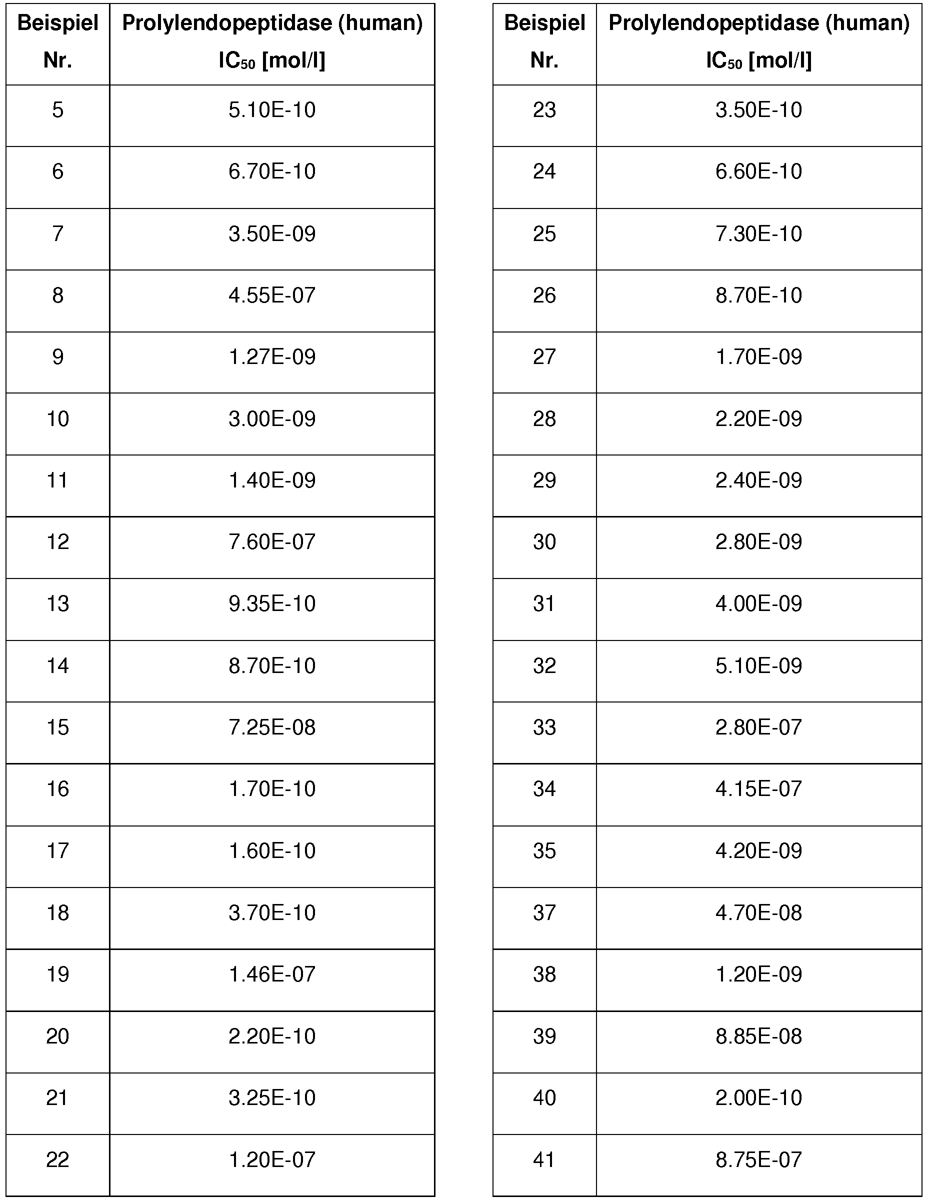

### B-2 Biochemischer muriner Prolyl Endopeptidase (PREP) Assay

### Aktivitätsbestimmung:

IC₅₀-Werte wurden durch die Auftragung der prozentualen PREP-Aktivität gegen die Konzentration der Testsubstanz durch Interpolation ermittelt.

### Mausgehirnhomogenat Präparation:

Maushirn aus BalbC Mäusen in 0.8 ml eines Gemisches aus 100mM Natrium-Phosphat (pH 7.0) sowie 3 mM Dithiothreitol wurde im OmniBead Ruptor 4x25sec homogenisiert. Das erhaltene Homogenat wurde 20 min bei 13000 Upm und 4°C z entrifugiert. Der aliquotierte Überstand wurde bei -80°C eingefroren.

### Assaybeschreibung:

Mausgehirnhomogenat wurde 1:100 verdünnt in einem Reaktionspuffer (50 mM Tris-HCl, pH 7,5; 150 mM NaCl; 0,13 % BSA, 5 mM EDTA, 3 mM GSH, 0,005% Brij-35) und 25 µl von der Lösung wurden in eine Vertiefung einer 384-well-Mikrotiterplatte gegeben. Eine Testverbindung (in DMSO, in einem passenden Endkonzentrationsbereich von z.B. 1 nM bis 30 µM, Volumen: 1 µl) wurde zugegeben. Die Reaktion wird durch Zugabe des Substrats Z-Gly-Pro-AMC (Endkonzentration 50 µM; Z = Carboxybenzyl; AMC = 7-Amino-4-methylcoumarin, Volumen: 25 µl) gestartet. Der Fortschritt der PREP Reaktion wurde durch Messung der Fluoreszenzintensität in einem geeigneten Plattenspektrometer über einen Zeitraum von z.B. 60 min bei 32°C beobachtet (Anregungswellenlänge: 360 nm, Emis sionswellenlänge: 465 nm).

In der folgenden Tabelle 1 sind die so erhaltenen IC₅₀-Werte aus dem KDR- und dem PDGFRβ-Kinase-Assay für individuelle Ausführungsbeispiele der Erfindung zusammengestellt (zum Teil als Mittelwerte aus mehreren unabhängigen Einzelbestimmungen):

### B-3 Wirksamkeit von PREP-Inhibitoren in Zigarettenrauch-exponierten Mäusen

Die Eignung der erfindungsgemäßen Substanzen für die Behandlung/Prophylaxe der genannten Krankheiten kann in folgendem Tiermodell gezeigt werden.

### Material und Methoden:

**Mäuse:** Stamm: BALB/C, Herkunft: Charles River Niederlande, Geschlecht: männlich, Alter: 8 bis 10 Wochen, Gewicht: 25 g, n = 21.

**Vehikel:** Solutol, EtOH, Water (S: 40 %, EtOH 10 %, Water 50 % (v/v))

Für die Verabreichung von PREP-Inhibitoren per Schlundsonde wurden diese in dem oben beschriebenen Vehikel gelöst (c = 3750 µg/ml).

Die Mäuse wurden in drei Gruppen zu je sieben Tieren eingeteilt und folgendermaßen behandelt:
Gruppe 1: keine Rauchexposition (Raumluft), zweimal täglich 200 µl Vehikel ohne Wirkstoff
Gruppe 2: Rauchexposition, zweimal täglich 200 µl Vehikel ohne Wirkstoff
Gruppe 3: Rauchexposition, zweimal täglich 200 µl Vehikel mit Wirkstoff (jeweils 30 mg/kg)

### Rauchexposition:

Zur Exposition mit Zigarettenrauch wurden die Tiere der Gruppen 2 und 3 (die Tiere der Gruppe 1 erfuhren keine Rauchexposition und verblieben in den Haltungskäfigen) zweimal täglich in eine Expositionskammer mit einem Gesamtvolumen von 52 Litern eingebracht, die durch Trenngitter in 16 Einzelkäfige eingeteilt war. Dabei wurden die Tiere, die gruppenweise gehalten wurden (7 Tiere pro Käfig), zur Berauchung in jeweils einen Einzelkäfig der Expositionskammer eingebracht (7 Tiere pro Käfig). Zwischen den zweimal täglichen Expositionen wurde eine rauchfreie Zeit von 5 Stunden gelegt. Insgesamt wurden die Tiere an fünf aufeinanderfolgenden Tagen beraucht. Zigaretten wurden jeweils paarweise verbrannt. Der Hauptstromrauch der Zigaretten wurde in die Expositionskammer eingeleitet.

Die Rauchexposition wurde nach folgenden Schema durchgeführt:
- Tag 1: 1. Berauchung: 2 mal 2 Zigaretten, 2. Berauchung: 3 mal 2 Zigaretten
- Tag 2: 1. Berauchung: 4mal 2 Zigaretten, 2. Berauchung: 5 mal 2 Zigaretten
- Tag 3: 1. Berauchung: 6 mal 2 Zigaretten, 2. Berauchung: 7 mal 2 Zigaretten
- Tage 4 und 5: 1. und 2. Berauchung: jeweils 7 mal 2 Zigaretten

Die Behandlung der Tiere mit PREP-Inhibitoren bzw. Vehikel wurde 15 Minuten vor der ersten Berauchung am Tag 1 begonnen. Im Folgenden erhielten die Tiere zweimal täglich im Abstand von 8 Stunden PREP-Inhibitoren bzw. Vehikel in der oben beschriebenen Dosierung.

Am Tag 6 wurden die Tiere durch die intraperitoneale Gabe von 150 mg/kg Pentobarbital (Euthesate^{®}) getötet. Die Tracheen wurden freipräpariert und zur Einführung von Kanülen eingeschnitten. Über diese Kanülen wurden die Lungen viermal mit 37 °C warmer, physiologischer Kochsalzlösung gespült. Die Zellen dieser jeweils vier Fraktionen der Broncho-Alveolären Lavage-Flüssigkeit (BALF) wurden bei 4 °C bei 400 xg für 5 Minuten zentrifugiert. Im Anschluss wurden die Zellpellets vereinigt und in jeweils 150 µl physiologischer Kochlösung (4 °C) resuspendiert. Im Anschluss an die Färbung mit Türk'scher Lösung wurden die Gesamtzahl der Zellen lichtmikroskopisch gezählt. Zur Quantifizierung der neutrophilen Granulozyten (kurz: Neutrophile) wurden die Zellen auf Objektträger transferiert (Cytospin) und mit DiffQuick (Dade A.G., Düdingen, Switzerland) gefärbt. Abschließend wurden Neutrophile, Makrophagen und Lymphozyten gezählt und die relativen Anteile an der zuvor bestimmten Gesamtzahl berechnet. Zur Bestimmung der PGP-Konzentration in der BALF wurden jeweils 200 µl des Überstandes der ersten BALF-Fraktion mit Bestatin versetzt (Endkonzentration: 1 mM) und bis zur Messung bei -20 °C gelagert.

### Quantifizierung von PGP in der Broncho-Alveolären Lavage-Flüssigkeit (BALF)

PGP wurde nach der Methode von Hardison et al. [Hardison et al., J. Immunol. 2009, 182:4423-4431] unter Verwendung folgender Ausrüstung bestimmt: ESI-LC-MS/MS (Shimadzu HPLC, Columbia, MA, USA) mit einem Finnigan TSQ quantum discovery Max Quarupole Massenspektrometer in Verbindung mit Elektrospray-Wärmeionisation (Thermo Fisher Scientific, San Jose, CA, USA) und einer Atlantis dC18 Säule (100 mm × 2.1 mm, dp = 3 µm, Waters Chromatography, Milford, MA, USA) bzw. einer Atlantis pre-column Vorsäule (10 mm × 2.1 mm, dp = 3 µm, Waters).

### C. Ausführunasbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesium¬stearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5 %-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96 %), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 mL orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungs¬gemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Natriumchlorid-Lösung, Glucose¬lösung 5 % und/oder PEG 400-Lösung 30 %) gelöst. Die Lösung wird steril filtriert und in sterile und pyrogenfreie Injektions¬behältnisse abgefüllt.

## Patentansprüche

1. Verbindung der Formel (I) 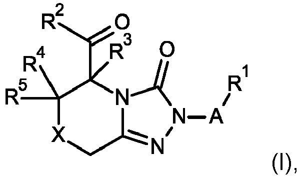 in welcher
A für (C₁-C₄)-Alkylen oder CD₂ steht,
wobei (C₁-C₄)-Alkylen mit Hydroxy, (C₁-C₄)-Alkoxy sowie bis zu fünfmal mit Fluor substituiert sein kann,
oder
für eine Gruppe der Formel 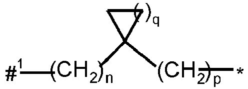 steht, worin
n für 0 oder 1 steht,
p für 0 oder 1 steht,
q für 1 oder 2 steht,
wobei
#¹ die Verknüpfung zum Stickstoffatom des 5-Oxo-4,5-dihydro-1H-1,2,4-triazol-1-yl-Ringes markiert,
* die Verknüpfung zu R¹ markiert,
X für eine Gruppe der Formel 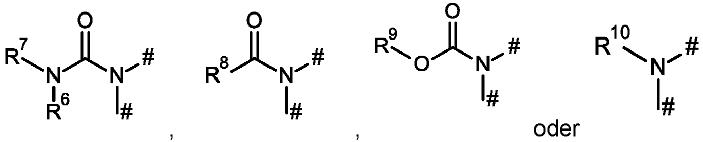 steht,
wobei
# die Verknüpfung zu dem jeweiligen Kohlenstoffatom des Ringes markiert,
R⁶ für (C₁-C₆)-Alkyl, (C₃-C₆)-Cycloalkyl, Phenyl oder 5- bis 6-gliedriges Heteroaryl steht,
worin (C₁-C₆)-Alkyl mit Hydroxy, Cyano, (C₁-C₄)-Alkoxy oder bis zu fünfmal mit Fluor substituiert sein kann,
worin (C₃-C₆)-Cycloalkyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann, w
orin Phenyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin 5- bis 6-gliedriges Heteroaryl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
R⁷ für Wasserstoff oder (C₁-C₄)-Alkyl steht,
worin (C₁-C₄)-Alkyl bis zu fünfmal mit Fluor substituiert sein kann,
R⁸ für (C₁-C₄)-Alkyl, (C₃-C₆)-Cycloalkyl, Phenyl oder 5- bis 6-gliedriges Heteroaryl steht,
worin (C₁-C₄)-Alkyl mit Hydroxy, Cyano, (C₁-C₄)-Alkoxyoder bis zu fünfmal mit Fluor substituiert sein kann,
worin (C₃-C₆)-Cycloalkyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin Phenyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin 5- bis 6-gliedriges Heteroaryl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
R⁹ für (C₁-C₄)-Alkyl, (C₃-C₆)-Cycloalkyl oder Phenyl steht,
worin (C₁-C₄)-Alkyl bis zu fünfmal mit Fluor substituiert sein kann,
worin (C₃-C₆)-Cycloalkyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin Phenyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
R¹⁰ für Wasserstoff, (C₁-C₄)-Alkyl, (C₃-C₆)-Cycloalkyl oder Phenyl steht,
worin (C₁-C₄)-Alkyl mit Hydroxy, Cyano, (C₁-C₄)-Alkoxy, (C₃-C₆)-Cycloalkyl oder bis zu fünfmal mit Fluor substituiert sein kann,
worin (C₃-C₆)-Cycloalkyl bis zu viermal mit Fluor substituiert sein kann,
worin (C₃-C₆)-Cycloalkyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin Phenyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin 5- bis 6-gliedriges Heteroaryl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
oder
für Sauerstoff steht,
R¹ für (C₃-C₇)-Cycloalkyl, Phenyl oder 5- bis 10-gliedriges Heteroaryl steht,
wobei (C₃-C₇)-Cycloalkyl mit 1 bis 3 Substituenten unabhängig voneinander ausgewählt aus der Gruppe Halogen, Cyano, Trifluormethyl, (C₁-C₄)-Alkyl und (C₁-C₄)-Alkoxy substituiert sein kann,
worin (C₁-C₄)-Alkyl bis zu fünfmal mit Fluor substituiert sein kann,
wobei Phenyl mit 1 bis 4 Substituenten unabhängig voneinander ausgewählt aus der Gruppe Halogen, Cyano, (C₁-C₄)-Alkyl, (C₃-C₆)-Cycloalkyl, Trifluormethyl, (C₁-C₄)-Alkoxy, (C₃-C₅)-Cycloalkoxy, Monofluormethoxy, Difluormethoxy, Trifluormethoxy, Hydroxycarbonyl, (C₁-C₄)-Alkoxycarbonyl, Aminocarbonyl, Mono-(C₁-C₄)-alkylaminocarbonyl, Di-(C₁-C₄)-alkylaminocarbonyl, (C₃-C₅)-Cycloalkyl-aminocarbonyl, (C₁-C₄)-Alkylsulfanyl, (C₁-C₄)-Alkylsulfonyl, (C₁-C₄)-Alkylsulfonimidoyl, (C₁-C₄)-Cycloalkylsulfonyl, Aminosulfonyl, Mono-(C₁-C₄)-alkylaminosulfonyl, Di-(C₁-C₄)-alkylaminosulfonyl, (C₁-C₄)-Alkylsulfinyl, Amino, Mono-(C₁-C₄)-alkylamino oder Di-(C₁-C₄)-alkylamino substituiert ist,
oder
wobei 5- bis 10-gliedriges Heteroaryl mit 1 bis 4 Substituenten unabhängig voneinander ausgewählt aus der Gruppe Halogen, Cyano, (C₁-C₄)-Alkyl, (C₃-C₆)-Cycloalkyl, Trifluormethyl, (C₁-C₄)-Alkoxy, (C₃-C₅)-Cycloalkoxy, Difluormethoxy, Trifluormethoxy, 2,2,2-Trifluorethoxy, Phenyl, Hydroxycarbonyl, (C₁-C₄)-Alkoxy-carbonyl, Aminocarbonyl, Mono-(C₁-C₄)-alkylaminocarbonyl, Di-(C₁-C₄)-alkylaminocarbonyl, (C₃-C₅)-Cycloalkyl-aminocarbonyl, (C₁-C₄)-Alkylsulfanyl, (C₁-C₄)-Alkylsulfonyl, (C₁-C₄)-Alkylsulfonimidoyl, (C₁-C₄)-Cycloalkylsulfonyl, Aminosulfonyl, Mono-(C₁-C₄)-alkylaminosulfonyl, Di-(C₁-C₄)-alkylaminosulfonyl, (C₁-C₄)-Alkylsulfinyl, Amino, Mono-(C₁-C₄)-alkylamino oder Di-(C₁-C₄)-alkylamino substituiert sein kann,
worin Phenyl mit Methyl, Ethyl, Chlor, Fluor oder Methoxy substituiert sein kann,
R² für eine Gruppe der Formel 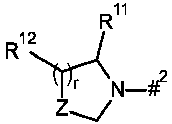 steht, wobei
#² die Verknüpfung zum Carbonyl-Kohlenstoffatom markiert,
r für 0 oder 1 steht,
Z für O, NR¹⁴, S, SO, SO₂ oder CR^{13A}R^{13B} steht,
worin
R^{13A} für Wasserstoff, Halogen, Cyano, (C₁-C₄)-Alkyl, (C₃-C₆)-Cycloalkyl, Trifluormethyl, Difluormethyl, Fluormethyl, Hydroxy, (C₁-C₄)-Alkoxy, (C₃-C₅)-Cycloalkoxy, Difluormethoxy, Trifluormethoxy oder 2,2,2-Trifluorethoxy steht,
worin (C₁-C₄)-Alkyl mit Hydroxy, Amino, Mono-(C₁-C₄)-alkylamino oder Di-(C₁-C₄)-alkylamino substituiert sein kann,
R^{13B} für Wasserstoff, Fluor oder (C₁-C₄)-Alkyl, steht, worin (C₁-C₄)-Alkyl bis zu fünfmal mit Fluor substituiert sein kann,
R¹⁴ für Wasserstoff oder Methyl steht,
R¹¹ für Wasserstoff, Cyano, (C₁-C₄)-Alkyl, Acetyl oder Formyl steht,
worin (C₁-C₄)-Alkyl mit Hydroxy oder bis zu fünfmal mit Fluor substituiert sein kann,
worin Acetyl mit Hydroxy oder bis zu dreimal mit Fluor substituiert sein kann,
R¹² für Wasserstoff, Fluor oder (C₁-C₄)-Alkyl steht,
oder
R¹¹ und R¹² zusammen mit den Kohlenstoffatomen, an die sie gebunden sind, einen Cyclopropyl- oder Cyclobutyl-Ring bilden,
worin der Cyclopropyl- oder Cyclobutyl-Ring bis zu zweimal mit Fluor substituiert sein kann,
oder
R¹² und R^{13A} zusammen mit den Kohlenstoffatomen, an die sie gebunden sind, einen Cyclopropyl- oder Cyclobutyl-Ring bilden,
worin der Cyclopropyl- oder Cyclobutyl -Ring bis zu zweimal mit Fluor substituiert sein kann,
oder
R^{13A} und R^{13B} zusammen mit dem Kohlenstoffatom, an die sie gebunden sind, einen Cyclopropyl- oder Cyclobutyl-Ring bilden,
worin der Cyclopropyl- oder Cyclobutyl-Ring bis zu zweimal mit Fluor substituiert sein kann,
wobei R¹², R^{13A} und R^{13B} für Wasserstoff stehen, wenn R¹¹ nicht für Wasserstoff steht,
wobei R¹¹ für Wasserstoff steht, wenn einer der Substituenten R¹², R^{13A} oder R^{13B} nicht für Wasserstoff steht,
oder
für eine Gruppe der Formel 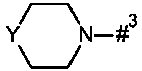 steht, wobei
#³ die Verknüpfung zum Carbonyl-Kohlenstoffatom markiert,
Y für NR¹⁵, CR^{16A}R^{16B}, Sauerstoff oder Schwefel steht,
worin
R¹⁵ für Wasserstoff oder Methyl steht,
R^{16A} für Wasserstoff oder Methyl steht,
R^{16B} für Wasserstoff oder Methyl steht,
R³ für Wasserstoff steht,
R⁴ für Wasserstoff oder (C₁-C₄)-Alkyl steht,
worin (C₁-C₄)-Alkyl mit (C₁-C₄)-Alkoxy, Hydroxy, Mono-(C₁-C₄)-alkylamino oder Di-(C₁-C₄)-alkylamino sowie bis zu fünfmal mit Fluor substituiert sein kann,
R⁵ für Wasserstoff steht,
sowie die Salze, Solvate und Solvate der Salze der Verbindungen der Formel (I).

2. Verbindung der Formel (I) nach Anspruch 1, in welcher
A für (C₁-C₄)-Alkylen steht,
X für eine Gruppe der Formel 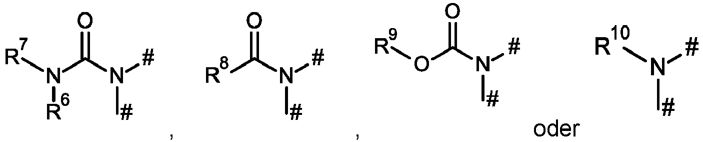 steht,
wobei
# die Verknüpfung zu dem jeweiligen Kohlenstoffatom des Ringes markiert,
R⁶ für (C₁-C₆)-Alkyl, (C₃-C₅)-Cycloalkyl, Phenyl oder 6-gliedriges Heteroaryl steht,
worin (C₁-C₆)-Alkyl bis zu dreimal mit Fluor substituiert sein kann,
worin (C₃-C₅)-Cycloalkyl bis zu dreimal mit Fluor substituiert sein kann,
worin Phenyl mit 1 bis 2 Substituenten unabhängig voneinander ausgewählt aus der Gruppe Fluor, Chlor Brom, Cyano, Trifluormethyl, Methyl, Ethyl, Methoxy und Ethoxy substituiert sein kann,
worin 6-gliedriges Heteroaryl mit 1 bis 2 Substituenten unabhängig voneinander ausgewählt aus der Gruppe Fluor, Chlor, Cyano, Trifluormethyl, Methyl, Ethyl, Methoxy und Ethoxy substituiert sein kann,
R⁷ für Wasserstoff oder Methyl steht, worin Methyl bis zu dreimal mit Fluor substituiert sein kann,
R⁸ für (C₁-C₄)-Alkyl, (C₃-C₆)-Cycloalkyl, Phenyl oder 6-gliedriges Heteroaryl steht,
worin (C₁-C₆)-Alkyl bis zu dreimal mit Fluor substituiert sein kann,
worin (C₃-C₅)-Cycloalkyl bis zu dreimal mit Fluor substituiert sein kann,
R⁹ für (C₁-C₄)-Alkyl oder Phenyl steht, worin (C₁-C₄)-Alkyl bis zu dreimal mit Fluor substituiert sein kann,
R¹⁰ für Wasserstoff, (C₁-C₄)-Alkyl, (C₃-C₆)-Cycloalkyl oder Phenyl steht,
worin (C₁-C₄)-Alkyl mit Cyclopropyl oder Cyclobuytl oder bis zu fünfmal mit Fluor substituiert sein kann,
worin Cyclopropyl oder Cyclobuytl bis zu zweimal mit Fluor substituiert sein kann,
worin (C₃-C₆)-Cycloalky bis zu dreimal mit Fluor substituiert sein kann,
worin Phenyl mit 1 bis 3 Substituenten unabhängig voneinander ausgewählt aus der Gruppe Fluor, Chlor Brom, Cyano, Trifluormethyl, Methyl, Ethyl, Methoxy und Ethoxy substituiert sein kann,
oder
für Sauerstoff steht,
R¹ für Phenyl oder 6-gliedriges Heteroaryl steht,
wobei Phenyl mit 1 bis 3 Substituenten unabhängig voneinander ausgewählt aus der Gruppe Fluor, Chlor, Brom, Cyano, Methyl, Ethyl, Cyclopropyl, Trifluormethyl, Methoxy, Ethoxy, Monofluormethoxy, Difluormethoxy, Trifluormethoxy, Hydroxycarbonyl, Hydroxycarbonyl, Methoxycarbonyl, Ethoxycarbonyl, tert-Butoxycarbonyl, Methylaminocarbonyl, Dimethylaminocarbonyl substituiert ist,
wobei 6-gliedriges Heteroaryl mit 1 bis 3 Substituenten unabhängig voneinander ausgewählt aus der Gruppe Fluor, Chlor, Brom, Cyano, Methyl, Ethyl, Cyclopropyl, Trifluormethyl, Methoxy, Ethoxy, Monofluormethoxy, Difluormethoxy, Trifluormethoxy, Hydroxycarbonyl, Hydroxycarbonyl, Methoxycarbonyl, Ethoxycarbonyl, tert-Butoxycarbonyl, Methylaminocarbonyl, Dimethylaminocarbonyl substituiert ist,
R² für eine Gruppe der Formel 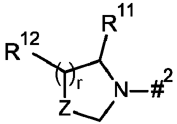 steht, wobei
#² die Verknüpfung zum Carbonyl-Kohlenstoffatom markiert,
r für 1 steht,
Z für CR^{13A}R^{13B} steht,
worin
R^{13A} für Wasserstoff, Fluor, Methyl, Trifluormethyl, Difluormethyl, Fluormethyl, Hydroxy, Methoxy, Difluormethoxy oder Trifluormethoxy steht,
R^{13B} für Wasserstoff oder Fluor steht,
R¹¹ für Wasserstoff steht,
R¹² für Wasserstoff oder Fluor steht,
R³ für Wasserstoff steht,
R⁴ für Wasserstoff, Fluor oder Methyl, steht, worin Methyl bis zu dreimal mit Fluor substituiert sein kann,
R⁵ für Wasserstoff steht,
sowie die Salze, Solvate und Solvate der Salze der Verbindungen der Formel (I).

3. Verbindung der Formel (I) nach Anspruch 1 oder 2, in welcher
A für ―CH₂- steht,
X für eine Gruppe der Formel 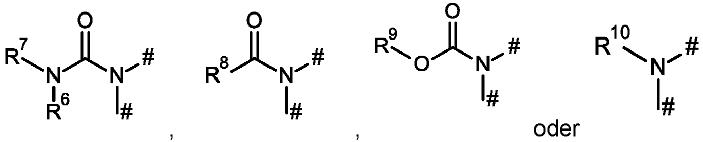 steht,
wobei
# die Verknüpfung zu dem jeweiligen Kohlenstoffatom des Ringes markiert,
R⁶ für (C₁-C₄)-Alkyl, Cyclopropyl oder Phenyl steht,
worin (C₁-C₄)-Alkyl bis zu dreimal mit Fluor substituiert sein kann,
worin Phenyl mit 1 oder 2 Substituenten unabhängig voneinander ausge-wählt aus der Gruppe Chlor oder Trifluormethyl substituiert ist,
R⁷ für Wasserstoff steht,
R⁸ für Methyl, Ethyl, Propyl, 3,3,3-Trifluorpropyl oder Cyclopropyl steht,
R⁹ für tert.-Butyl steht,
R¹⁰ für Wasserstoff, Methyl, Ethyl, 2,2,2-Trifluorethyl, Cyclopropyl oder Cyclobutyl steht,
worin Methyl mit Cyclopropyl substituiert sein kann,
worin Cyclopropyl bis zu zweimal mit Fluor substituiert sein kann,
R¹ für Phenyl oder Pyridyl steht,
wobei Phenyl mit 1 oder 2 Substituenten unabhängig voneinander ausgewählt aus der Gruppe Fluor, Methyl oder Trifluormethyl substituiert ist,
wobei Pyridyl mit 1 oder 2 Substituenten unabhängig voneinander ausgewählt aus der Gruppe Fluor, Chlor oder Trifluormethyl substituiert ist,
R² für eine Gruppe der Formel 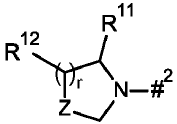 steht, wobei
#² die Verknüpfung zum Carbonyl-Kohlenstoffatom markiert,
r für 1 steht,
Z für CR^{13A}R^{13B} steht,
worin
R^{13A} für Wasserstoff oder Fluor steht,
R^{13B} für Wasserstoff steht,
R¹¹ für Wasserstoff steht,
R¹² für Wasserstoff oder Fluor steht,
R³ für Wasserstoff steht,
R⁴ für Wasserstoff oder Methyl steht,
R⁵ für Wasserstoff steht,
sowie die Salze, Solvate und Solvate der Salze der Verbindungen der Formel (I).

4. Verfahren zur Herstellung einer Verbindung, wie in den Ansprüchen 1 bis 3 definiert, **dadurch gekennzeichnet, dass** man entweder
[A] eine Verbindung der Formel (II) 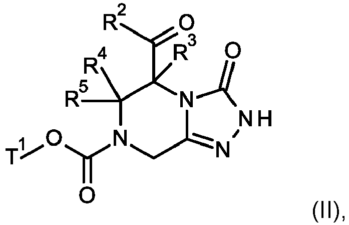 in welcher R¹, R², R³, R⁴ und R⁵ jeweils die in Anspruch 1 genannten Bedeutungen haben,
und
T¹ für (C₁-C₄)-Alkyl oder Benzyl steht,
in einem inerten Lösungsmittel in Gegenwart einer geeigneten Base mit einer Verbindung der Formel (III) 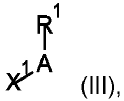 in welcher
A und R¹ die in Anspruch 1 genannten Bedeutungen haben,
und
X¹ für eine geeignete Abgangsgruppe, insbesondere Chlor, Brom, Iod, Mesylat {(methylsulfonyl)oxy}, Triflat {[(trifluormethyl)sulfonyl]oxy}, Nonaflat {[(nonafluor-butyl)sulfonyl]oxy}, Nosylat {[(4-nitrophenyl)sulfonyl]oxy} oder Tosylat {[(4-methyl-phenyl)sulfonyl]oxy} steht,
zu einer Verbindung der Formel (IV) 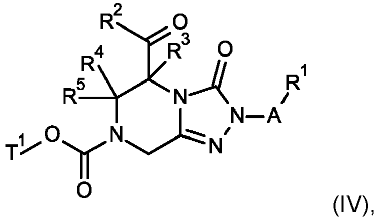 in welcher A, R¹, R², R³, R⁴, R⁵ und T¹ jeweils die in Anspruch 1 genannten Bedeutungen haben,
umsetzt,
diese dann gegebenenfalls durch Entfernen der Gruppe "T¹" in einem inerten Lösungsmittel in Gegenwart einer geeigneten Base oder Säure in eine Verbindung der Formel (V) 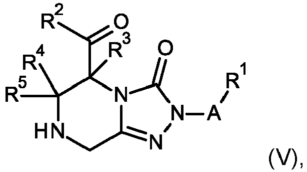 überführt,
in welcher A, R¹, R², R³, R⁴ und R⁵ jeweils die in Anspruch 1 genannten Bedeutungen haben,
und diese dann gegebenenfalls in einem inerten Lösungsmittel
[A1] in Gegenwart einer geeigneten Base mit einer Verbindung der Formel (VI) 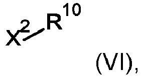 in welcher
R¹⁰ die in Anspruch 1 genannten Bedeutungen hat,
und
X² für eine geeignete Abgangsgruppe, insbesondere Chlor, Brom, Iod, Mesylat {(methylsulfonyl)oxy}, Triflat {[(trifluormethyl)sulfonyl]oxy}, Nonaflat {[(nonafluor-butyl)sulfonyl]oxy}, Nosylat {[(4-nitrophenyl)sulfonyl]oxy} oder Tosylat {[(4-methyl-phenyl)sulfonyl]oxy} steht,
umsetzt und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt,
oder
[A2] in Gegenwart einer geeigneten Base mit einer Verbindung der Formel (VII) 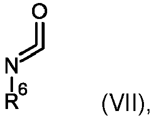 in welcher
R⁶ die in Anspruch 1 genannten Bedeutungen hat,
umsetzt und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt,
oder
[A3] in Gegenwart einer geeigneten Base mit einer Verbindung der Formel (VIII) 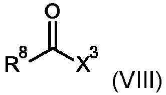 in welcher
R⁸ die in Anspruch 1 genannten Bedeutungen hat,
und
X³ für eine geeignete Abgangsgruppe, insbesondere Chlor, Brom oder Hydroxy steht,
umsetzt, und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt,
oder
[B] eine Verbindung der Formel (IX) 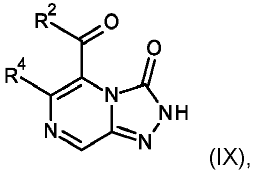 in welcher R², R⁴ und R⁵ jeweils die in Anspruch 1 genannten Bedeutungen haben,
in einem inerten Lösungsmittel in Gegenwart einer geeigneten Base mit einer Verbindung der Formel (III) 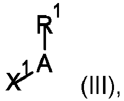 in welcher
A und R¹ die in Anspruch 1 genannten Bedeutungen haben,
und
X¹ für eine geeignete Abgangsgruppe, insbesondere Chlor, Brom, Iod, Mesylat {(methylsulfonyl)oxy}, Triflat {[(trifluormethyl)sulfonyl]oxy}, Nonaflat {[(nonafluor-butyl)sulfonyl]oxy}, Nosylat {[(4-nitrophenyl)sulfonyl]oxy} oder Tosylat {[(4-methyl-phenyl)sulfonyl]oxy} steht,
zu einer Verbindung der Formel (X) 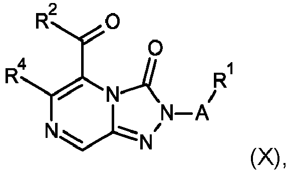 in welcher A, R¹, R² und R⁴ jeweils die in Anspruch 1 genannten Bedeutungen haben,
umsetzt, und anschließend in einem geeigneten Lösungsmittel in Gegenwart eines Palladiumkatalysators in einer Wasserstoffatmosphäre hydriert und die resultierende Verbindung der Formel (V) 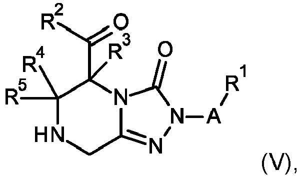 wie unter [A] beschrieben umsetzt und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

5. Verbindung, wie in einem der Ansprüche 1 bis 3 definiert, zur Behandlung und/oder Prophylaxe von Krankheiten.

6. Verbindung der Formel (I), wie in den Ansprüchen 1 bis 3 definiert, zur Verwendung in einem Verfahren zur Behandlung und/oder Prophylaxe von inflammatorischen Lungenerkrankungen, vor allem der chronisch-obstruktiven Lungenerkrankung (COPD), des Lungenemphysems, der chronischen Bronchitis, der Bronchiektasie, der pulmonalen Hypertonie in der COPD (PH-COPD), der akuten Exazerbation in der COPD, der zystischen Fibrose (Mukoviszidose, CF), des Asthmas, sowie der idiopathischen Lungenfibrose (IPF) des Bronchiolitis obliterans-Syndrom (BOS), der Arteriosklerose, der Myokarditis, sowie entzündlichen Haut- und Augenerkrankungen bzw. entzündlichen Erkrankungen der inneren Organe.

7. Verwendung einer Verbindung der Formel (I), wie in den Ansprüchen 1 bis 3 definiert, zur Herstellung eines Arzneimittels zur Behandlung und/oder Prophylaxe von inflammatorischen Lungenerkrankungen, vor allem der chronisch-obstruktiven Lungenerkrankung (COPD), des Lungenemphysems, der chronischen Bronchitis, der Bronchiektasie, der pulmonalen Hypertonie in der COPD (PH-COPD), der akuten Exazerbation in der COPD, der zystischen Fibrose (Mukoviszidose, CF), des Asthmas, sowie der idiopathischen Lungenfibrose (IPF) des Bronchiolitis obliterans-Syndrom (BOS), der Arteriosklerose, der Myokarditis, sowie entzündlichen Haut- und Augenerkrankungen bzw. entzündlichen Erkrankungen der inneren Organe.

8. Arzneimittel enthaltend eine Verbindung, wie in einem der Ansprüche 1 bis 3 definiert, in Kombination mit einem oder mehreren inerten, nicht-toxischen, pharmazeutisch geeigneten Hilfsstoffen.

9. Arzneimittel enthaltend eine Verbindung, wie in den Ansprüchen 1 bis 3 definiert, in Kombination mit einem oder mehreren Wirkstoffen ausgewählt aus der Gruppe der PDE 5-Inhibitoren, sGC-Aktivatoren, sGC-Stimulatoren, Prostacyclin-Analoga, IP-Rezeptor-Agonisten, Endothelin-Antagonisten, der die Signaltransduktionskaskade inhibierenden Verbindungen und Pirfenidon.

10. Arzneimittel nach Anspruch 8 oder 9 zur Behandlung und/oder Prophylaxe von von inflammatorischen Lungenerkrankungen, vor allem der chronisch-obstruktiven Lungenerkrankung (COPD), des Lungenemphysems, der chronischen Bronchitis, der Bronchiektasie, der pulmonalen Hypertonie in der COPD (PH-COPD), der akuten Exazerbation in der COPD, der zystischen Fibrose (Mukoviszidose, CF), des Asthmas, sowie der idiopathischen Lungenfibrose (IPF) des Bronchiolitis obliterans-Syndrom (BOS), der Arteriosklerose, der Myokarditis, sowie entzündlichen Haut- und Augenerkrankungen bzw. entzündlichen Erkrankungen der inneren Organe.

## Claims

1. Compound of the formula (I) 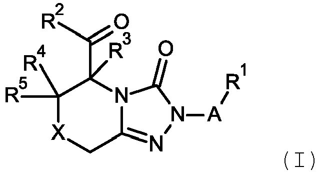 in which
A is (C₁-C₄)-alkylene or CD₂,
where (C₁-C₄)-alkylene may be substituted by hydroxyl and (C₁-C₄)-alkoxy and up to pentasubstituted by fluorine,
or
is a group of the formula 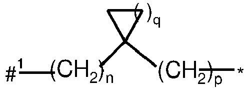 in which
n is 0 or 1,
p is 0 or 1,
q is 1 or 2,
where
#¹ marks the bond to the nitrogen atom of the 5-oxo-4,5-dihydro-1H-1,2,4-triazol-1-yl ring,
* marks the bond to R¹,
X is a group of the formula 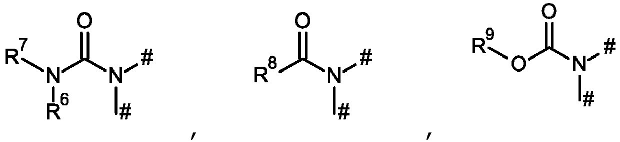 or 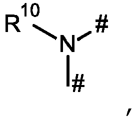 where
# marks the bond to the respective carbon atom of the ring,
R⁶ is (C₁-C₆)-alkyl, (C₃-C₆)-cycloalkyl, phenyl or 5- to 6-membered heteroaryl,
in which (C₁-C₆)-alkyl may be substituted by hydroxyl, cyano or (C₁-C₄)-alkoxy or up to pentasubstituted by fluorine,
in which (C₃-C₆)-cycloalkyl may be substituted by 1 to 3 substituents selected independently from the group of halogen, cyano, trifluoromethyl, (C₁-C₄)-alkyl and (C₁-C₄)-alkoxy,
in which phenyl may be substituted by 1 to 3 substituents selected independently from the group of halogen, cyano, trifluoromethyl, (C₁-C₄)-alkyl and (C₁-C₄)-alkoxy,
in which 5- to 6-membered heteroaryl may be substituted by 1 to 3 substituents selected independently from the group of halogen, cyano, trifluoromethyl, (C₁-C₄)-alkyl and (C₁-C₄)-alkoxy,
R⁷ is hydrogen or (C₁-C₄)-alkyl,
in which (C₁-C₄)-alkyl may be up to pentasubstituted by fluorine
R⁸ is (C₁-C₄)-alkyl, (C₃-C₆)-cycloalkyl, phenyl or 5- to 6-membered heteroaryl,
in which (C₁-C₄)-alkyl may be substituted by hydroxyl, cyano or (C₁-C₄)-alkoxy or up to pentasubstituted by fluorine,
in which (C₃-C₆)-cycloalkyl may be substituted by 1 to 3 substituents selected independently from the group of halogen, cyano, trifluoromethyl, (C₁-C₄)-alkyl and (C₁-C₄)-alkoxy,
in which phenyl may be substituted by 1 to 3 substituents selected independently from the group of halogen, cyano, trifluoromethyl, (C₁-C₄)-alkyl and (C₁-C₄)-alkoxy,
in which 5- to 6-membered heteroaryl may be substituted by 1 to 3 substituents selected independently from the group of halogen, cyano, trifluoromethyl, (C₁-C₄)-alkyl and (C₁-C₄)-alkoxy,
R⁹ is (C₁-C₄)-alkyl, (C₃-C₆)-cycloalkyl or phenyl,
in which (C₁-C₄)-alkyl may be up to pentasubstituted by fluorine,
in which (C₃-C₆)-cycloalkyl may be substituted by 1 to 3 substituents selected independently from the group of halogen, cyano, trifluoromethyl, (C₁-C₄)-alkyl and (C₁-C₄)-alkoxy,
in which phenyl may be substituted by 1 to 3 substituents selected independently from the group of halogen, cyano, trifluoromethyl, (C₁-C₄)-alkyl and (C₁-C₄)-alkoxy,
R¹⁰ is hydrogen, (C₁-C₄)-alkyl, (C₃-C₆)-cycloalkyl or phenyl,
in which (C₁-C₄)-alkyl may be substituted by hydroxyl, cyano, (C₁-C₄)-alkoxy or (C₃-C₆)-cycloalkyl or up to pentasubstituted by fluorine,
in which (C₃-C₆)-cycloalkyl may be up to tetrasubstituted by fluorine,
in which (C₃-C₆)-cycloalkyl may be substituted by 1 to 3 substituents selected independently from the group of halogen, cyano, trifluoromethyl, (C₁-C₄)-alkyl and (C₁-C₄)-alkoxy,
in which phenyl may be substituted by 1 to 3 substituents selected independently from the group of halogen, cyano, trifluoromethyl, (C₁-C₄)-alkyl and (C₁-C₄)-alkoxy,
in which 5- to 6-membered heteroaryl may be substituted by 1 to 3 substituents selected independently from the group of halogen, cyano, trifluoromethyl, (C₁-C₄)-alkyl and (C₁-C₄)-alkoxy,
or
is oxygen,
R¹ is (C₃-C₇)-cycloalkyl, phenyl or 5- to 10-membered heteroaryl,
where (C₃-C₇)-cycloalkyl may be substituted by 1 to 3 substituents selected independently from the group of halogen, cyano, trifluoromethyl, (C₁-C₄)-alkyl and (C₁-C₄)-alkoxy,
in which (C₁-C₄)-alkyl may be up to pentasubstituted by fluorine,
where phenyl is substituted by 1 to 4 substituents selected independently from the group of halogen, cyano, (C₁-C₄)-alkyl, (C₃-C₆)-cycloalkyl, trifluoromethyl, (C₁-C₄)-alkoxy, (C₃-C₅)-cycloalkoxy, monofluoromethoxy, difluoromethoxy, trifluoromethoxy, hydroxycarbonyl, (C₁-C₄)-alkoxycarbonyl, aminocarbonyl, mono- (C₁-C₄)-alkylaminocarbonyl, di-(C₁-C₄)-alkylaminocarbonyl, (C₃-C₅)-cycloalkylaminocarbonyl, (C₁-C₄)-alkylsulfanyl, (C₁-C₄)-alkylsulfonyl, (C₁-C₄)-alkylsulfonimidoyl, (C₁-C₄)-cycloalkylsulfonyl, aminosulfonyl, mono-(C₁-C₄)-alkylaminosulfonyl, di-(C₁-C₄)-alkylaminosulfonyl, (C₁-C₄)-alkylsulfinyl, amino, mono- (C₁-C₄)-alkylamino or di-(C₁-C₄)-alkylamino,
or
where 5- to 10-membered heteroaryl may be substituted by 1 to 4 substituents selected independently from the group of halogen, cyano, (C₁-C₄)-alkyl, (C₃-C₆)-cycloalkyl, trifluoromethyl, (C₁-C₄)-alkoxy, (C₃-C₅)-cycloalkoxy, difluoromethoxy, trifluoromethoxy, 2,2,2-trifluoroethoxy, phenyl, hydroxycarbonyl, (C₁-C₄)-alkoxycarbonyl, aminocarbonyl, mono-(C₁-C₄)-alkylaminocarbonyl, di-(C₁-C₄)-alkylaminocarbonyl, (C₃-C₅)-cycloalkylaminocarbonyl, (C₁-C₄)-alkylsulfanyl, (C₁-C₄)-alkylsulfonyl, (C₁-C₄)-alkylsulfonimidoyl, (C₁-C₄)-cycloalkylsulfonyl, aminosulfonyl, mono-(C₁-C₄)-alkylaminosulfonyl, di-(C₁-C₄)-alkylaminosulfonyl, (C₁-C₄)-alkylsulfinyl, amino, mono- (C₁-C₄)-alkylamino or di-(C₁-C₄)-alkylamino,
in which phenyl may be substituted by methyl, ethyl, chlorine, fluorine or methoxy,
R² is a group of the formula 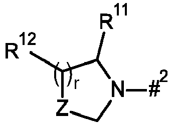 where
#² marks the bond to the carbonyl carbon atom,
r is 0 or 1,
Z is O, NR¹⁴, S, SO, SO₂ or CR^{13A}R^{13B},
in which
R^{13A} is hydrogen, halogen, cyano, (C₁-C₄)-alkyl, (C₃-C₆)-cycloalkyl, trifluoromethyl, difluoromethyl, fluoromethyl, hydroxyl, (C₁-C₄)-alkoxy, (C₃-C₅)-cycloalkoxy, difluoromethoxy, trifluoromethoxy or 2,2,2-trifluoroethoxy,
in which (C₁-C₄)-alkyl may be substituted by hydroxyl, amino, mono-(C₁-C₄)-alkylamino or di-(C₁-C₄)-alkylamino,
R^{13B} is hydrogen, fluorine or (C₁-C₄)-alkyl,
in which (C₁-C₄)-alkyl may be up to pentasubstituted by fluorine
R¹⁴ is hydrogen or methyl,
R¹¹ is hydrogen, cyano, (C₁-C₄)-alkyl, acetyl or formyl,
in which (C₁-C₄)-alkyl may be substituted by hydroxyl or up to pentasubstituted by fluorine,
in which acetyl may be substituted by hydroxyl or up to trisubstituted by fluorine,
R¹² is hydrogen, fluorine or (C₁-C₄)-alkyl,
or
R¹¹ and R¹² together with the carbon atoms to which they are bonded form a cyclopropyl or cyclobutyl ring,
in which the cyclopropyl or cyclobutyl ring may be up to disubstituted by fluorine,
or
R¹² and R^{13A} together with the carbon atoms to which they are bonded form a cyclopropyl or cyclobutyl ring,
in which the cyclopropyl or cyclobutyl ring may be up to disubstituted by fluorine,
or
R^{13A} and R^{13B} together with the carbon atom to which they are bonded form a cyclopropyl or cyclobutyl ring,
in which the cyclopropyl or cyclobutyl ring may be up to disubstituted by fluorine,
where R¹², R^{13A} and R^{13B} are hydrogen when R¹¹ is not hydrogen, where R¹¹
is hydrogen when one of the R¹², R^{13A} and R^{13B} substituents is not hydrogen,
or
is a group of the formula 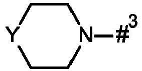 where
#³ marks the bond to the carbonyl carbon atom,
Y is NR¹⁵, CR^{16A}R^{16B}, oxygen or sulfur,
in which
R¹⁵ is hydrogen or methyl,
R^{16A} is hydrogen or methyl,
R^{16B} is hydrogen or methyl;
R³ is hydrogen,
R⁴ is hydrogen or (C₁-C₄)-alkyl, in which (C₁-C₄)-alkyl may be substituted by (C₁-C₄)-alkoxy, hydroxyl, mono-(C₁-C₄)-alkylamino or di-(C₁-C₄)-alkylamino and up to pentasubstituted by fluorine,
R⁵ is hydrogen,
and the salts, solvates and solvates of the salts of the compounds of the formula (I).

2. Compound of the formula (I) according to Claim 1, in which
A is (C₁-C₄)-alkylene,
X is a group of the formula 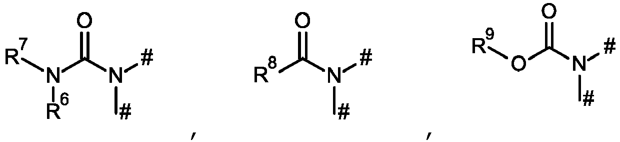 or 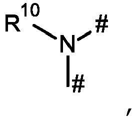 where
# marks the bond to the respective carbon atom of the ring,
R⁶ is (C₁-C₆)-alkyl, (C₃-C₅)-cycloalkyl, phenyl or 6-membered heteroaryl,
in which (C₁-C₆)-alkyl may be up to trisubstituted by fluorine,
in which (C₃-C₅)-cycloalkyl may be up to trisubstituted by fluorine,
in which phenyl may be substituted by 1 to 2 substituents selected independently from the group of fluorine, chlorine, bromine, cyano, trifluoromethyl, methyl, ethyl, methoxy and ethoxy,
in which 6-membered heteroaryl may be substituted by 1 to 2 substituents selected independently from the group of fluorine, chlorine, cyano, trifluoromethyl, methyl, ethyl, methoxy and ethoxy,
R⁷ is hydrogen or methyl,
in which methyl may be up to trisubstituted by fluorine,
R⁸ is (C₁-C₄)-alkyl, (C₃-C₆)-cycloalkyl, phenyl or 6-membered heteroaryl,
in which (C₁-C₆)-alkyl may be up to trisubstituted by fluorine,
in which (C₃-C₅)-cycloalkyl may be up to trisubstituted by fluorine,
R⁹ is (C₁-C₄)-alkyl or phenyl,
in which (C₁-C₄)-alkyl may be up to trisubstituted by fluorine,
R¹⁰ is hydrogen, (C₁-C₄)-alkyl, (C₃-C₆)-cycloalkyl or phenyl,
in which (C₁-C₄)-alkyl may be substituted by cyclopropyl or cyclobutyl or up to pentasubstituted by fluorine,
in which cyclopropyl or cyclobutyl may be up to disubstituted by fluorine,
in which (C₃-C₆)-cycloalkyl may be up to trisubstituted by fluorine, in which phenyl may be substituted by 1 to 3 substituents selected independently from the group of fluorine, chlorine, bromine, cyano, trifluoromethyl, methyl, ethyl, methoxy and ethoxy,
or
is oxygen,
R¹ is phenyl or 6-membered heteroaryl, where phenyl is substituted by 1 to 3 substituents selected independently from the group of fluorine, chlorine, bromine, cyano, methyl, ethyl, cyclopropyl, trifluoromethyl, methoxy, ethoxy, monofluoromethoxy, difluoromethoxy, trifluoromethoxy, hydroxycarbonyl, hydroxycarbonyl, methoxycarbonyl, ethoxycarbonyl, tertbutoxycarbonyl, methylaminocarbonyl, dimethylaminocarbonyl,
where 6-membered heteroaryl is substituted by 1 to 3 substituents selected independently from the group of fluorine, chlorine, bromine, cyano, methyl, ethyl, cyclopropyl, trifluoromethyl, methoxy, ethoxy, monofluoromethoxy, difluoromethoxy, trifluoromethoxy, hydroxycarbonyl, hydroxycarbonyl, methoxycarbonyl, ethoxycarbonyl, tert-butoxycarbonyl, methylaminocarbonyl, dimethylaminocarbonyl,
R² is a group of the formula 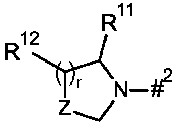 where
#² marks the bond to the carbonyl carbon atom,
r is 1,
Z is CR^{13A}R^{13B},
in which
R^{13A} is hydrogen, fluorine, methyl, trifluoromethyl, difluoromethyl, fluoromethyl, hydroxyl, methoxy, difluoromethoxy or trifluoromethoxy,
R^{13B} is hydrogen or fluorine,
R¹¹ is hydrogen,
R¹² is hydrogen or fluorine,
R³ is hydrogen,
R⁴ is hydrogen, fluorine or methyl,
in which methyl may be up to trisubstituted by fluorine,
R⁵ is hydrogen,
and the salts, solvates and solvates of the salts of the compounds of the formula (I).

3. Compound of the formula (I) according to Claim 1 or 2 in which
A is -CH₂-,
X is a group of the formula 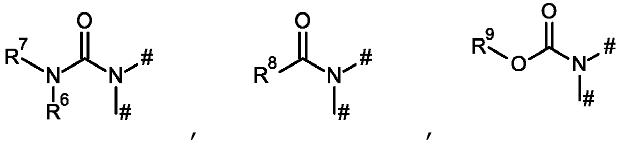 or 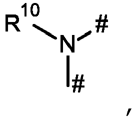 where
# marks the bond to the respective carbon atom of the ring,
R⁶ is (C₁-C₄)-alkyl, cyclopropyl or phenyl,
in which (C₁-C₄)-alkyl may be up to trisubstituted by fluorine, in which phenyl is substituted by 1 or 2 substituents selected independently from the group of chlorine and trifluoromethyl,
R⁷ is hydrogen,
R⁸ is methyl, ethyl, propyl, 3,3,3-trifluoropropyl or cyclopropyl,
R⁹ is tert-butyl,
R¹⁰ is hydrogen, methyl, ethyl, 2,2,2-trifluoroethyl, cyclopropyl or cyclobutyl,
in which methyl may be substituted by cyclopropyl,
in which cyclopropyl may be up to disubstituted by fluorine,
R¹ is phenyl or pyridyl, where phenyl is substituted by 1 or 2 substituents selected independently from the group of fluorine, methyl and trifluoromethyl, where pyridyl is substituted by 1 or 2 substituents selected independently from the group of fluorine, chlorine and trifluoromethyl,
R² is a group of the formula 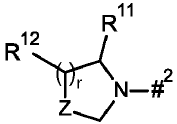 where
#² marks the bond to the carbonyl carbon atom,
r is 1,
Z is CR^{13A}R^{13B},
in which
R^{13A} is hydrogen or fluorine,
R^{13B} is hydrogen,
R¹¹ is hydrogen,
R¹² is hydrogen or fluorine,
R³ is hydrogen,
R⁴ is hydrogen or methyl,
R⁵ is hydrogen,
and the salts, solvates and solvates of the salts of the compounds of the formula (I).

4. Process for preparing a compound as defined in Claims 1 to 3, **characterized in that** either
[A] a compound of the formula (II) 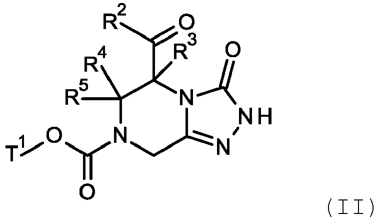 in which R¹, R², R³, R⁴ and R⁵ each have the definitions given in Claim 1,
and
T¹ is (C₁-C₄)-alkyl or benzyl,
is reacted in an inert solvent in the presence of a suitable base with a compound of the formula (III) 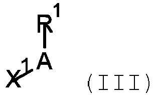 in which
A and R¹ have the definitions given in Claim 1,
and
X¹ is a suitable leaving group, especially chlorine, bromine, iodine, mesylate {(methylsulfonyl)oxy}, triflate {[(trifluoromethyl)sulfonyl]oxy}, nonaflate {[(nonafluorobutyl)sulfonyl]oxy}, nosylate {[(4-nitrophenyl)sulfonyl]oxy} or tosylate {[(4-methylphenyl)sulfonyl]oxy},
to give a compound of the formula (IV) 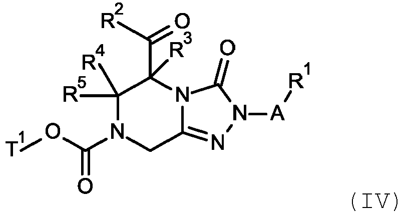 in which A, R¹, R², R³, R⁴, R⁵ and T¹ each have the definitions given in Claim 1,
then the latter are optionally converted by removing the "T¹" group in an inert solvent in the presence of a suitable base or acid to a compound of the formula (V) 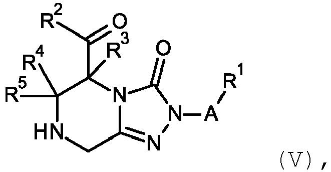 in which A, R¹, R², R³, R⁴ and R⁵ each have the definitions given in Claim 1,
and the latter are then reacted, optionally in an inert solvent,
[A1] in the presence of a suitable base, with a compound of the formula (VI) 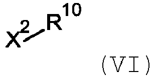 in which
R¹⁰ has the definitions given in Claim 1,
and
X² is a suitable leaving group, especially chlorine, bromine, iodine, mesylate {(methylsulfonyl)oxy}, triflate {[(trifluoromethyl)sulfonyl]oxy}, nonaflate {[(nonafluorobutyl)sulfonyl]oxy}, nosylate {[(4-nitrophenyl)sulfonyl]oxy} or tosylate {[(4-methylphenyl)sulfonyl]oxy},
and then any protecting groups present are detached by methods known to those skilled in the art, and the resulting compounds of the formula (I) are optionally converted with the appropriate (i) solvents and/or (ii) acids or bases to the solvates, salts and/or solvates of the salts thereof,
or
[A2] in the presence of a suitable base, with a compound of the formula (VII) 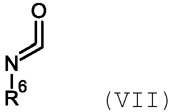 in which
R⁶ has the definitions given in Claim 1, and then any protecting groups present are detached by methods known to those skilled in the art, and the resulting compounds of the formula (I) are optionally converted with the appropriate (i) solvents and/or (ii) acids or bases to the solvates, salts and/or solvates of the salts thereof,
or
[A3] in the presence of a suitable base, with a compound of the formula (VIII) 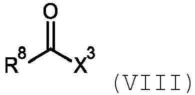 in which
R⁸ has the definitions given in Claim 1,
and
X³ is a suitable leaving group, especially chlorine, bromine or hydroxyl,
and then any protecting groups present are detached by methods known to those skilled in the art, and the resulting compounds of the formula (I) are optionally converted with the appropriate (i) solvents and/or (ii) acids or bases to the solvates, salts and/or solvates of the salts thereof,
or
[B] a compound of the formula (IX) 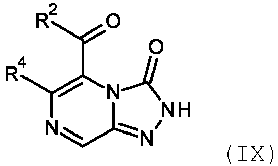 in which R², R⁴ and R⁵ each have the definitions given in Claim 1,
is reacted in an inert solvent in the presence of a suitable base with a compound of the formula (III) 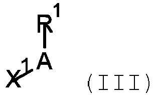 in which
A and R¹ have the definitions given in Claim 1, and
X¹ is a suitable leaving group, especially chlorine, bromine, iodine, mesylate {(methylsulfonyl)oxy}, triflate {[(trifluoromethyl)sulfonyl]oxy}, nonaflate {[(nonafluorobutyl)sulfonyl]oxy}, nosylate {[(4-nitrophenyl)sulfonyl]oxy} or tosylate {[(4-methylphenyl)sulfonyl]oxy},
to give a compound of the formula (X) 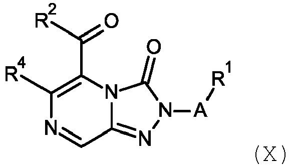 in which A, R¹, R² and R⁴ each have the definitions given in Claim 1,
and then, in a suitable solvent, hydrogenated in the presence of a palladium catalyst in a hydrogen atmosphere, and the resulting compound of the formula (V) 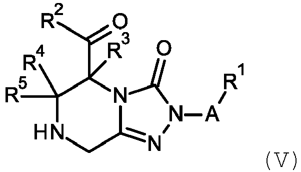 is converted as described under [A] and then any protecting groups present are detached by methods known to those skilled in the art, and the resulting compounds of the formula (I) are optionally converted with the appropriate (i) solvents and/or (ii) acids or bases to the solvates, salts and/or solvates of the salts thereof.

5. Compound as defined in any of Claims 1 to 3 for treatment and/or prophylaxis of diseases.

6. Compound of the formula (I) as defined in Claims 1 to 3 for use in a method for treatment and/or prophylaxis of pulmonary inflammation disorders, particularly of chronic-obstructive pulmonary disease (COPD), of pulmonary emphysema, of chronic bronchitis, of bronchiectasis, of pulmonary hypertension in COPD (PH-COPD), of acute exacerbation in COPD, of cystic fibrosis (mucoviscidosis, CF), of asthma, and of idiopathic pulmonary fibrosis (IPF), of bronchiolitis obliterans syndrome (BOS), of arteriosclerosis, of myocarditis, and of skin and eye inflammation disorders or inflammation disorders of the internal organs.

7. Use of a compound of the formula (I) as defined in Claims 1 to 3 for production of a medicament for treatment and/or prophylaxis of pulmonary inflammation disorders, particularly of chronic-obstructive pulmonary disease (COPD), of pulmonary emphysema, of chronic bronchitis, of bronchiectasis, of pulmonary hypertension in COPD (PH-COPD), of acute exacerbation in COPD, of cystic fibrosis (mucoviscidosis, CF), of asthma, and of idiopathic pulmonary fibrosis (IPF), of bronchiolitis obliterans syndrome (BOS), of arteriosclerosis, of myocarditis, and of skin and eye inflammation disorders or inflammation disorders of the internal organs.

8. Medicament comprising a compound as defined in any of Claims 1 to 3 in combination with one or more inert, nontoxic, pharmaceutically suitable excipients.

9. Medicament comprising a compound as defined in Claims 1 to 3 in combination with one or more active ingredients selected from the group of the PDE 5 inhibitors, sGC activators, sGC stimulators, prostacyclin analogs, IP receptor agonists, endothelin antagonists, compounds that inhibit the signal transduction cascade, and pirfenidone.

10. Medicament according to Claim 8 or 9 for treatment and/or prophylaxis of pulmonary inflammation disorders, particularly of chronic-obstructive pulmonary disease (COPD), of pulmonary emphysema, of chronic bronchitis, of bronchiectasis, of pulmonary hypertension in COPD (PH-COPD), of acute exacerbation in COPD, of cystic fibrosis (mucoviscidosis, CF), of asthma, and of idiopathic pulmonary fibrosis (IPF), of bronchiolitis obliterans syndrome (BOS), of arteriosclerosis, of myocarditis, and of skin and eye inflammation disorders or inflammation disorders of the internal organs.

## Revendications

1. Composé de formule (I) 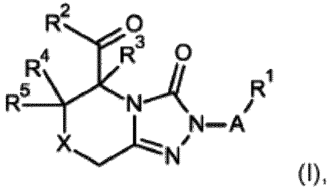 dans laquelle
A représente (C₁-C₄)-alkylène ou CD₂,
(C₁-C₄)-alkylène pouvant être substitué par hydroxy, (C₁-C₄)-alcoxy ainsi que jusqu'à cinq fois par fluor,
ou
représente un groupe de formule 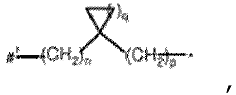 dans laquelle
n représente 0 ou 1,
p représente 0 ou 1,
q représente 1 ou 2,
#¹ marquant la liaison à l'atome d'azote du cycle 5-oxo-4,5-dihydro-1H-1,2,4-triazol-1-yle,
* marquant la liaison à R¹,
X représente un groupe de formule 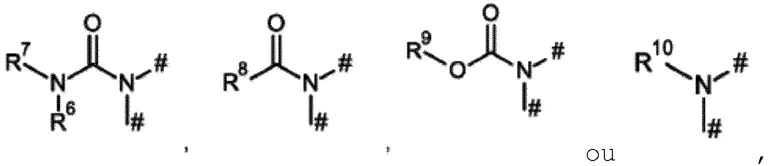
# marquant la liaison à l'atome de carbone respectif du cycle,
R⁶ représentant (C₁-C₆)-alkyle, (C₃-C₆)-cycloalkyle, phényle ou hétéroaryle à 5 ou 6 chaînons,
dans lequel (C₁-C₆)-alkyle peut être substitué par hydroxy, cyano, (C₁-C₄)-alcoxy ou jusqu'à cinq fois par fluor, dans lequel (C₃-C₆)-cycloalkyle peut être substitué par 1 à 3 substituants choisis indépendamment les uns des autres dans le groupe composé par halogène, cyano, trifluorométhyle, (C₁-C₄)-alkyle et (C₁-C₄)-alcoxy,
dans lequel phényle peut être substitué par 1 à 3 substituants choisis indépendamment les uns des autres dans le groupe composé par halogène, cyano, trifluorométhyle, (C₁-C₄)-alkyle et (C₁-C₄)-alcoxy,
dans lequel hétéroaryle à 5 ou 6 chaînons peut être substitué par 1 à 3 substituants choisis indépendamment les uns des autres dans le groupe composé par halogène, cyano, trifluorométhyle, (C₁-C₄)-alkyle et (C₁-C₄)-alcoxy,
R⁷ représente hydrogène ou (C₁-C₄)-alkyle, dans lequel (C₁-C₄)-alkyle peut être substitué jusqu'à cinq fois par fluor,
R⁸ représente (C₁-C₄)-alkyle, (C₃-C₆)-cycloalkyle, phényle ou hétéroaryle à 5 ou 6 chaînons, dans lequel (C₁-C₄)-alkyle peut être substitué par hydroxy, cyano, (C₁-C₄)-alcoxy ou jusqu'à cinq fois par fluor, dans lequel (C₃-C₆)-cycloalkyle peut être substitué par 1 à 3 substituants choisis indépendamment les uns des autres dans le groupe composé par halogène, cyano, trifluorométhyle, (C₁-C₄)-alkyle et (C₁-C₄)-alcoxy,
dans lequel phényle peut être substitué par 1 à 3 substituants choisis indépendamment les uns des autres dans le groupe composé par halogène, cyano, trifluorométhyle, (C₁-C₄)-alkyle et (C₁-C₄)-alcoxy,
dans lequel hétéroaryle à 5 ou 6 chaînons peut être substitué par 1 à 3 substituants choisis indépendamment les uns des autres dans le groupe composé par halogène, cyano, trifluorométhyle, (C₁-C₄)-alkyle et (C₁-C₄)-alcoxy,
R⁹ représente (C₁-C₄)-alkyle, (C₃-C₆)-cycloalkyle ou phényle,
dans lequel (C₁-C₄)-alkyle peut être substitué jusqu'à cinq fois par fluor, dans lequel (C₃-C₆)-cycloalkyle peut être substitué par 1 à 3 substituants choisis indépendamment les uns des autres dans le groupe composé par halogène, cyano, trifluorométhyle, (C₁-C₄)-alkyle et (C₁-C₄)-alcoxy,
dans lequel phényle peut être substitué par 1 à 3 substituants choisis indépendamment les uns des autres dans le groupe composé par halogène, cyano, trifluorométhyle, (C₁-C₄)-alkyle et (C₁-C₄)-alcoxy,
R¹⁰ représente hydrogène, (C₁-C₄)-alkyle, (C₃-C₆)-cycloalkyle ou phényle, dans lequel (C₁-C₄)-alkyle peut être substitué par hydroxy, cyano, (C₁-C₄)-alcoxy, (C₃-C₆)-cycloalkyle ou jusqu'à cinq fois par fluor, dans lequel (C₃-C₆)-cycloalkyle peut être substitué jusqu'à quatre fois par fluor,
dans lequel (C₃-C₆)-cycloalkyle peut être substitué par 1 à 3 substituants choisis indépendamment les uns des autres dans le groupe composé par halogène, cyano, trifluorométhyle, (C₁-C₄)-alkyle et (C₁-C₄)-alcoxy,
dans lequel phényle peut être substitué par 1 à 3 substituants choisis indépendamment les uns des autres dans le groupe composé par halogène, cyano, trifluorométhyle, (C₁-C₄)-alkyle et (C₁-C₄)-alcoxy,
dans lequel hétéroaryle à 5 ou 6 chaînons peut être substitué par 1 à 3 substituants choisis indépendamment les uns des autres dans le groupe composé par halogène, cyano, trifluorométhyle, (C₁-C₄)-alkyle et (C₁-C₄)- alcoxy,
ou
représente oxygène,
R¹ représente (C₃-C₇)-cycloalkyle, phényle ou hétéroaryle à 5 à 10 chaînons, (C₃-C₇)-cycloalkyle pouvant être substitué par 1 à 3 substituants choisis indépendamment les uns des autres dans le groupe composé par halogène, cyano, trifluorométhyle, (C₁-C₄)-alkyle et (C₁-C₄)-alcoxy,
dans lequel (C₁-C₄)-alkyle peut être substitué jusqu'à cinq fois par fluor,
phényle étant substitué par 1 à 4 substituants choisis indépendamment les uns des autres dans le groupe composé par halogène, cyano, (C₁-C₄)-alkyle, (C₃-C₆)-cycloalkyle, trifluorométhyle, (C₁-C₄)-alcoxy, (C₃-C₅)-cycloalcoxy, monofluorométhoxy, difluorométhoxy, trifluorométhoxy, hydroxycarbonyle, (C₁-C₄)-alcoxycarbonyle, aminocarbonyle, mono-(C₁-C₄)-alkylaminocarbonyle, di-(C₁-C₄)-alkylaminocarbonyle, (C₃-C₅)-cycloalkyl-amino-carbonyle, (C₁-C₄)-alkylsulfanyle, (C₁-C₄)- alkylsulfonyle, (C₁-C₄)-alkylsulfonimidoyle, (C₁-C₄)-cycloalkylsulfonyle, aminosulfonyle, mono-(C₁-C₄)-alkylaminosulfonyle, di-(C₁-C₄)- alkylaminosulfonyle, (C₁-C₄)-alkylsulfinyle, amino, mono- (C₁-C₄)-alkylamino ou di-(C₁-C₄)- alkylamino,
ou
hétéroaryle à 5 à 10 chaînons pouvant être substitué par 1 à 4 substituants choisis indépendamment les uns des autres dans le groupe composé par halogène, cyano, (C₁-C₄)-alkyle, (C₃-C₆)-cycloalkyle, trifluorométhyle, (C₁-C₄)-alcoxy, (C₃-C₅)-cycloalcoxy, difluorométhoxy, trifluorométhoxy, 2,2,2-trifluoréthoxy, phényle, hydroxycarbonyle, (C₁-C₄)-alcoxy-carbonyle, aminocarbonyle, mono- (C₁-C₄)- alkylaminocarbonyle, di-(C₁-C₄)-alkyl-aminocarbonyle, (C₃-C₅)-cycloalkyl-aminocarbonyle, (C₁-C₄)-alkylsulfanyle, (C₁ -C₄)-alkylsulfonyle, (C₁-C₄)-alkylsulfonimidoyle, (C₁-C₄)-cycloalkylsulfonyle, aminosulfonyle, mono-(C₁-C₄)-alkylaminosulfonyle, di-(C₁-C₄)- alkylaminosulfonyle, (C₁-C₄)-alkylsulfinyle, amino, mono- (C₁-C₄)-alkylamino ou di-(C₁-C₄)- alkylamino,
dans lequel phényle peut être substitué par méthyle, éthyle, chlore, fluor ou méthoxy,
R² représente un groupe de formule 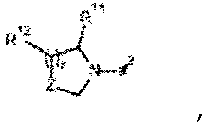
#² marquant la liaison à l'atome de carbone du carbonyle,
r représentant 0 ou 1,
Z représentant O, NR¹⁴, S, SO, SO₂ ou CR^{13A}R^{13B}, dans lequel
R^{13A} représente hydrogène, halogène, cyano, (C₁-C₄)-alkyle, (C₃-C₆)-cycloalkyle,
trifluorométhyle, difluorométhyle, fluorométhyle, hydroxy, (C₁-C₄)-alcoxy, (C₃-C₅)-cycloalcoxy, difluorométhoxy, trifluorométhoxy ou 2,2,2-trifluoréthoxy, dans lequel (C₁-C₄)-alkyle peut être substitué par hydroxy, amino, mono-(C₁-C₄)-alkylamino ou di-(C₁-C₄)-alkylamino,
R^{13B} représente hydrogène, fluor ou (C₁-C₄)- alkyle,
dans lequel (C₁-C₄)-alkyle peut être substitué jusqu'à cinq fois par fluor,
R¹⁴ représente hydrogène ou méthyle,
R¹¹ représentant hydrogène, cyano, (C₁-C₄)- alkyle, acétyle ou formyle,
dans lequel (C₁-C₄)-alkyle peut être substitué par hydroxy ou jusqu'à cinq fois par fluor, dans lequel acétyle peut être substitué par hydroxy ou jusqu'à trois fois par fluor,
R¹² représentant hydrogène, fluor ou (C₁-C₄)-alkyle,
ou
R¹¹ et R¹², conjointement avec les atomes de carbone auxquels ils sont liés, formant un cycle cyclopropyle ou cyclobutyle, dans lequel le cycle cyclopropyle ou cyclobutyle peut être substitué jusqu'à deux fois par fluor,
ou
R¹² et R^{13A}, conjointement avec les atomes de carbone auxquels ils sont liés, formant un cycle cyclopropyle ou cyclobutyle,
dans lequel le cycle cyclopropyle ou cyclobutyle peut être substitué jusqu'à deux fois par fluor,
ou
R^{13A} et R^{13B}, conjointement avec l'atome de carbone auquel ils sont liés, formant un cycle cyclopropyle ou cyclobutyle,
dans lequel le cycle cyclopropyle ou cyclobutyle peut être substitué jusqu'à deux fois par fluor,
R¹², R^{13A} et R^{13B} représentant hydrogène, lorsque
R¹¹ ne représente pas hydrogène,
R¹¹ représentant hydrogène, lorsque l'un des substituants R¹², R^{13A} ou R^{13B} ne représente pas hydrogène, ou représentant un groupe de formule 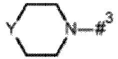
#³ marquant la liaison à l'atome de carbone du carbonyle,
Y représentant NR¹⁵, CR^{16A}R^{16B}, oxygène ou soufre, dans lequel
R¹⁵ représente hydrogène ou méthyle,
R^{16A} représente hydrogène ou méthyle,
R^{16B} représente hydrogène ou méthyle,
R³ représente hydrogène,
R⁴ représente hydrogène ou (C₁-C₄)-alkyle,
dans lequel (C₁-C₄)-alkyle peut être substitué par (C₁-C₄)-alcoxy, hydroxy, mono- (C₁-C₄)- alkylamino ou di-(C₁-C₄)-alkylamino ainsi que jusqu'à cinq fois par fluor,
R⁵ représente hydrogène, ainsi que les sels, les solvates et les solvates des sels des composés de formule (I).

2. Composé de formule (I) selon la revendication 1, dans laquelle
A représente (C₁-C₄)-alkylène,
X représente un groupe de formule 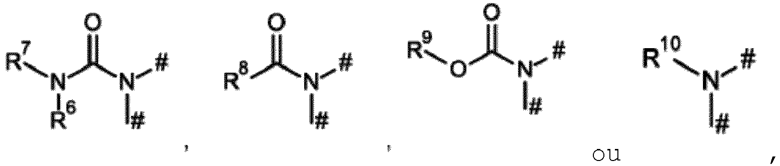
# marquant la liaison à l'atome de carbone respectif du cycle,
R⁶ représentant (C₁-C₆)-alkyle, (C₃-C₅)- cycloalkyle, phényle ou hétéroaryle à 6 chaînons,
dans lequel (C₁-C₆)-alkyle peut être substitué jusqu'à trois fois par fluor, dans lequel (C₃-C₅)-cycloalkyle peut être substitué jusqu'à trois fois par fluor,
dans lequel phényle peut être substitué par 1 ou 2 substituants choisis indépendamment l'un de l'autre dans le groupe composé par fluor, chlore brome, cyano, trifluorométhyle, méthyle, éthyle, méthoxy et éthoxy,
dans lequel hétéroaryle à 6 chaînons peut être substitué par 1 ou 2 substituants choisis indépendamment l'un de l'autre dans le groupe composé par fluor, chlore, cyano, trifluorométhyle, méthyle, éthyle, méthoxy et éthoxy,
R⁷ représentant hydrogène ou méthyle,
dans lequel méthyle peut être substitué jusqu'à trois fois par fluor,
R⁸ représentant (C₁-C₄)-alkyle, (C₃-C₆)-cycloalkyle, phényle ou hétéroaryle à 6 chaînons,
dans lequel (C₁-C₆)-alkyle peut être substitué jusqu'à trois fois par fluor,
dans lequel (C₃-C₅)-cycloalkyle peut être substitué jusqu'à trois fois par fluor,
R⁹ représentant (C₁-C₄)-alkyle ou phényle,
dans lequel (C₁-C₄)-alkyle peut être substitué jusqu'à trois fois par fluor,
R¹⁰ représentant hydrogène, (C₁-C₄)-alkyle, (C₃-C₆)-cycloalkyle ou phényle,
dans lequel (C₁-C₄)-alkyle peut être substitué par cyclopropyle ou cyclobutyle ou jusqu'à cinq fois par fluor,
dans lequel cyclopropyle ou cyclobutyle peut être substitué jusqu'à deux fois par fluor,
dans lequel (C₃-C₆)-cycloalkyle peut être substitué jusqu'à trois fois par fluor, dans lequel phényle peut être substitué par 1 à 3 substituants choisis indépendamment les uns des autres dans le groupe composé par fluor, chlore, brome, cyano, trifluorométhyle, méthyle, éthyle, méthoxy et éthoxy,
ou
représente oxygène,
R¹ représente phényle ou hétéroaryle à 6 chaînons, phényle étant substitué par 1 à 3 substituants choisis indépendamment les uns des autres dans le groupe composé par fluor, chlore, brome, cyano, méthyle, éthyle, cyclopropyle, trifluorométhyle, méthoxy, éthoxy, monofluorométhoxy, difluorométhoxy, trifluorométhoxy, hydroxycarbonyle, hydroxycarbonyle, méthoxycarbonyle, éthoxycarbonyle, tert-butoxycarbonyle, méthylaminocarbonyle, diméthylaminocarbonyle, hétéroaryle à 6 chaînons étant substitué par 1 à 3 substituants choisis indépendamment les uns des autres dans le groupe composé par fluor, chlore, brome, cyano, méthyle, éthyle, cyclopropyle, trifluorométhyle, méthoxy, éthoxy, monofluorométhoxy, difluorométhoxy, trifluorométhoxy, hydroxycarbonyle, hydroxycarbonyle, méthoxycarbonyle, éthoxycarbonyle, tert-butoxycarbonyle, méthylaminocarbonyle, diméthylaminocarbonyle,
R² représente un groupe de formule 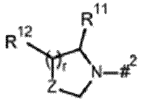
#² marquant la liaison à l'atome de carbone du carbonyle,
r représentant 1,
Z représentant CR^{13A}R^{13B},
dans lequel
R^{13A} représente hydrogène, fluor, méthyle, trifluorométhyle, difluorométhyle, fluorométhyle, hydroxy, méthoxy, difluorométhoxy ou trifluorométhoxy,
R^{13B} représente hydrogène ou fluor,
R¹¹ représentant hydrogène,
R¹² représentant hydrogène ou fluor,
R³ représente hydrogène,
R⁴ représente hydrogène, fluor ou méthyle, dans lequel méthyle peut être substitué jusqu'à trois fois par fluor,
R⁵ représente hydrogène,
ainsi que les sels, les solvates et les solvates des sels des composés de formule (I).

3. Composé de formule (I) selon la revendication 1 ou 2, dans laquelle
A représente -CH₂-,
X représente un groupe de formule 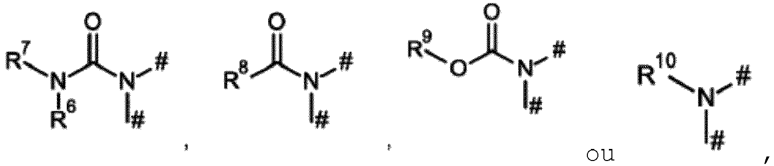
# marquant la liaison à l'atome de carbone respectif du cycle,
R⁶ représentant (C₁-C₄)-alkyle, cyclopropyle ou phényle,
dans lequel (C₁-C₄)-alkyle peut être substitué jusqu'à trois fois par fluor,
dans lequel phényle est substitué par 1 ou 2 substituants choisis indépendamment l'un de l'autre dans le groupe composé par chlore et trifluorométhyle,
R⁷ représentant hydrogène,
R⁸ représentant méthyle, éthyle, propyle, 3,3,3-trifluoropropyle ou cyclopropyle,
R⁹ représentant tert.-butyle,
R¹⁰ représentant hydrogène, méthyle, éthyle, 2,2,2-trifluoréthyle, cyclopropyle ou cyclobutyle,
dans lequel méthyle peut être substitué par cyclopropyle,
dans lequel cyclopropyle peut être substitué jusqu'à deux fois par fluor,
R¹ représente phényle ou pyridinyle, phényle étant substitué par 1 ou 2 substituants choisis indépendamment l'un de l'autre dans le groupe composé par fluor, méthyle ou trifluorométhyle,
pyridinyle étant substitué par 1 ou 2 substituants choisis indépendamment l'un de l'autre dans le groupe composé par fluor, chlore ou trifluorométhyle,
R² représente un groupe de formule 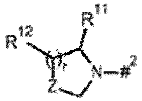
#² marquant la liaison à l'atome de carbone du carbonyle,
r représentant 1,
Z représentant CR^{13A}R^{13B}, dans lequel
R^{13A} représente hydrogène ou fluor,
R^{13B} représente hydrogène,
R¹¹ représente hydrogène,
R¹² représente hydrogène ou fluor,
R³ représente hydrogène,
R⁴ représente hydrogène ou méthyle,
R⁵ représente hydrogène,
ainsi que les sels, les solvates et les solvates des sels des composés de formule (I).

4. Procédé pour la préparation d'un composé tel que défini dans les revendications 1 à 3, **caractérisé en ce que**
[A] soit on transforme un composé de formule (II) 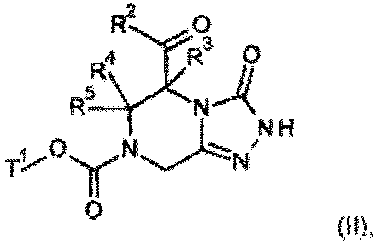 dans laquelle R¹, R², R³, R⁴ et R⁵ possèdent à chaque fois les significations mentionnées dans la revendication 1,
et
T¹ représente (C₁-C₄)-alkyle ou benzyle,
dans un solvant inerte en présence d'une base appropriée avec un composé de formule (III) 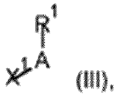 dans laquelle
A et R¹ possèdent les significations mentionnées dans la revendication 1,
et
X¹ représente un groupe partant approprié, en particulier chlore, brome, iode, mésylate {(méthylsulfonyl)oxy}, triflate {[(trifluorométhyl)sulfonyl]oxy}, nonaflate {[(nonafluorobutyl)sulfonyl]oxy}, nosylate {[(4-nitrophényl)sulfonyl]oxy} ou tosylate {[(4-méthyl-phényl)sulfonyl]oxy},
pour donner un composé de formule (IV) 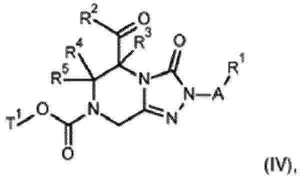 dans laquelle A, R¹, R², R³, R⁴, R⁵ et T¹ possèdent à chaque fois les significations mentionnées dans la revendication 1,
on convertit ensuite celui-ci le cas échéant par élimination du groupe « T¹ » dans un solvant inerte en présence d'une base ou d'un acide approprié(e) en un composé de formule (V) 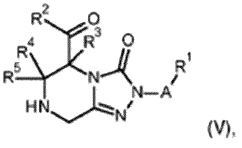 dans laquelle A, R¹, R², R³, R⁴ et R⁵ possèdent à chaque fois les significations mentionnées dans la revendication 1,
et on transforme celui-ci le cas échéant dans un solvant inerte
[A1] en présence d'une base appropriée avec un composé de formule (VI) 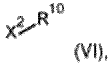 dans laquelle
R¹⁰ possède les significations mentionnées dans la revendication 1,
et
X² représente un groupe partant approprié, en particulier chlore, brome, iode, mésylate {(méthylsulfonyl)oxy}, triflate {[(trifluorométhyl)-sulfonyl]oxy}, nonaflate {[(nonafluorobutyl)sulfonyl]oxy}, nosylate {[(4-nitrophényl)sulfonyl]oxy} ou tosylate {[(4-méthyl- phényl)sulfonyl]oxy},
et par la suite on élimine les groupes protecteurs éventuellement présents par des méthodes connues de l'homme du métier, et on convertit les composés résultants de formule (I) le cas échéant avec les (i) solvants et/ou (ii) acides ou bases respectifs/respectives en leurs solvates, leurs sels et/ou leurs solvates des sels,
ou
[A2] en présence d'une base appropriée avec un composé de formule (VII) 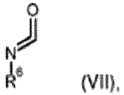 dans laquelle
R⁶ possède les significations mentionnées dans la revendication 1,
et par la suite on élimine les groupes protecteurs éventuellement présents par des méthodes connues de l'homme du métier, et on convertit les composés résultants de formule (I) le cas échéant avec les (i) solvants et/ou (ii) acides ou bases respectifs/respectives en leurs solvates, leurs sels et/ou leurs solvates des sels,
ou
[A3] en présence d'une base appropriée avec un composé de formule (VIII) 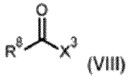 dans laquelle
R⁸ possède les significations mentionnées dans la revendication 1,
et
X³ représente un groupe partant approprié, en particulier chlore, brome ou hydroxy,
et par la suite on élimine les groupes protecteurs éventuellement présents par des méthodes connues de l'homme du métier, et on convertit les composés résultants de formule (I) le cas échéant avec les (i) solvants et/ou (ii) acides ou bases respectifs/respectives en leurs solvates, leurs sels et/ou leurs solvates des sels,
soit
[B] on transforme un composé de formule (IX) 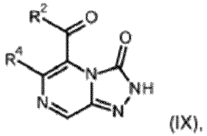 dans laquelle R², R⁴ et R⁵ possèdent à chaque fois les significations mentionnées dans la revendication 1,
dans un solvant inerte en présence d'une base appropriée avec un composé de formule (III) 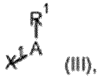 dans laquelle
A et R¹ possèdent les significations mentionnées dans la revendication 1,
et X¹ représente un groupe partant approprié, en particulier chlore, brome, iode, mésylate {(méthylsulfonyl)oxy}, triflate {[(trifluorométhyl)sulfonyl]oxy}, nonaflate {[(nonafluorobutyl)sulfonyl]oxy}, nosylate {[(4-nitrophényl)sulfonyl]oxy} ou tosylate {[(4-méthyl-phényl)sulfonyl]oxy},
en un composé de formule (X) 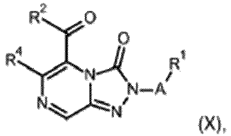 dans laquelle A, R¹, R² et R⁴ possèdent à chaque fois les significations mentionnées dans la revendication 1, et ensuite on hydrogène dans un solvant approprié en présence d'un catalyseur au palladium dans une atmosphère d'hydrogène et on transforme le composé résultant de formule (V) 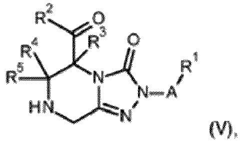 comme décrit en [A] et on élimine ensuite les groupes protecteurs éventuellement présents par des méthodes connues de l'homme du métier, et on convertit les composés résultants de formule (I) le cas échéant avec les (i) solvants et/ou (ii) acides ou bases respectifs/respectives en leurs solvates, leurs sels et/ou leurs solvates des sels.

5. Composé, tel que défini dans l'une quelconque des revendications 1 à 3, pour le traitement et/ou la prophylaxie de maladies.

6. Composé de formule (I), tel que défini dans l'une quelconque des revendications 1 à 3, pour une utilisation dans un procédé de traitement et/ou de prophylaxie de maladies pulmonaires inflammatoires, notamment de la maladie pulmonaire chronique obstructive (MPCO), de l'emphysème pulmonaire, de la bronchite chronique, de la bronchiectasie, de l'hypertonie pulmonaire dans la MPCO (HP-MPCO), de l'exacerbation aiguë dans la MPCO, de la fibrose kystique (mucoviscidose, CF), de l'asthme, ainsi que de la fibrose pulmonaire idiopathique (FPI) du syndrome de la bronchite oblitérante (SBO), de l'artériosclérose, de la myocardite, ainsi que de maladies cutanées et oculaires inflammatoires ou de maladies inflammatoires des organes internes.

7. Utilisation d'un composé de formule (I), tel que défini dans l'une quelconque des revendications 1 à 3, pour la préparation d'un médicament pour le traitement et/ou la prophylaxie de maladies pulmonaires inflammatoires, notamment de la maladie pulmonaire chronique obstructive (MPCO), de l'emphysème pulmonaire, de la bronchite chronique, de la bronchiectasie, de l'hypertonie pulmonaire dans la MPCO (HP-MPCO), de l'exacerbation aiguë dans la MPCO, de la fibrose kystique (mucoviscidose, CF), de l'asthme, ainsi que de la fibrose pulmonaire idiopathique (FPI) du syndrome de la bronchite oblitérante (SBO), de l'artériosclérose, de la myocardite, ainsi que de maladies cutanées et oculaires inflammatoires ou de maladies inflammatoires des organes internes.

8. Médicament contenant un composé, tel que défini dans l'une quelconque des revendications 1 à 3, en combinaison avec un ou plusieurs excipients inertes, non toxiques, pharmaceutiquement appropriés.

9. Médicament contenant un composé, tel que défini dans l'une quelconque des revendications 1 à 3, en combinaison avec un ou plusieurs principes actifs choisis dans le groupe composé par des inhibiteurs de PDE 5, des activateurs de sGC, des stimulateurs de sGC, des analogues de prostacycline, des agonistes du récepteur IP, des antagonistes d'endothéline, des composés inhibant la cascade de transduction de signal et la pirfénidone.

10. Médicament selon la revendication 8 ou 9 pour le traitement et/ou la prophylaxie de maladies pulmonaires inflammatoires, notamment de la maladie pulmonaire chronique obstructive (MPCO), de l'emphysème pulmonaire, de la bronchite chronique, de la bronchiectasie, de l'hypertonie pulmonaire dans la MPCO (HP-MPCO), de l'exacerbation aiguë dans la MPCO, de la fibrose kystique (mucoviscidose, CF), de l'asthme, ainsi que de la fibrose pulmonaire idiopathique (FPI) du syndrome de la bronchite oblitérante (SBO), de l'artériosclérose, de la myocardite, ainsi que de maladies cutanées et oculaires inflammatoires ou de maladies inflammatoires des organes internes.
